# EUROPEAN PATENT APPLICATION

(11) **EP 4 368 023 A1**
(43) Date of publication of application: **15.05.2024**
(21) Application number: 22837526.7
(22) Date of filing: 27.06.2022
(51) Int. Cl.: A01N 43/56, A01N 25/00, A01N 43/653, A01P 5/00, C07D 231/12, C07D 249/08

(54) **NEMATOCIDAL COMPOSITION**

(30) Priority: 08.07.2021 JP 2021113233
(71) Applicant: Ishihara Sangyo Kaisha, Ltd., Osaka-shi, Osaka 550-0002 (JP)
(72) Inventor: KIRIYAMA, Kazuhisa, Osaka-shi, Osaka 550-0002 (JP); KUBOTA, Yasuhiro, Osaka-shi, Osaka 550-0002 (JP); ITO, Tomoya, Osaka-shi, Osaka 550-0002 (JP); SHIMIZU, Manabu, Osaka-shi, Osaka 550-0002 (JP); SASAMI, Takahiro, Osaka-shi, Osaka 550-0002 (JP); NAKAGAWA, Tomomi, Osaka-shi, Osaka 550-0002 (JP)
(74) Representative: Müller-Boré & Partner Patentanwälte PartG mbB
(86) International application number: PCT/JP2022/025567
(87) International publication number: WO 2023/282110

(57) **Abstract**

To provide a nematicidal composition exhibiting excellent nematicidal activity.

A nematicidal composition comprising as an active ingredient a compound represented by the formula (I) or its salt: 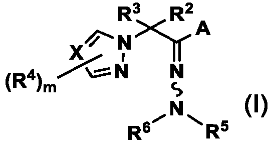 wherein the symbols are as defined in the specification.

## Description

### TECHNICAL FIELD

The present invention relates to a nematicidal composition comprising as an active ingredient a novel formhydrazone compound bonded to a quaternary carbon atom or its salt.

### BACKGROUND ART

Compounds having a specific hydrazone structure are known to be useful for controlling various pests. Patent Document 1 discloses a hydrazone derivative having a chemical structure comprising a tertiary carbon atom such as -CH(Me)- or -CH(i-Pr)-useful as an agricultural and horticultural insecticide or an agricultural and horticultural nematicide, and a method of its use. However, it failed to disclose specific examples relating to a compound of the after-described formula (I), that is a hydrazone derivative having a chemical structure characterized by a quaternary carbon atom, a nematicidal composition comprising it as an active ingredient, and a method for their use.

### PRIOR ART DOCUMENTS

### PATENT DOCUMENTS

Patent Document 1: WO2020/179910

### DISCLOSURE OF INVENTION

### TECHNICAL PROBLEM

Nematodes harmful to growth of agricultural and horticultural crop plants are various, and development of agricultural and horticultural nematicides for controlling such nematodes is proceeding. Continuous use of the same agricultural and horticultural chemical over a long period may promote the aimed nematodes to acquire chemical resistance. Thus, development of a novel agricultural and horticultural nematicide having high control effects against nematodes, regardless of the application site, has been desired.

Under these circumstances, the object of the present invention is to provide a nematicidal composition comprising as an active ingredient a compound represented by the after-described formula (I) or its salt, showing excellent nematicidal activity.

### SOLUTION TO PROBLEM

The present inventors have conducted extensive studies to achieve the above object and as a result, found that a composition comprising as an active ingredient a compound represented by the after-described formula (I) or its salt has excellent control effects against nematodes.

That is, the present invention provides the following.
[1] A nematicidal composition (hereinafter sometimes referred to as "the present composition") comprising as an active ingredient a compound represented by the formula (I) or its salt (hereinafter sometimes referred to as compound (I)): 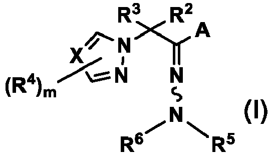 [0009]
   wherein X is N, CH or CR⁴,
   A is an unsubstituted phenyl, or a phenyl substituted by one to five R¹,
   R¹ is halogen, alkyl, haloalkyl, alkenyl, alkynyl, cycloalkyl, alkoxy or haloalkoxy, provided that when there are two R¹, the two R¹ together may form a ring which may be substituted by one or two Z¹,
   Z¹ is halogen or alkyl,
   R² and R³ are alkyl, or R² and R³ together form a (C₃-C₆)-carbon ring,
   R⁴ is halogen, alkyl, haloalkyl, alkenyl, alkynyl, cycloalkyl, alkoxy, haloalkoxy, alkylthio, alkylsulfinyl, alkylsulfonyl, nitro or cyano,
   R⁵ is CHO,
   R⁶ is H, -SO₂CF₃, -C(=O)OZ³, alkyl, alkenyl, alkynyl, alkoxyalkyl, or aralkyl which may be substituted by Z²,
   Z² is alkoxy,
   Z³ is aralkyl which may be substituted by Z², or alkyl, and
   m is an integer of from 0 to 2.
[2] A method for controlling nematodes, which comprises applying a nematicidal composition containing an effective amount of a compound represented by the formula (I) or its salt to soil, to nematodes or to a plant body: 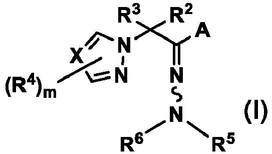
   wherein X is N, CH or CR⁴,
   A is an unsubstituted phenyl, or a phenyl substituted by one to five R¹,
   R¹ is halogen, alkyl, haloalkyl, alkenyl, alkynyl, cycloalkyl, alkoxy or haloalkoxy, provided that when there are two R¹, the two R¹ together may form a ring which may be substituted by one or two Z¹,
   Z¹ is halogen or alkyl,
   R² and R³ are alkyl, or R² and R³ together form a (C₃-C₆)-carbon ring,
   R⁴ is halogen, alkyl, haloalkyl, alkenyl, alkynyl, cycloalkyl, alkoxy, haloalkoxy, alkylthio, alkylsulfinyl, alkylsulfonyl, nitro or cyano,
   R⁵ is CHO,
   R⁶ is H, -SO₂CF₃, -C(=O)OZ³, alkyl, alkenyl, alkynyl, alkoxyalkyl, or aralkyl which may be substituted by Z²,
   Z² is alkoxy,
   Z³ is aralkyl which may be substituted by Z², or alkyl, and
   m is an integer of from 0 to 2.
[3] The nematicidal composition according to the above [1], wherein in the compound represented by the formula (I) or its salt, A is phenyl substituted by one to five R¹, R¹ is halogen, alkyl, haloalkyl, alkenyl, alkynyl, cycloalkyl, alkoxy or haloalkoxy, provided that when there are two R¹, the two R¹ together may form a ring which may be substituted by one or two Z¹.
[4] The method for controlling nematodes according to the above [2], wherein in the compound represented by the formula (I) or its salt, A is phenyl substituted by one to five R¹, R¹ is halogen, alkyl, haloalkyl, alkenyl, alkynyl, cycloalkyl, alkoxy or haloalkoxy, provided that when there are two R¹, the two R¹ together may form a ring which may be substituted by one or two Z¹.
[5] A compound represented by the formula (IA) or its salt: 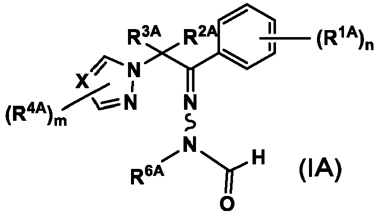
   wherein X is N, CH or CR^{4A},
   R^{1A} is halogen, (C₁-C₆)-alkyl, (C₁-C₆)-haloalkyl, (C₂-C₆)-alkenyl, (C₂-C₆)-alkynyl, (C₃-C₆)-cycloalkyl, (C₁-C₆)-alkoxy or (C₁-C₆)-haloalkoxy, provided that when there are two R^{1A}, the two R^{1A} together may form a ring which may be substituted by one or two Z^{1A},
   Z^{1A} is halogen or (C₁-C₆)-alkyl,
   R^{2A} and R^{3A} are (C₁-C₆)-alkyl, or R^{2A} and R^{3A} together may form a (C₃-C₆)-carbon ring,
   R^{4A} is halogen, (C₁-C₆)-alkyl, (C₁-C₆)-haloalkyl, (C₂-C₆)-alkenyl, (C₂-C₆)-alkynyl, (C₃-C₆)-cycloalkyl, (C₁-C₆)-alkoxy, (C₁-C₆)-haloalkoxy, (C₁-C₆)-alkylthio, (C₁-C₆)-alkylsulfinyl, (C₁-C₆)-alkylsulfonyl, nitro or cyano,
   R^{6A} is H, -SO₂CF₃, -C(=O)OZ^{3A}, (C₁-C₆)-alkyl, (C₂-C₆)-alkenyl, (C₂-C₆)-alkynyl, (C₁-C₆)-alkoxy-(C₁-C₆)-alkyl, or (C₇-C₁₂)-aralkyl which may be substituted by Z^{2A},
   Z^{2A} is (C₁-C₆)-alkoxy,
   Z^{3A} is (C₇-C₁₂)-aralkyl which may be substituted by Z^{2A}, or (C₁-C₆)-alkyl,
   n is an integer of from 1 to 5, and
   m is an integer of from 0 to 2.
[6] A compound represented by the formula (IB) or its salt: 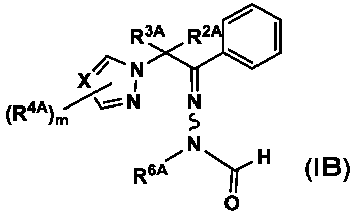
   wherein X is N, CH or CR^{4A},
   R^{2A} and R^{3A} are (C₁-C₆)-alkyl, or R^{2A} and R^{3A} together may form a (C₃-C₆)-carbon ring,
   R^{4A} is halogen, (C₁-C₆)-alkyl, (C₁-C₆)-haloalkyl, (C₂-C₆)-alkenyl, (C₂-C₆)-alkynyl, (C₃-C₆)-cycloalkyl, (C₁-C₆)-alkoxy, (C₁-C₆)-haloalkoxy, (C₁-C₆)-alkylthio, (C₁-C₆)-alkylsulfinyl, (C₁-C₆)-alkylsulfonyl, nitro or cyano,
   R^{6A} is H, -SO₂CF₃, -C(=O)OZ^{3A}, (C₁-C₆)-alkyl, (C₂-C₆)-alkenyl, (C₂-C₆)-alkynyl or (C₁-C₆)-alkoxy-(C₁-C₆)-alkyl, or (C₇-C₁₂)-aralkyl which may be substituted by Z^{2A},
   Z^{2A} is (C₁-C₆)-alkoxy,
   Z^{3A} is (C₇-C₁₂)-aralkyl which may be substituted by Z^{2A}, or (C₁-C₆)-alkyl, and
   m is an integer of from 0 to 2.
[7] A method for controlling nematodes, which comprises applying a nematicidal composition containing an effective amount of the compound or its salt as defined in the above [5] or [6], to soil, to nematodes or to a plant body.
[8] An agricultural and horticultural nematicide comprising as an active ingredient the compound or its salt as defined in the above [5] or [6].

In the following, the compound represented by the formula (I) or its salt will sometimes be referred to as "compound (I)", the compound represented by the formula (IA) or its salt as "compound (IA)", and the compound represented by the formula (IB) or its salt as "compound (IB)".

### ADVANTAGEOUS EFFECTS OF INVENTION

The present composition exhibits excellent control effects against nematodes and is thereby useful as an agricultural and horticultural nematicide.

### DESCRIPTION OF EMBODIMENTS

Substituents and chemical structures of the compound (I), the compound (IA) and the compound (IB) will be described.

In the compound (I), the number of R¹ as a substituent on the phenyl may be one or more, and in the case of two or more, the respective R¹ may be the same or different from each other. In the compound (IA), the number of R^{1A} as a substituent on the phenyl may be one or more, and in the case of two or more, the respective R^{1A} may be the same or different from each other.

In the compound (I), when the number of R⁴ as a substituent on the pyrazole (X is CR⁴) is two or three, the two or three R⁴ may be the same or different from each other. In the compound (I), when the number of R⁴ as a substituent on 1,2,4-triazole (X is N) is two, the two R⁴ may be the same or different from each other. In the compound (IA) and the compound (IB), and when the number of R^{4A} as a substituent on the pyrazole (X is CR^{4A}) is two or three, the two or three R^{4A} may be the same or different from each other. In the compound (IA) and the compound (IB), when the number of R^{4A} as a substituent on 1,2,4-triazole (X is N) is two, the two R^{4A} may be the same or different from each other.

In this specification, "n-" represents normal, "s-" secondary and "tert" tertiary.

The halogen or halogen as a substituent may be a fluorine, chlorine, bromine or iodine atom. The number of the halogen as a substituent may be one or more, and in the case of two or more, the respective halogen atoms may be the same or different from each other.

Further, the position of the halogen as a substituent may be any position.

In the compound (I), the compound (IA) and the compound (IB), "C_{P}-C_{T}" means that the number of carbon atoms is from P to T. For example, "C₁-C₃" means that the number of carbon atoms is from1 to 3. Further, the wording "which may be substituted" means the group or moiety being substituted or unsubstituted.

The alkyl, alkyl moiety or (C₁-C₆)-alkyl may, for example, be a linear or branched group such as methyl, ethyl, n-propyl, isopropyl, n-butyl, isobutyl, s-butyl, tert-butyl, n-pentyl, neopentyl or n-hexyl.

The alkenyl or (C₂-C₆)-alkenyl may, for example, be a linear or branched group such as vinyl, 1-propenyl, 2-propenyl, isopropenyl, 2-methyl-1-propenyl, 1-methyl-1-propenyl, 2-methyl-2-propenyl, 1-methyl-2-propenyl, 1-butenyl, 2-butenyl, 3-butenyl, 1-pentenyl, 2-pentenyl, 2-methyl-2-butenyl, 3-methyl-2-butenyl, 1-hexenyl or 2,3-dimethyl-2-butenyl. Further, in a case where such a group has geometric isomers, the group may be one of E-isomer and Z-isomer or may be a mixture of E-isomer and Z-isomer in an optional ratio and is not particularly limited so long as it has carbon atoms within a specified range.

The alkynyl or (C₂-C₆)-alkynyl may, for example, be a linear or branched group such as ethynyl, 1-propynyl, 2-propynyl, 1-butynyl, 2-butynyl, 3-butynyl, 1-methyl-2-propynyl, 2-methyl-3-butynyl, 1-hexynyl, 2-hexynyl, 3-hexynyl, 4-hexynyl or 5-hexynyl.

The cycloalkyl or (C₃-C₆)-cycloalkyl may, for example, be a group such as cyclopropyl, cyclobutyl, cyclopentyl or cyclohexyl.

The haloalkyl or (C₁-C₆)-haloalkyl may, for example, be a linear or branched alkyl group which is partially or entirely substituted by one to seven halogen atoms which are the same or different from each other, such as chloromethyl, difluoromethyl, trifluoromethyl, trichloromethyl, 1,1-dichloro-1-fluoromethyl, 1-fluoroethyl, 2,2-difluoroethyl, 2,2,2-trifluoroethyl, 2,2,2-trichloroethyl, 1,1,2,2-tetrafluoroethyl, 1,1,2,2-tetrachloroethyl, 1,1,2,2,2-pentafluoroethyl, 3,3,3-trifluoropropyl, 2,2,3,3,3-pentafluoropropyl or 1,1,1,2,3,3,3-heptafluoropropan-2-yl.

The alkoxy or (C₁-C₆)-alkoxy may, for example, be a linear or branched group such as methoxy, ethoxy, n-propoxy, isopropoxy, n-butoxy, isobutoxy, s-butoxy, tert-butoxy, n-pentyloxy, neopentyloxy or n-hexyloxy.

The haloalkoxy or (C₁-C₆)-haloalkoxy may, for example, be a linear or branched group which is partially or entirely substituted by one to six halogen atoms which are the same or different from each other, such as difluoromethoxy, trifluoromethoxy, 2-chloroethoxy, 2,2-difluoroethoxy, 2,2,2-trifluoroethoxy, 1,1,2,2-tetrafluoroethoxy, 1,1,2,2,2-pentafluoroethoxy, 2,2,3,3,3-pentafluoropropoxy or (1,1,1,3,3,3-hexafluoropropan-2-yl)oxy.

The carbon ring or (C₃-C₆)-carbon ring may, for example, be a saturated ring such as cyclopropane, cyclobutane, cyclopentane or cyclohexane.

The alkylthio or (C₁-C₆)-alkylthio may, for example, be a linear or branched group such as methylthio, ethylthio, isopropylthio, n-butylthio, isobutylthio, s-butylthio, tert-butylthio, n-pentylthio, neopentylthio or n-hexylthio.

The alkylsulfinyl or (C₁-C₆)-alkylsulfinyl may, for example, be a linear or branched group such as methylsulfinyl, ethylsulfinyl, isopropylsulfinyl, n-butylsulfinyl, isobutylsulfinyl, s-butylsulfinyl, tert-butylsulfinyl, n-pentylsulfinyl, neopentylsulfinyl or n-hexylsulfinyl.

The alkylsulfonyl or (C₁-C₆)-alkylsulfonyl may, for example, be a linear or branched group such as methylsulfonyl, ethylsulfonyl, isopropylsulfonyl, n-butylsulfonyl, isobutylsulfonyl, s-butylsulfonyl, tert-butylsulfonyl, n-pentylsulfonyl, neopentylsulfonyl or n-hexylsulfonyl.

In the compound (I), A on which two R¹ together form a ring, may be benzodioxolanyl or benzodioxanyl, which may be substituted by one or two Z¹. Further, in the compound (IA), the phenyl on which two R^{1A} together form a ring, may be benzodioxolanyl or benzodioxanyl which may be substituted by one or two Z^{1A}. In a case where there are two Z¹ or Z^{1A}, the two Z¹ or the two Z^{1A} may be the same or different from each other.

The aralkyl or (C₇-C₁₂)-aralkyl may, for example, be benzyl, 1-phenylethyl or naphthylmethyl. The aralkyl which may be substituted by Z², or the (C₇-C₁₂)-aralkyl which may be substituted by Z², may, for example, be p-methoxybenzyl, 2,3-dimethoxybenzyl or 2,4-dimethoxybenzyl.

The number of the substituent Z² or Z^{2A} may be two or more, and in the case of two or more, the respective substituents may be the same or different. Further, the position of Z² or Z^{2A} may be any position.

In the compound (I), the "phenyl substituted by one to five R¹" means phenyl substituted by one, two, three, four or five R¹, and the "unsubstituted phenyl" means phenyl having no R¹ as a substituent. Further, in a specific embodiment of the compound (I), the number of the substituent R¹ may be an integer of from 1 to 2, from 1 to 3, from 1 to 4, from 2 to 3, from 2 to 4, from 2 to 5, from 3 to 4, from 3 to 5, from 4 to 5, in some cases.

In the compound (IA), "n is an integer of from 1 to 5" means that n is an integer of 1, 2, 3, 4 or 5. Further, in a specific embodiment of the compound (IA), n may be an integer of from 1 to 2, from 1 to 3, from 1 to 4, from 2 to 3, from 2 to 4, from 2 to 5, from 3 to 4, from 3 to 5, or from 4 to 5, in some cases.

In the compound (IA), as shown in the following chemical formula, the position of the R^{1A} group may be any position on the phenyl ring. When m is 1 or more, as shown in the following chemical formula, the position of the R^{4A} group may be one or both of the 3- and 5-positions on the hetero 5-membered ring.

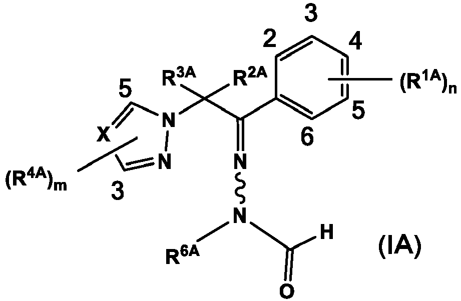

For example, when n is 1 in the compound (IA), the position of the substituent R¹ is any of positions as shown in the following formulae (IAa) to (IAc).

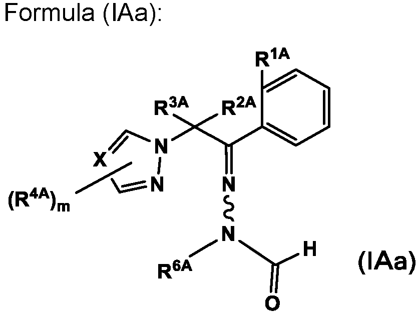

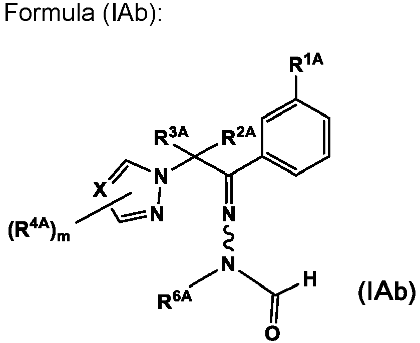

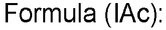

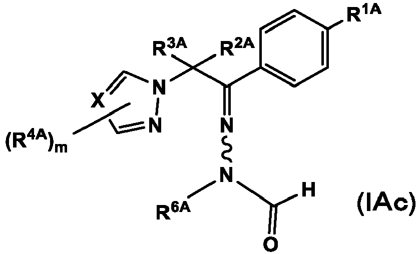

In the compound (IB), when m is 1 or more, as shown in the following chemical formula, the position of the R^{4A} group may be one or both of the 3- and 5-positions on the hetero 5-membered ring.

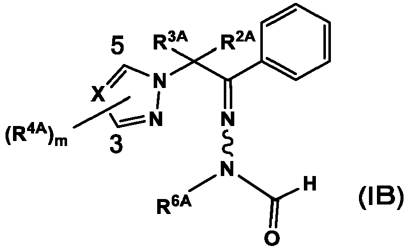

In the compound (I), the compound (IA) and the compound (IB), "m is an integer of from 0 to 2" means that m is an integer of 0, 1 or 2. Further, in a specific embodiment of the compound (I), the compound (IA) and the compound (IB), m may be an integer of from 0 to 1, or from 1 to 2, in some cases.

In the compound (I), the compound (IA) and the compound (IB), when m is 0, (R⁴)ₘ and (R^{4A})ₘ are H, that is both the 3- and 5-positions of the hetero-5-membered ring are not substituted.

In the following, the description regarding the compound (I) includes descriptions regarding the compound (IA) and the compound (IB), since the compound (I) includes the compound (IA) and the compound (IB).

The salt of the compound represented by the formula (I) includes all kinds of salts so long as they are agriculturally acceptable, and, for example, alkali metal salts (such as a sodium salt and a potassium salt), alkaline earth metal salts (such as a magnesium salt and a calcium salt), inorganic acid salts (such as a hydrochloride, a perchlorate, a sulfate and a nitrate), and organic acid salts (such as an acetate and a methanesulfonate) may be mentioned.

As the compound (I) of the present invention, isomers such as optical isomers and geometric isomers may sometimes be present, and the present invention includes the respective isomers and isomer mixtures.

More specifically, as the compound (I) of the present invention, two or more types of geometric isomers are present depending upon the number of double bonds (carbon-carbon or carbon-nitrogen) in the structure. Accordingly, the present invention includes all kinds of conceivable geometric isomers and mixtures of them at optional ratios.

For example, in the compound disclosed in this specification, the bond represented by the wave line in the structural formula means that all of two or more geometric isomers derived from a double bond (carbon-carbon or carbon-nitrogen) are included.

In the compound (I) of the present invention, various isomers other than the above-mentioned isomers are included within a range of common knowledge in this technical field. Further, an individual isomer may be synthesized by common knowledge and general experimental means in this technical field.

Further, depending upon the type of an isomer, it may have a chemical structure different from the described structural formula, but for those skilled in the art, it is sufficiently recognizable that such a chemical structure is in a relation of an isomer, and such an isomer is evidently within the scope of the present invention.

Now, the method for producing the compound (I) contained in the present composition will be described.

The compound (I) may be produced in accordance with the following Reaction A to O and a conventional method for producing a salt, however, the method to obtain the compound (I) is not limited to such a method. For example, it may be produced by applying a functional group transformation (such as hydrolysis, oxidation, reduction, esterification, amidation, alkylation or halogenation) known in this technical field to a substituent on the phenyl, and/or the pyrazole or 1,2,4-triazole. Further, in the production of the compound (I), protection and deprotection commonly employed in this technical field may be applied as the case requires.

### Scheme 1

Scheme 1 is to obtain compound of the formula (I-2) or the formula (2) from compound of the formula (4) by Reaction A or Reaction B, and to obtain compound (I-1) from the compound of the formula (I-2) or the formula (2) by Reaction A or Reaction B.

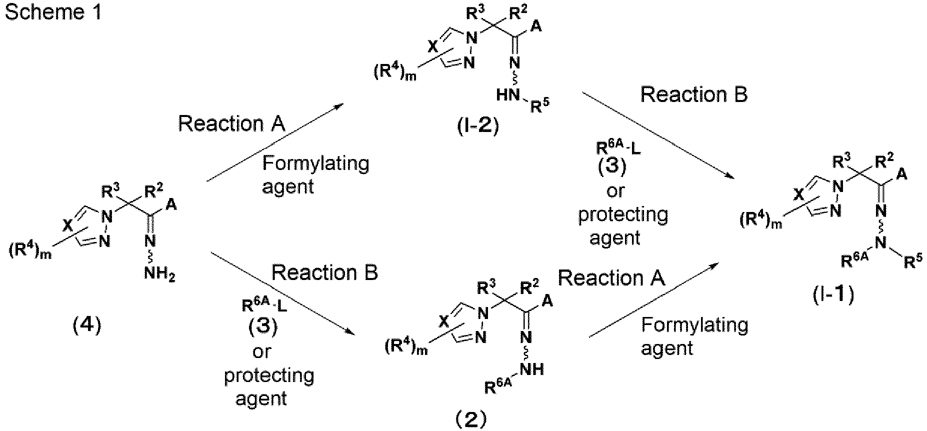

In the formulae, A, X, m, R², R³, R⁴ and R⁵ are as defined above. R^{6A} is - SO₂CF₃, -C(=O)OZ³, alkyl, alkenyl, alkynyl, alkoxyalkyl, or aralkyl which may be substituted by Z², and Z² and Z³ are as defined above. L is a leaving group, more specifically, a chlorine atom, a bromine atom, an iodine atom or a trifluoromethanesulfonyl group.

### Reaction A

Reaction A is to react the compound of the formula (2) with a formylating agent to obtain the compound (I-1), or to react the compound of the formula (4) with a formylating agent to obtain the compound of the formula (I-2).

Reaction A may be carried out usually in the presence of a formylating agent and a solvent.

The formylating agent is not particularly limited so long as the reaction proceeds, and may, for example, be formic acid or N-formylsaccharin. The formylating agent may be used in an amount of from 1 to 10 equivalents, preferably from 1 to 2 equivalents to the compound of the formula (2) or the formula (4).

"Equivalents" means "molar equivalents", and the same applies hereinafter.

The solvent is not particularly limited so long as the reaction proceeds, and one or more may be properly selected from aromatic hydrocarbons such as benzene, toluene, xylene and chlorobenzene; aliphatic hydrocarbons such as hexane, heptane, petroleum ether, ligroin and cyclohexane; halogenated hydrocarbons such as chloroform, dichloromethane, carbon tetrachloride and 1,2-dichloroethane; esters such as methyl formate, ethyl formate, methyl acetate and ethyl acetate; alcohols such as methanol, ethanol, propanol and tert-butanol; polar aprotic solvents such as dimethyl sulfoxide, sulfolane, dimethylacetamide, N,N-dimethylformamide (hereinafter referred to as DMF), N-methylpyrrolidone, pyridine, acetonitrile and propionitrile; ketones such as acetone and methyl ethyl ketone; acetic acid, etc.

Reaction A may be carried out in the presence of a formate such as methyl formate or ethyl formate as the case requires.

The formate may be used in an amount of from 1 to 50 equivalents, preferably from 1 to 30 equivalents to the compound of the formula (2) or the formula (4).

Reaction A may be carried out in the presence of a dehydration condensation agent and a base as the case requires.

The dehydration condensation agent may, for example, be N,N'-dicyclohexylcarbodiimide, chlorosulfonyl isocyanate, N,N'-carbonyl diimidazole or anhydrous trifluoroacetate. The dehydration condensation agent may be used in an amount of from 1 to 5 equivalents, preferably from 1 to 2 equivalents to the compound of the formula (2) or the formula (4).

As the base, one or more may be properly selected from alkali metals such as sodium and potassium; alkali metal alkoxides such as sodium methoxide, sodium ethoxide and potassium tert-butoxide; carbonates such as sodium carbonate and potassium carbonate; bicarbonates such as sodium bicarbonate and potassium bicarbonate; metal hydroxides such as sodium hydroxide and potassium hydroxide; metal hydrides such as sodium hydride and potassium hydride; amines such as monomethylamine, dimethylamine and triethylamine; pyridines such as pyridine and 4-dimethylaminopyridine; organolithium compounds such as methyllithium, n-butyllithium and lithium diisopropylamide, etc. The base may be used in an amount of from 1 to 5 equivalents, preferably from 1 to 2 equivalents to the compound of the formula (2) or the formula (4).

The reaction temperature is usually from about 0°C to about 200°C, preferably from about 0°C to about 100°C. The reaction time is usually from about 1 hour to about 24 hours.

The compound of the formula (2) used in Reaction A may be produced in accordance with the following Reaction B.

### Reaction B

Reaction B is to react the compound of the formula (I-2) with compound of the formula (3) or a protecting agent to obtain the compound (I-1), or to react compound of the formula (4) with compound of the formula (3) or a protecting agent to obtain the compound of the formula (2).

Reaction B may be carried out usually in the presence of a base and a solvent. The compound of the formula (3) or the protecting agent may be used in an amount of from 1 to 5 equivalents, preferably from 1 to 2 equivalents to the compound of the formula (I-2) or the compound of the formula (4).

As the base, ones mentioned for the above Reaction A may be used. The base may be used in an amount of from 1 to 5 equivalents, preferably from 1 to 2 equivalents to the compound of the formula (I-2) or the compound of the formula (4).

The protecting agent may be suitably selected from methoxymethyl chloride, benzyl bromide, p-methoxybenzyl chloride, di-tert-butyl dicarbonate, benzyl chloroformate, etc.

The solvent is not particularly limited so long as the reaction proceeds, and one or more may be properly selected from aromatic hydrocarbons such as benzene, toluene, xylene and chlorobenzene; aliphatic hydrocarbons such as hexane, heptane, petroleum ether, ligroin and cyclohexane; halogenated hydrocarbons such as chloroform, dichloromethane, carbon tetrachloride and 1,2-dichloroethane; ethers such as dioxane, tetrahydrofuran, diethyl ether and dimethoxyethane; esters such as methyl acetate and ethyl acetate; alcohols such as methanol, ethanol, propanol and tert-butanol; polar aprotic solvents such as dimethyl sulfoxide, sulfolane, dimethylacetamide, N,N-dimethylformamide, N-methylpyrrolidone, pyridine, acetonitrile and propionitrile; ketones such as acetone and methyl ethyl ketone; water, etc.

The reaction temperature is usually from about -78°C to about 200°C, preferably from about 0°C to about 100°C. The reaction time is usually from about 1 hour to about 48 hours.

The compound of the formula (3) may be produced in accordance with a known method, or a commercial product may be used.

The compound of the formula (4) may be produced in accordance with the after-described reaction H.

### Reaction C

Reaction C is to convert compound of the formula (I-3), using compound of the formula (b-1), to compound of the formula (I-4).

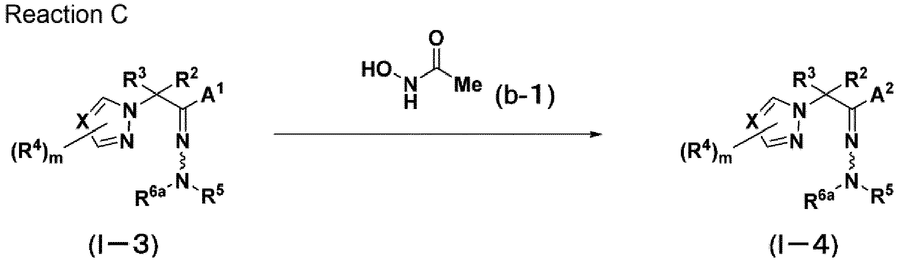

In the formulae, X, m, R², R³, R⁴ and R⁵ are as defined above. A¹ is phenyl substituted by fluorine, and A² is phenyl substituted by hydroxy. R^{6a} is methoxymethyl, benzyl, p-methoxybenzyl, tert-butoxycarbonyl or benzyloxycarbonyl. In the formula (b-1), Me is a methyl group.

Reaction C may be carried out usually in the presence of a base and a solvent. The compound of the formula (b-1) may be used in an amount of from 1 to 10 equivalents, preferably from 1 to 5 equivalents to the compound of the formula (I-3).

As the base, ones mentioned for the above Reaction A may be used. The base may be used in an amount of from 1 to 10 equivalents, preferably from 1 to 5 equivalents to the compound of the formula (I-3).

The solvent is not particularly limited so long as the reaction proceeds, and one or more may be properly selected from aromatic hydrocarbons such as benzene, toluene, xylene and chlorobenzene; aliphatic hydrocarbons such as carbon tetrachloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; polar aprotic solvents such as dimethyl sulfoxide, sulfolane, dimethylacetamide, N,N-dimethylformamide, N-methylpyrrolidone, pyridine, acetonitrile and propionitrile; ketones such as acetone and methyl ethyl ketone, etc.

The reaction temperature is usually from about 0 to about 150°C, preferably from about 50 to about 100°C, and the reaction time is usually from about 1 to about 24 hours, preferably from about 1 to about 12 hours.

### Reaction D

Reaction D is to convert the compound of the formula (I-4), using compound of the formula (b-2), compound of the formula (b-3) or a haloalkylating agent, to compound of the formula (I-5).

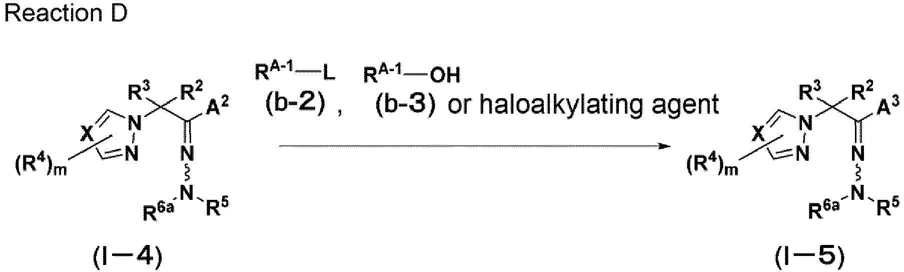

In the formulae, X, m, R², R³, R⁴, R⁵, R^{6a}, L and A² are as defined above, A³ is phenyl substituted by -OR^{A-1}, and R^{A-1} is alkyl or haloalkyl.

Reaction D may be carried out usually in the presence of a base and a solvent. Otherwise, it may be carried out in the presence of triphenylphosphine, diethyl azodicarboxylate and a solvent in accordance with conventional Mitsunobu Reaction. The haloalkylating agent may, for example, be bromodifluoromethyl trimethylsilane or Togni reagent. The compound of the formula (b-2), the compound of the formula (b-3) or the haloalkylating agent may be used in an amount of from 1 to 3 equivalents, preferably from 1 to 2 equivalents to the compound of the formula (I-4). Triphenylphosphine may be used in an amount of from 1 to 3 equivalents, preferably from 1 to 1.5 equivalents to the compound of the formula (I-4). Diethyl azodicarboxylate may be used in an amount of from 1 to 3 equivalents, preferably from 1 to 1.5 equivalents to the compound of the formula (I-4).

As the base, ones mentioned for the above Reaction A may be used. The base may be used in an amount of from 1 to 10 equivalents, preferably from 1 to 5 equivalents to the compound of the formula (I-4).

As the solvent, ones mentioned for the above Reaction B may be used.

The reaction temperature is usually from about -78 to about 100°C, preferably from about 0 to about 50°C, and the reaction time is usually from about 0.1 to about 24 hours, preferably from about 0.1 to about 12 hours.

### Reaction E

Reaction E is to deprotect the compound of the formula (I-5) into compound of the formula (I-6).

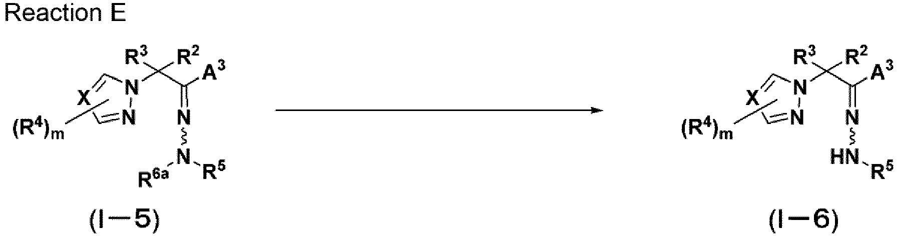

Symbols in the formulae are as defined above.

Reaction E may be carried out under conventional deprotection reaction conditions, for example, under acidic, oxidizing or catalytic reduction conditions. The reaction under acidic conditions may be carried out in usually in the presence of an acid and a solvent, and the acid may, for example, be hydrochloric acid or trifluoroacetic acid. The acid may be used in an amount of from 1 to 10 equivalents, preferably from 1 to 3 equivalents to the compound of the formula (I-5). The reaction under oxidizing conditions may be carried out usually in the presence of an oxidizing agent and a solvent. The oxidizing agent may, for example, be 2,3-dichloro-5,6-dicyano-1,4-benzoquinone. The oxidizing agent may be used in an amount of from 1 to 5 equivalents, preferably from 1 to 2 equivalents to the compound of the formula (I-5). The reaction under catalytic reduction conditions may be carried out usually in a hydrogen atmosphere in the presence of a catalyst and a solvent, and the catalyst may, for example, be platinum, platinum oxide, platinum black, Raney nickel, palladium, palladium-carbon, rhodium, or rhodium-aluminum. The catalyst may be used in an amount of from 0.01 to 1 equivalents, preferably from 0.1 to 1 equivalents to the compound of the formula (I-5).

As the solvent, ones mentioned for the above Reaction B may be used.

The reaction temperature is usually from about -30 to about 100°C, preferably from about 0 to about 50°, and the reaction time is usually from about 0.1 to about 48 hours, preferably from about 1 to about 24 hours.

### Reaction F

Reaction F is to convert compound of the formula (I-8) to compound of the formula (I-7).

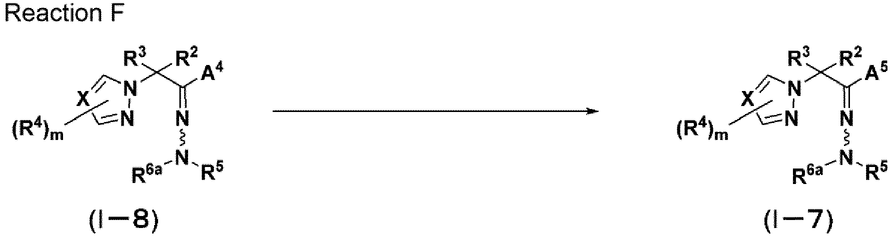

In the formulae, X, m, R², R³, R⁴, R⁵ and R^{6a} are as defined above. A⁴ is phenyl substituted by Y, and A⁵ is phenyl substituted by R^{A-2}. Y is halogen, more specifically a chlorine atom, a bromine atom or an iodine atom, and R^{A-2} is alkyl, alkenyl, alkynyl or cycloalkyl.

Reaction F may be carried out usually by reacting the compound of the formula (I-8) with an organometallic reagent in the presence of a transition metal catalyst, a solvent and an inert gas and as the case requires, in the presence of a base.

The organometallic reagent is not particularly limited so long as the reaction proceeds, and may, for example, be a Grignard reagent, an organozinc reagent or an organoboron compound. Such an organometallic reagent may be prepared in accordance with a known method, or a commercial product may be used. The organometallic reagent may be used in an amount of from 1 to 10 equivalents, preferably from 1 to 5 equivalents to the compound of the formula (I-8).

The transition metal catalyst may be a catalyst containing a transition metal such as palladium, rhodium, ruthenium, nickel, cobalt or molybdenum. As the transition metal catalyst, known ones having various structures used for a cross coupling reaction of an organic halide may be used, and for the present reaction, a transition metal catalyst containing palladium is particularly useful. For example, palladium-carbon, palladium chloride, palladium acetate, dichlorobis(acetonitrile) palladium, bis (dibenzylideneacetone)palladium, dichlorobis(triphenylphosphine)palladium, tetrakis(triphenylphosphine)palladium or dichlorobis(1,4-bis(diphenylphosphino)butane)palladium may be mentioned. Further, a tertiary phosphine or a tertiary phosphite may be used as the ligand as the case requires. The tertiary phosphine or the tertiary phosphite may, for example, be triphenylphosphine, phenyldimethylphosphine, tri-o-tolylphosphine, tri-p-tolylphosphine, 1,2-bis(diphenylphosphino)ethane, 1,3-bis(diphenylphosphino)propane, 1,4-bis(diphenylphosphino)butane, 1,1'-bis(diphenylphosphino)ferrocene or triphenyl phosphite. The transition metal catalyst may be used in an amount of from 0.001 to 0.5 equivalents, preferably from 0.05 to 0.2 equivalents to the compound of the formula (I-8). Further, the ligand may be used usually in an amount of from 1 to 50 equivalents, preferably from 1 to 10 equivalents to 1 equivalent of the transition metal catalyst. However, they may be used in amounts out of these ranges, depending upon the reaction conditions.

The base may, for example, be an alkali metal carbonate such as sodium carbonate, potassium carbonate or cesium carbonate; an alkali metal alkoxide such as sodium methoxide, sodium ethoxide or potassium tert-butoxide; an alkali metal hydrogencarbonate such as sodium hydrogencarbonate; an alkaline earth metal carbonate such as calcium carbonate; a metal hydroxide such as sodium hydroxide or potassium hydroxide; a metal hydride such as sodium hydride or potassium hydride; or an organic amine such as triethylamine, diisopropylethylamine, pyridine, 4-(N,N-dimethylamino)pyridine or imidazole. The base may be used in an amount of form 1 to 20 equivalents, preferably from 1 to 10 equivalents to the compound of the formula (I-8).

The solvent is not particularly limited so long as the reaction proceeds, and one or more may be properly selected from aromatic hydrocarbons such as benzene, toluene, xylene and chlorobenzene; aliphatic hydrocarbons such as carbon tetrachloride, chloroform, dichloromethane, dichloroethane, trichloroethane, hexane and cyclohexane; ethers such as dioxane, tetrahydrofuran, diethyl ether and dimethoxyethane; esters such as methyl acetate and ethyl acetate; polar aprotic solvents such as dimethyl sulfoxide, sulfolane, dimethylacetamide, N,N-dimethylformamide, N-methylpyrrolidone and pyridine; nitriles such as acetonitrile, propionitrile and acrylonitrile; ketones such as acetone and methyl ethyl ketone; alcohols such as methanol, ethanol, propanol and tert-butanol; water, etc.

The inert gas may, for example, be nitrogen gas or argon gas.

The reaction temperature is usually from about -100 to about 200°C, preferably from about -78 to about 100°C, and the reaction time is usually from about 0.5 to about 96 hours, preferably from about 1 to about 48 hours.

### Reaction G

Reaction G is to convert compound of the formula (I-10) to compound of the formula (I-9).

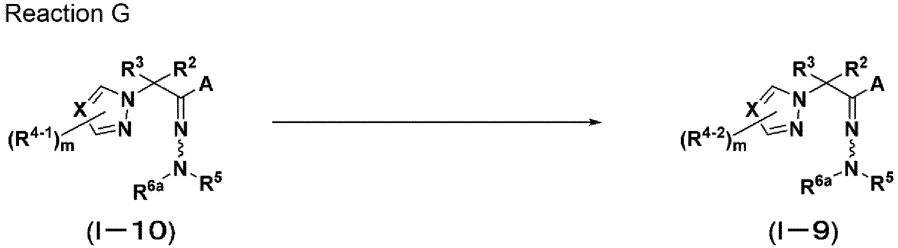

In the formulae, A, X, m, R², R³, R⁵ and R^{6a} are as defined above. R⁴⁻¹ is halogen, more specifically a chlorine atom, a bromine atom or an iodine atom. R⁴⁻² is alkyl, alkenyl, alkynyl or cycloalkyl.

Reaction G may be carried out in accordance with Reaction F.

### Reaction H

Reaction H is to react compound of the formula (5) with compound of the formula (6) or hydrazine to obtain the compound of the formula (2) or the compound of the formula (4).

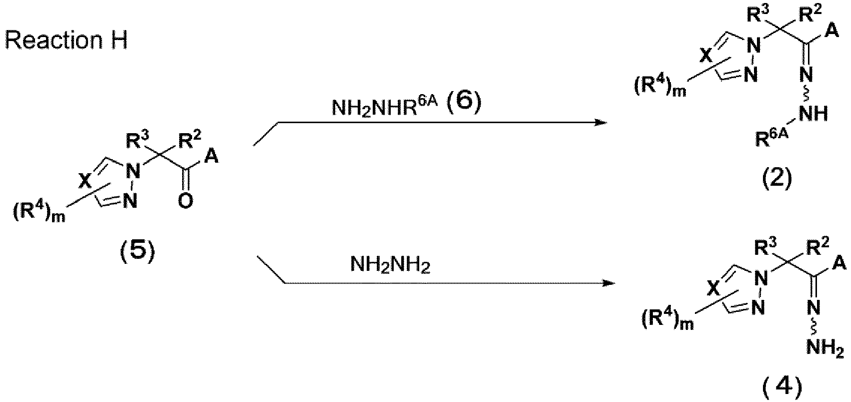

Symbols in the formulae are as defined above.

Reaction H may be carried out usually in the presence of a solvent, and as the case requires, in the presence of an acid also. The compound of the formula (6) or hydrazine may be used in an amount of from 1 to 10 equivalents, preferably from 1 to 3 equivalents to the compound of the formula (5).

The acid is not particularly limited so long as the reaction proceeds, and one or more may be properly selected from inorganic acids such as hydrochloric acid and sulfuric acid; organic acids such as acetic acid, propionic acid, methanesulfonic acid and p-toluenesulfonic acid, etc. The acid may be used in an amount of from 0.01 to 2 equivalents, preferably from 0.01 to 1 equivalents to the compound of the formula (5).

As the solvent, ones mentioned for the above Reaction B may be used.

The reaction temperature is usually from about 0°C to about 200°C, preferably from about 20°C to about 150°C. The reaction time is usually from about 1 hour to about 72 hours.

The compound of the formula (5) used in Reaction H may be produced by the method of the following Reaction I or Reaction J, for example by the method disclosed in e.g. U.S. Patent No. 4829075, or a method in accordance with such a method. The compound of the formula (6) may be produced in accordance with a known method, or a commercial product may be used.

### Reaction I

Reaction I is to react compound of the formula (7) with compound of the formula (8) to obtain the compound of the formula (5).

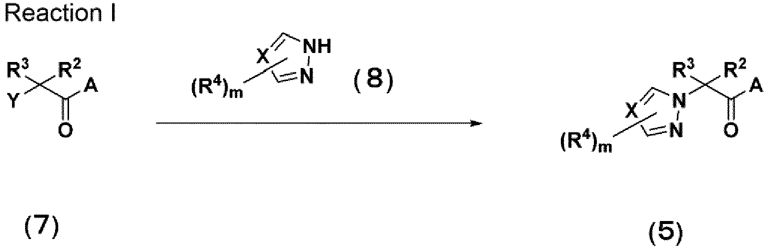

Symbols in the formulae are as defined above.

Reaction I may be carried out usually in the presence of a base and a solvent. As the base and the solvent, ones mentioned for the above Reaction B may be used. The compound of the formula (8) may be used in an amount of from 1 to 2 equivalents, preferably from 1 to 1.2 equivalents to the compound of the formula (7). The base may be used in an amount of from 0.5 to 3 equivalents, preferably from 0.5 to 1 equivalents to the compound of the formula (7).

The reaction temperature is usually from about 0°C to about 150°C, preferably from 0°C to 100°C. The reaction time is usually from about 1 hour to about 48 hours.

The compound of the formula (7) used in Reaction I may be produced, for example, by the method disclosed in e.g. JP-A-2021-035913 or JP-A-2021-035914 or a method in accordance with such a method, or a commercial product may be used. The compound of the formula (8) may be produced, for example, by the method disclosed in e.g. WO2010/029461, WO2015/095788 or WO2017/019804 or a method in accordance with such a method, or a commercial product may be used.

### Reaction J

Reaction J is to react compound of the formula (9) with compound of the formula (10) to obtain the compound of the formula (5).

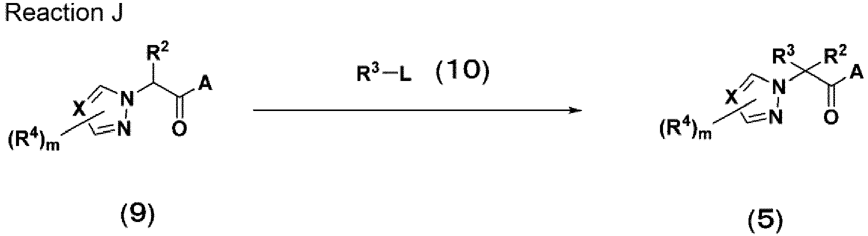

Symbols in the formulae are as defined above.

Reaction J may be carried out in accordance with Reaction B.

The reaction temperature is usually from about -78°C to about 100°C, preferably from about -78°C to about 80°C, and the reaction time is usually from about 1 hour to about 48 hours, preferably from about 1 hour to about 24 hours.

The compound of the formula (9) used in Reaction J may be produced in accordance with the following Reaction K. The compound of the formula (10) may be produced in accordance with a known method, or a commercial product may be used.

### Reaction K

Reaction K is to react compound of the formula (11) with the compound of the formula (8) to obtain the compound of the formula (9).

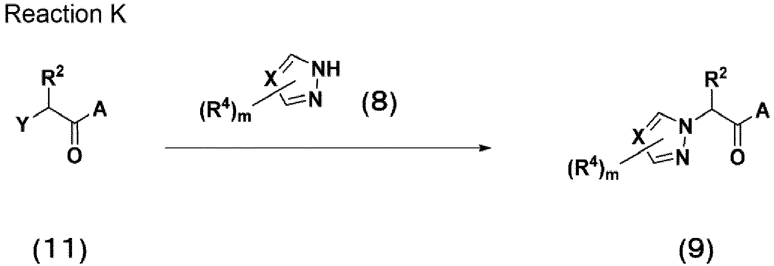

Symbols in the formulae are as defined above.

Reaction K may be carried out in accordance with Reaction I.

The compound of the formula (11) used in Reaction K may be produced, for example, by the method disclosed in e.g. WO2010/121022, European Journal of Medicinal Chemistry, vol. 148, pages 86 to 94 (2018) or a method in accordance with such a method, or a commercial product may be used.

### Reaction L

Reaction L is to react compound of the formula (12) with the compound of the formula (8) to obtain the compound of the formula (I-2).

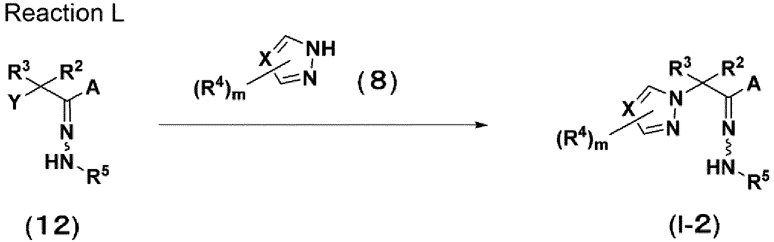

Symbols in the formulae are as defined above.

Reaction L may be carried out usually in the presence of a base and a solvent. As the solvent, ones mentioned for the above Reaction B may be used. The compound of the formula (8) may be used in an amount of from 1 to 2 equivalents, preferably from 1 to 1.2 equivalents to the compound of the formula (12).

As the base, one or more may be properly selected from alkali metals such as sodium and potassium; alkali metal alkoxides such as sodium methoxide, sodium ethoxide and potassium tert-butoxide; carbonates such as sodium carbonate and potassium carbonate; bicarbonates such as sodium bicarbonate and potassium bicarbonate; metal hydroxides such as sodium hydroxide and potassium hydroxide; metal hydrides such as sodium hydride and potassium hydride; amines such as monomethylamine, dimethylamine and triethylamine; pyridines such as pyridine and 4-dimethylaminopyridine; organolithium compounds such as methyllithium, n-butyllithium and lithium diisopropylamide; silicon compounds such as lithium bis(trimethylsilyl)amide, sodium bis(trimethylsilyl)amide and potassium bis(trimethylsilyl) amide, etc. The base may be used in an amount of from 0.5 to 5 equivalents, preferably from 1 to 2 equivalents to the compound of the formula (12).

The reaction temperature is usually from about -20°C to about 100°C, preferably from about 0°C to about 50°C. The reaction time is usually from about 0.1 hour to about 48 hours, preferably from about 0.5 hour to about 24 hours.

The compound of the formula (12) used in Reaction L may be produced in accordance with the method of the following Reaction M.

### Reaction M

Reaction M is to react compound of the formula (13) with a halogenating agent to obtain the compound of the formula (12).

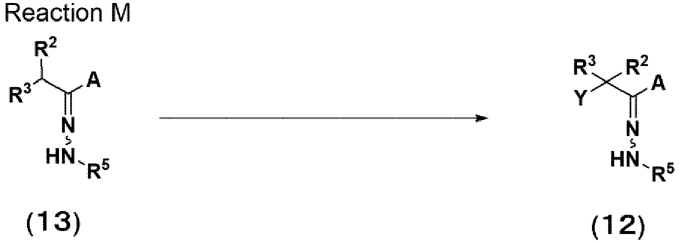

Symbols in the formulae are as defined above.

Reaction M may be carried out usually in the presence of a halogenating agent (for example, a chlorinating agent, a brominating agent or an iodinating agent) and a solvent, and in the presence of a base as the case requires. The halogenating agent may be used in an amount of from 1 to 2 equivalents, preferably from 1 to 1.5 equivalents to the compound of the formula (13).

As the chlorinating agent, one or more may be properly selected from chlorine, N-chlorosuccinimide, sulfuryl chloride, etc., as the brominating agent, one or more may be properly selected from bromine, N-bromosuccinimide, trimethylphenylammonium tribromide, etc., and as the iodinating agent, one or more may be properly selected from iodine, N-iodosuccinimide, etc.

The solvent is not particularly limited so long as the reaction proceeds, and one or more may be properly selected from aromatic hydrocarbons such as benzene, toluene, xylene and chlorobenzene; aliphatic hydrocarbons such as hexane, heptane, petroleum ether, ligroin and cyclohexane; halogenated hydrocarbons such as chloroform, dichloromethane, carbon tetrachloride and 1,2-dichloroethane; esters such as methyl formate, ethyl formate, methyl acetate and ethyl acetate; ethers such as dioxane, tetrahydrofuran, diethyl ether and dimethoxyethane; polar aprotic solvents such as dimethyl sulfoxide, sulfolane, dimethylacetamide, N,N-dimethylformamide, N-methylpyrrolidone and pyridine; organic acids such as acetic acid and propionic acid; water, etc.

The base may, for example, be lithium diisopropylamide. The base may be used in an amount of from 1 to 2 equivalents, preferably from 1 to 1.2 equivalents to the compound of the formula (13).

In a case where the reaction is carried out in the presence of a base, usually as the solvent, one or more may be properly selected from ethers such as tetrahydrofuran and diethyl ether.

Reaction M may be carried out in the presence of an organic acid such as acetic acid or propionic acid or a Lewis acid such as aluminum chloride as a catalyst, as the case requires. Further, an organic acid used as the solvent in excess may function also as a catalyst.

The reaction temperature is usually from about -100°C to about 150°C, preferably from about -78°C to about 100°C. The reaction time is usually from about 0.1 hour to about 48 hours, preferably from about 0.5 to about 24 hours. In a case where the reaction is carried out in the presence of a base, the reaction temperature is usually from about -100°C to about 50°C, preferably from about -78°C to about 20°C, and the reaction time is usually from about 0.1 to about 12 hours, preferably from about 0.5 to about 6 hours. In a case where the reaction is carried out in the presence of an acid, the reaction temperature is usually from about 0°C to about 150°C, preferably from about 20°C to about 120°C, and the reaction time is usually from about 0.1 to about 48 hours, preferably from about 1 to about 24 hours.

The compound of the formula (13) used in Reaction M may be produced in accordance with the method of the following Reaction N or Reaction O.

### Reaction N

Reaction N is to react compound of the formula (14) with a formylating agent to obtain the compound of the formula (13).

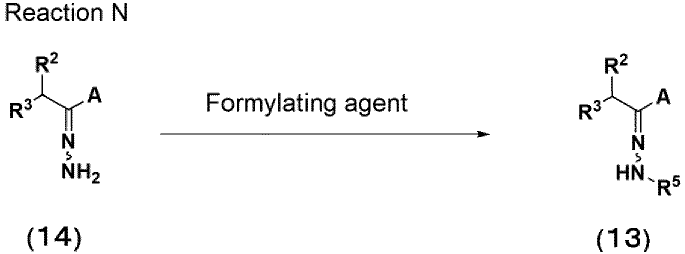

Symbols in the formulae are as defined above.

Reaction N may be carried out in accordance with Reaction A. As the formylating agent used in Reaction N, the compounds mentioned for the above Reaction A may be used.

The compound of the formula (14) used in Reaction N may be produced in accordance with the method of the following Reaction O.

### Reaction O

Reaction O is to react compound of the formula (15) with compound of the formula (16) or hydrazine to obtain the compound of the formula (13) or the compound of the formula (14).

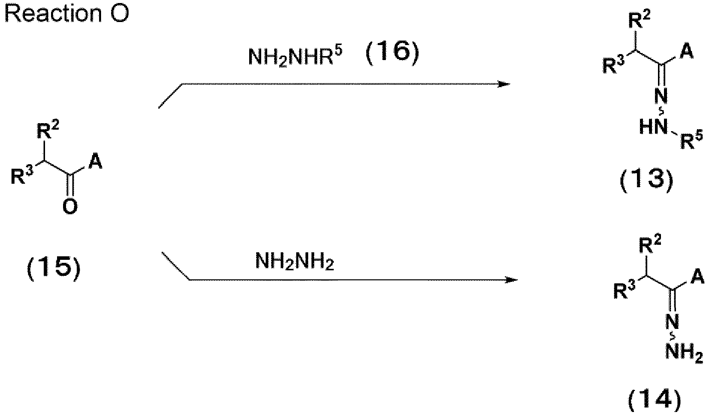

Symbols in the formulae are as defined above.

Reaction O may be carried out in accordance with Reaction H.

The compound of the formula (15) used in Reaction O may be produced, for example, by the method disclosed in e.g. JP-A-2021-035913, JP-A-2021-035914 or WO2006/016708 or a method in accordance with such a method, or a commercial product may be used. The compound of the formula (16) may be produced in accordance with a known method, or a commercial product may be used.

Now, the application site, the application manner, the treatment method, etc., of the present composition will be described.

Nematodes can be controlled by applying an effective amount of the present composition directly to nematodes and/or to soil or a plant body to be inhabited by nematodes. The soil or the plant body to be inhabited by nematodes includes not only one inhabited by nematodes but also one which is expected to be inhabited by nematodes in future. The plant body may be above-ground parts (e.g. stems, leaves, trunks), below-ground parts (e.g. roots) or seeds of a plant to which the present composition is to be applied. The seeds mean diaspores (so-called seeds) formed by sexual reproduction, plant organs (e.g. tubers, scaly bulbs, corms, rhizomes, seed tubers), and scions of fruit trees, flowering trees, flowers and ornamental plants (for example, branches of grape which is a fruit tree).

The present composition is useful to control nematodes belonging to genera Meloidogyne, Pratylenchus, Ditylenchus, Heterodera, Globodera, Aphelenchoides, Aphelenchus, Radopholus, Tylenchulus, Rotylenchulus, Rotylenchus, Anguina, Belonolaimus, Bursaphelenchus, Criconema, Criconemoides, Mesocriconema, Dolichodorus, Helicotylenchus, Hirschmanniella, Hoplolaimus, Nacobbus, Longidorus, Scutellonema, Trichodorus, Paratrichodorus, Tylenchorhynchus, Xiphinema, etc., and is particularly effective to control nematodes belonging to genera Meloidogyne, Pratylenchus, Heterodera, Globodera, etc.

As specific examples of the nematodes, the following may be mentioned.

Meloidogyne nematodes such as Meloidogyne incognita, Meloidogyne hapla, Meloidogyne arenaria and Meloidogyne javanica, Pratylenchus nematodes such as Pratylenchus penetrans, Pratylenchus coffeae, Pratylenchus loosi and Pratylenchus vulnus, Ditylenchus nematodes such as Ditylenchus destructor and Ditylenchus dipsaci, Heterodera nematodes such as Heterodera glycines, Heterodera schachtii and Heterodera trifolii, Globodera nematodes such as Globodera rostochiensis and Globodera pallida, Aphelenchoides nematodes such as Aphelenchoides besseyi, Aphelenchoides fragariae and Aphelenchoides ritzemabosi, Aphelenchus nematodes such as Aphelenchus avenae, Radopholus nematodes such as Radopholus similis, Tylenchulus nematodes such as Tylenchulus semipenetrans, Rotylenchulus nematodes such as Rotylenchulus reniformis, Rotylenchus nematodes such as Rotylenchus robustus, Anguina nematodes such as Anguina tritici, Belonolaimus nematodes such as Belonolaimus longicaudatus, Bursaphelenchus nematodes such as Bursaphelenchus xylophilus and Bursaphelenchus cocophilus, Criconema nematodes such as Criconema jaejuense and Criconema palliatum, Criconemoides nematodes such as Criconemoides informis and Criconemoides morgensis, Mesocriconema nematodes such as Mesocriconema xenoplax and Mesocriconema curvatum, Dolichodorus nematodes such as Dolichodorus heterocephalus, Helicotylenchus nematodes such as Helicotylenchus pseudorobustus and Helicotylenchus multicinctus, Hirschmanniella nematodes such as Hirschmanniella diversa and Hirschmanniella oryzae, Hoplolaimus nematodes such as Hoplolaimus columbus and Hoplolaimus magnistylus, Nacobbus nematodes such as Nacobbus aberrans and Nacobbus dorsalis, Longidorus nematodes such as Longidorus martini and Longidorus diadecturus, Scutellonema nematodes such as Scutellonema brachyurum and Scutellonema bradys, Trichodorus nematodes such as Trichodorus cedarus, Paratrichodorus nematodes such as Paratrichodorus porosus and Paratrichodorus minor, Tylenchorhynchus nematodes such as Tylenchorhynchus claytoni, Xiphinema nematodes such as Xiphinema index and Xiphinema americanum, etc.

The plants to which the present composition is applied are not particularly limited so long as they are agriculturally and horticulturally useful plants, and may, for example, be fruit vegetables such as tomato, cherry tomato, sweet pepper, eggplant, cucumber, zucchini, watermelon, melon, pumpkin, okra, hot pepper, Oriental pickling melon, wax gourd, bitter melon and Oriental melon, leaf vegetables such as lettuce, leaf lettuce, spinach, cabbage, onion, garlic, asparagus, broccoli, cauliflower, Chinese cabbage, Malabar spinach, Welsh onion, Potherb mustard, Perilla, Komatsuna and celery, root vegetables such as potato, sweet potato, Japanese radish, carrot, burdock, Lotus root, Eddoe, Konjac, Japanese yam, ginger, turnip, Allium chinense, Myoga and Cirsium dipsacolepis, beans such as soybean, adzuki bean, pea, kidney bean, peanut, broad bean and Edamame, cereal crops such as rice, wheat, oat, rye and corn, fruit trees, fruits and nuts such as apple, citrus fruits, pear, grape, strawberry, walnut, almond, banana, fig, pineapple, peach, nectarine, cherry, apricot, coconut, avocado, persimmon, plum, pistachio, kiwifruit and loquat, lawn such as Zoysia tenuifolia and bentgrass, flowers and ornamental plants such as daisy, lily, rose, carnation, dahlia, gerbera, saintpaulia, tulip, daffodil, petunia, snowflake, peony, cyclamen, rose balsam and eustoma, trees and flowering trees such as pine, Cryptomeria, cypress and azalea, industrial crops such as cotton, rapeseed, beet, tea, tobacco, coffee, olive, sugar cane, hop, sesame, parsley, thymus, rosemary, basil and lavender, foliage plants such as orchidaceae, cactus and fern, and Pasture plants such as clover, alfalfa, orchard grass and Johnson grass.

The useful crops in the present invention include plants having, imparted by classical breeding methods, resistance to a herbicide (for example, a HPPD inhibitor such as isoxaflutole; an ALS inhibitor such as imazethapyr or thifensulfuron-methyl; an EPSP synthase inhibitor such as glyphosate; a glutamine synthase inhibitor such as glufosinate; an acetyl CoA carboxylase inhibitor such as sethoxydim; bromoxynil; dicamba; or 2,4-D). The useful crops in the present invention further include transgenic plants generated by gene recombination or gene editing. Examples of the transgenic plants include herbicide-resistant transgenic plants, noxious insect-resistant transgenic plants, transgenic plants relating to plant components, and phytopathogen-resistant transgenic plants.

The present composition may be obtained by mixing the compound (I) with various agricultural and horticultural additives and applied in the form of various formulations such as dusts, granules, water dispersible granules, wettable powders, water-based suspensions, oil-based suspensions, water soluble powders, emulsifiable concentrates, liquid, pastes, aerosols, or ultra low-volume formulations. It may be formed into any usual formulation used in this field, so long as the object of the present invention is thereby met. Above all, from the viewpoint of the method of soil application, granules, water-based suspensions, oil-based suspensions, emulsifiable concentrates or liquid are preferable.

The additives to be used for the formulation include, for example, a solid carrier such as diatomaceous earth, slaked lime, calcium carbonate, talc, white carbon, kaoline, bentonite, kaolinite, sericite, clay, sodium carbonate, sodium bicarbonate, salt cake, zeolite or starch; a solvent such as water, toluene, xylene, solvent naphtha, dioxane, acetone, isophorone, methyl isobutyl ketone, chlorobenzene, cyclohexane, dimethyl sulfoxide, N,N-dimethylformamide, N,N-dimethylacetamide, N-methyl-2-pyrrolidone or an alcohol; an anionic surfactant such as a salt of fatty acid, a benzoate, an alkylsulfosuccinate, a dialkylsulfosuccinate, a polycarboxylate, a salt of alkylsulfuric acid ester, an alkyl sulfate, an alkylaryl sulfate, an alkyl diglycol ether sulfate, a salt of alcohol sulfuric acid ester, an alkyl sulfonate, an alkylaryl sulfonate, an aryl sulfonate, a lignin sulfonate, an alkyldiphenyl ether disulfonate, a polystyrene sulfonate, a salt of alkylphosphoric acid ester, an alkylaryl phosphate, a styrylaryl phosphate, a salt of polyoxyethylene alkyl ether sulfuric acid ester, a polyoxyethylene alkylaryl ether sulfate, a salt of polyoxyethylene alkylaryl ether sulfuric acid ester, a polyoxyethylene alkyl ether phosphate, a salt of polyoxyethylene alkylaryl phosphoric acid ester or a naphthalene sulfonate condensed with formaldehyde; a nonionic surfactant such as a sorbitan fatty acid ester, a glycerin fatty acid ester, a fatty acid polyglyceride, a fatty acid alcohol polyglycol ether, acetylene glycol, acetylene alcohol, an oxyalkylene block polymer, a polyoxyethylene alkyl ether, a polyoxyethylene alkylaryl ether, a polyoxyethylene styrylaryl ether, a polyoxyethylene glycol alkyl ether, a polyethylene glycol, a polyoxyethylene fatty acid ester, a polyoxyethylene sorbitan fatty acid ester, a polyoxyethylene glycerin fatty acid ester, a polyoxyethylene hydrogenated castor oil or a polyoxypropylene fatty acid ester; and a vegetable oil or mineral oil such as olive oil, kapok oil, castor oil, palm oil, camellia oil, coconut oil, sesame oil, corn oil, rice bran oil, peanut oil, cottonseed oil, soybean oil, rapeseed oil, linseed oil, tung oil or liquid paraffins. These additive components may suitably be selected for use alone or in combination as a mixture of two or more of them, so long as the object of the present invention is met. Further, in addition to the above, various additives known in this technical field, such as a filler, a thickener, an anti-settling agent, an anti-freezing agent, a dispersion stabilizer, a safener and an anti-mold agent, may be used. The mixing ratio of the compound (I) to such various additives (compound (I):additives) is usually from 0.001:99.999 to 95:5, preferably from 0.005:99.995 to 90:10 in weight ratio. In the actual application of such a formulation, it may be used as it is, or may be diluted to a predetermined concentration with a diluent such as water, and may be mixed with various spreaders (e.g. surfactants, vegetable oils or mineral oils), as the case requires.

The application of the present composition cannot generally be defined, as it varies depending upon the weather conditions, the type of the formulation, the plants to be treated, the application season, the application site, the type or degree of outbreak of nematodes, etc. However, the present composition or a diluted product thereof may be applied by a conventional application method, that is soil treatment, foliar treatment, irrigation treatment, seed treatment, trunk injection, nursery box application, etc. In the case of soil treatment, foliar treatment or irrigation treatment, the application amount is such that the amount of the present composition is usually from about 0.01 g to about 50,000 g, preferably from about 1 g to about 30,000 g, per hectare. In the case of seed treatment, the application amount is such that the amount of the present composition is from about 0.01 g to about 200 g, preferably from about 0.05 g to about 100 g per 1 kg of seeds. In the case of nursery box application, the application amount is such that the amount of the present composition is from about 0.001 g to about 3,000 g, preferably from about 0.01 g to about 1,000 g per nursery box. In the case of trunk injection, the application amount is such that the amount of the present composition is from about 0.001 g to about 5,000 g, preferably from about 0.01 g to about 3,000 g per tree.

Soil treatment may, for example, be a method wherein the present composition is formulated into a liquid formulation (liquid, water-based suspension, oil-based suspension, emulsifiable concentrate or the like) or a solid formulation (granules, dusts, wettable powder, water soluble powder, water dispersible granules, water soluble granules or the like), and the formulation as it is or after diluted with water is applied over the entire surface of the soil and mixed, before planting of the plant body or before sowing; the formulation is spread into holes made to plant the plant body (planting holes); or the formulation is spread into furrows made with a certain width for sowing or planting of the plant body.

Foliar treatment may, for example, be a method wherein the present composition is formulated into a liquid formulation (liquid, water-based suspension, oil-based suspension, emulsifiable concentrate or the like) or a solid formulation (wettable powder, water soluble powder, water dispersible granules, water soluble granules or the like), and the formulation is spread after diluted with water to the entire plant body.

Irrigation treatment may, for example, be a method wherein the present composition is formulated into a liquid formulation (liquid, water-based suspension, oil-based suspension or the like) or a solid formulation (wettable powder, water soluble powder, water dispersible granules, water soluble granules or the like), and the formulation as it is or after diluted with water is applied by irrigation to a seedling container, to the plant foot during cultivation, or to a vicinity thereof.

Seed treatment may, for example, be a method wherein the present composition is formulated into a liquid formulation (water-based suspension, oil-based suspension, emulsifiable concentrate, liquid or the like) or a solid formulation (dusts, wettable powder, water soluble powder, water dispersible granules, water soluble granules or the like), and the formulation as it is or after diluted with water is mixed with seeds to attach the present composition to the surface of the seeds; the formulation is mixed with seeds together with a coating material and attached to the surface of the seeds; the formulation is sprayed and attached to the surface of seeds; or seeds are dipped in the formulation and infiltrated with the chemical.

Nursery box application may, for example, be a method wherein the present composition is formulated into a solid formulation (granules, dusts or the like) or a liquid formulation (water-based suspension, oil-based suspension, liquid or the like), and the formulation is applied as it is or after diluted with water to a nursery box before sowing, after sowing, before and after sowing, at the time of cultivation in the nursery box, or before planting into paddies; or a method wherein when the seeds are into a nursery box, the present composition is mixed with culture soil, for example, the present composition formulated into a solid formulation (granules, dusts, water dispersible granules or the like) and mixed with the entire seedbed soil, cover soil or culture soil.

Trunk injection may, for example, be a method wherein the present composition is formulated into a liquid formulation (liquid or the like) and is injected as it is into a plant body through a hole made on the trunk.

The present composition may be mixed with or may be used in combination with other components selected from other agricultural and horticultural chemicals, fertilizers and phytotoxicity-reducing agents, whereby more excellent effects or activities may sometimes be obtained.

Mixing with or use in combination with other components means that the present composition and other components are used simultaneously, separately or with an interval.

Such other agricultural and horticultural chemicals include, for example, a herbicide, an insecticide, a miticide, a nematicide, a soil pesticide, a fungicide, an antivirus agent, an attractant, an antibiotic, a plant hormone, a plant growth regulator, and so on. Especially, a mixed nematicidal composition having the present composition mixed with or used in combination with one or more active ingredient compounds of other insecticide and/or nematocide, may improve the application range, the application time, the pesticidal activities, etc. to preferred directions. The present composition and the active ingredient compounds of other insecticide and/or nematicide may separately be formulated so that they are mixed for use at the time of application, or they may be formulated together. The present invention includes such a mixed nematicidal composition.

The mixing weight ratio of the compound (I) to the active ingredient compounds of other insecticide and/or nematicide (compound (I): active ingredient compounds) can not generally be defined as it varies depending upon the weather conditions, the types of formulations, the crop plants to be treated, the application time, the application site, the types or degree of outbreak of nematodes, etc., but it is usually within a range of from 1:300 to 300:1, preferably from 1:100 to 100:1. Further, the dose for the application is such that the total amount of the active ingredient compounds is from 0.1 to 70,000 g, preferably from 5 to 50,000 g, per hectare. The present invention includes a method for controlling nematodes, and in some cases, nematodes and insects, by application of such a mixed nematicidal composition.

More specific examples of the pests are as follows.

Aphids such as green peach aphid (Myzus persicae) and cotton aphid (Aphis gossypii); Lepidoptera insects such as diamondback moth (Plutella xylostella), cabbage armyworm (Mamestra brassicae), cotton leafworm (Spodoptera litura), codling moth (Cydia pomonella), bollworm (Heliothis zea), tobacco budworm (Helicoverpa armigera), gypsy moth (Lymantria dispar), rice leafroller (Cnaphalocrocis medinalis), smaller tea tortrix (Adoxophyes sp.), black cutworm (Aarotis ipsilon) and turnip moth (Agrotis seqetum); Coleoptera insects such as colorado potato beetle (Leptinotarsa decemlineata), cucurbit leaf beetle (Aulacophora femoralis) and scarabs; planthoppers such as brown planthopper (Nilaparvata lugens) and whitebacked planthopper (Sogatella furcifera); leafhoppers; scale insects; bugs; whiteflies such as tobacco whitefly (Bemisia tabaci); thrips; grasshoppers; Anthomyiidae flies; ants; gastropods such as slugs and snails; plant parasitic mites such as two-spotted spider mite (Tetranychus urticae), carmine spider mite (Tetranychus cinnabarinus), kanzawa spider mite (Tetranychus kanzawai), citrus red mite (Panonychus citri), European red mite (Panonychus ulmi), broad mite (Polyphagotarsonemus latus), pink citrus rust mite (Aculops pelekassi), bulb mite (Rhizoglyphus echinopus) and Acaridae; and isopods such as pill bugs (Armadillidium vulgare) and woodlouses (Porcellio scaber).

When the nematicidal composition containing the compound (I) is applied, it may be used in combination with other agricultural and horticultural chemicals, such as a fungicide, an insecticide, a miticide, a nematicide, a soil insect pesticide, an antivirus agent, an attractant, a herbicide and a plant growth regulator.

The active ingredient compounds of an insecticide, a nematicide, a miticide or a soil insect pesticide, in the above-mentioned other agricultural and horticultural chemicals may properly be selected, for example, from the following group of compounds (by common names or test codes of Japan Plant Protection Association). In a case where these compounds have their salts, alkyl esters, various structural isomers such as optical isomers, etc., all of them are included, even if no specific disclosure thereof is made.

Organic phosphate compounds, such as profenofos, dichlorvos, fenamiphos, fenitrothion, EPN (O-ethyl-O-4-nitrophenyl phenylphosphonothioate), diazinon, chlorpyrifos, chlorpyrifos-methyl, acephate, prothiofos, fosthiazate, cadusafos, disulfoton, isoxathion, isofenphos, ethion, etrimfos, quinalphos, dimethylvinphos, dimethoate, sulprofos, thiometon, vamidothion, pyraclofos, pyridaphenthion, pirimiphos-methyl, propaphos, phosalone, formothion, malathion, tetrachlorvinphos, chlorfenvinphos, cyanophos, trichlorfon, methidathion, phenthoate, ESP (oxydeprofos), azinphos-methyl, fenthion, heptenophos, methoxychlor, parathion, phosphocarb, demeton-S-methyl, monocrotophos, methamidophos, imicyafos, parathion-methyl, terbufos, phosphamidon, phosmet and phorate;
carbamate compounds, such as carbaryl, propoxur, aldicarb, carbofuran, thiodicarb, methomyl, oxamyl, ethiofencarb, pirimicarb, fenobucarb, carbosulfan, benfuracarb, bendiocarb, furathiocarb, isoprocarb, metolcarb, xylylcarb, XMC (3,5-xylyl methylcarbamate) and fenothiocarb;
nereistoxin derivatives, such as cartap, thiocyclam, thiocyclam hydrochloride, bensultap, thiosultap, monosultap (another name: thiosultap-monosodium), bisultap (another name: thiosultap-disodium) and polythialan;
organic chlorine compounds, such as dicofol, tetradifon, endosulfan, dienochlor and dieldrin;
pyrethroid compounds, such as fenvalerate, permethrin, cypermethrin, alpha-cypermethrin, zeta-cypermethrin, theta-cypermethrin, beta-cypermethrin, deltamethrin, cyhalothrin, gamma-cyhalothrin, lambda-cyhalothrin, tefluthrin, kappa-tefluthrin, ethofenprox, flufenprox, cyfluthrin, beta-cyfluthrin, fenpropathrin, flucythrinate, fluvalinate, cycloprothrin, pyrethrins, esfenvalerate, tetramethrin, resmethrin, protrifenbute, bifenthrin, kappa-bifenthrin, acrinathrin, allethrin, tau-fluvalinate, tralomethrin, profluthrin, metofluthrin, epsilon-metofluthrin, heptafluthrin, phenothrin, flumethrin, momfluorothrin and silafluofen;
benzoylurea compounds, such as diflubenzuron, chlorfluazuron, teflubenzuron, flufenoxuron, lufenuron, novaluron, triflumuron, hexaflumuron, bistrifluron, noviflumuron, fluazuron and flufenoxuron;
juvenile hormone-like compounds, such as methoprene, pyriproxyfen, fenoxycarb and diofenolan;
pyridazinone compounds, such as pyridaben;
pyrazole compounds, such as fenpyroximate, fipronil, ethiprole, acetoprole, pyrafluprole, pyriprole, cyenopyrafen, flufiprole, tebufenpyrad and tolfenpyrad;
diamide compounds, such as chlorantraniliprole, cyantraniliprole, cyclaniliprole, tetraniliprole, flubendiamide, tetrachlorantraniliprole, cyhalodiamide, fluchlordiniliprole and tiorantraniliprole;
neonicotinoid compounds, such as imidacloprid, nitenpyram, acetamiprid, thiacloprid, thiamethoxam, clothianidin, dinotefuran and nithiazine;
hydrazine compounds, such as tebufenozide, methoxyfenozide, chromafenozide and halofenozide;
pyridine compounds, such as pyridalyl and flonicamid;
tetronic acid compounds, such as spirodiclofen, spiromesifen and spirobudifen;
tetramic acid compounds, such as spirotetramat and spiropidion;
strobilurin compounds, such as fluacrypyrim and pyriminostrobin;
pyrimidinamine compounds, such as flufenerim and pyrimidifen;
triazine compounds, such as cyromazine;
hydrazone compounds, such as hydramethylnon;
thiourea compounds, such as diafenthiuron and chloromethiuron;
formamidine compounds, such as amitraz, chlordimeform and chloromebuform;
pyridine azomethine compounds, such as pymetrozine and pyrifluquinazone;
isooxazoline compounds, such as afoxolaner, fluralaner, fluxametamide and sarolaner; and
other compounds, such as buprofezin, hexythiazox, triazamate, chlorfenapyr, indoxacarb, acequinocyl, etoxazole, 1,3-dichloropropene, benclothiaz, bifenazate, propargite, clofentezine, metaflumizone, cyflumetofen, fenazaquin, amidoflumet, sulfluramid, hydramethylnon, metaldehyde, sulfoxaflor, fluensulfone, verbutin, dicloromezotiaz, triflumezopyrim, fluhexafon, tioxazafen, afidopyropen, flometoquin, flupyradifurone, fluazaindolizine, acynonapyr, benzpyrimoxan, flupyrimin, oxazosulfyl, tyclopyrazoflor, broflanilide, isocycloseram, dimpropyridaz, flupentiofenox, nicofluprole, cyproflanilide, spidoxamat, cyclobutrifluram, fenmezoditiaz and trifluenfuronate.

Further, the compound of the present invention may be mixed with or used in combination with the following compounds.

Microbial pesticides, such as insecticidal crystal proteins produced by Bacillus thuringiensis aizawai, Bacillus thuringiensis kurstaki, Bacillus thuringiensis israelensis, Bacillus thuringiensis japonensis, Bacillus thuringiensis tenebrionis, Bacillus thuringiensis, Paecilomyces lilacinus, Bacillus methylotrophicus, Bacillus subtilis, Bacillus amyloliquefaciens, Bacillus licheniformis, Bacillus firmus, Pasteuria nishizawae, Myrothecium verrucaria, Burkholderia rinojensis or Chromobacterium subtsugae, insect viruses, entomopathogenic fungi, and nematophagous fungi; and
antibiotics or semisynthetic antibiotics, such as abamectin, emamectin benzoate, ivermectin, milbemectin, milbemycin oxime, lepimectin, spinosad and spinetoram.

The active ingredient compounds of a fungicide in the above-mentioned other agricultural and horticultural chemicals may properly be selected, for example, from the following group of compounds (by common names or test codes of Japan Plant Protection Association). In a case where these compounds have their salts, alkyl esters, various structural isomers such as optical isomers, etc., all of them are included, even if no specific disclosure thereof is made.

Anilinopyrimidine compounds such as mepanipyrim, pyrimethanil and cyprodinil;
triazolopyrimidine compounds such as ametoctradin;
pyridinamine compounds such as fluazinam;
azole compounds such as triadimefon, bitertanol, triflumizole, etaconazole, propiconazole, penconazole, flusilazole, myclobutanil, cyproconazole, tebuconazole, hexaconazole, furconazole-cis, prochloraz, metconazole, epoxiconazole, tetraconazole, oxpoconazole fumarate, prothioconazole, triadimenol, flutriafol, difenoconazole, fluquinconazole, fenbuconazole, bromuconazole, diniconazole, tricyclazole, simeconazole, pefurazoate, ipconazole, imibenconazole, azaconazole, triticonazole, imazalil, ipfentrifluconazole and mefentrifluconazole;
quinoxaline compounds such as quinomethionate;
dithiocarbamate compounds such as maneb, zineb, mancozeb, polycarbamate, metiram, propineb and thiram;
organic chlorine compounds such as fthalide, chlorothalonil and quintozene;
imidazole compounds such as benomyl, thiophanate-methyl, carbendazim, thiabendazole and fuberiazole;
cyanoacetamide compounds such as cymoxanil;
acyl amino acid compounds such as metalaxyl, metalaxyl-M (another name: mefenoxam), oxadixyl, ofurace, benalaxyl, benalaxyl-M (another name: kiralaxyl, chiralaxyl), furalaxyl and valifenalate;
anilide compounds such as cyprofuram, carboxin, oxycarboxin, thifluzamide, boscalid, fenhexamid, isotianil, tiadinil and pyraziflumid;
sulfamide compounds such as dichlofluanid;
copper compounds such as cupric hydroxide, oxine copper, anhydrous copper sulfate, copper nonylphenol sulfonate, copper 8-hydroxyquinoline and dodecylbenzenesulfonic acid bisethylenediamine copper(II) salt (another name: DBEDC);
organophosphorus compounds such as fosetyl-Al, tolclofos-Methyl, edifenphos and iprobenfos;
phthalimide compounds such as captan, captafol and folpet;
dicarboxyimide compounds such as procymidone, iprodione and vinclozolin;
benzanilide compounds such as flutolanil, mepronil and benodanil;
amide compounds such as carpropamid, diclocymet, silthiopham and fenoxanil;
pyrazolecarboxamide compounds such as benzovindiflupyr, bixafen, fluindapyr, fluxapyroxad, furametpyr, isopyrazam, penflufen, penthiopyrad, pydiflumetofen, sedaxane, isoflucypram, inpyrfluxam and pyrapropoyne;
benzamide compounds such as fluopicolide, fluopyram, zoxamide and fluopimomide;
furanilide compounds such as fenfuram;
thiophenamide compounds such as isofetamid;
piperazine compounds such as triforine;
pyridine compounds such as pyrifenox, pyrisoxazole and aminopyrifen;
pyrimidine compounds such as fenarimol, ferimzone and nuarimol;
piperidine compounds such as fenpropidin;
morpholine compounds such as fenpropimorph and tridemorph;
organic tin compounds such as fentin hydroxide and fentin acetate;
urea compounds such as pencycuron;
carboxylic acid amide compounds such as dimethomorph, flumorph, pyrimorph, iprovalicarb, benthiavalicarb-isopropyl and mandipropamid;
phenylcarbamate compounds such as diethofencarb;
cyanopyrrole compounds such as fludioxonil and fenpiclonil;
strobilurin compounds such as azoxystrobin, kresoxim-methyl, metominostrobin, trifloxystrobin, picoxystrobin, oryzastrobin, dimoxystrobin, pyraclostrobin, fluoxastrobin, enestroburin, pyraoxystrobin, pyrametostrobin, coumoxystrobin, enoxastrobin, fenaminstrobin, flufenoxystrobin, triclopyricarb and mandestrobin;
oxazole compounds such as famoxadone and oxathiapiprolin;
thiazolecarboxamide compounds such as ethaboxam;
imidazolinone compounds such fenamidone;
benzenesulfonamide compounds such as flusulfamide;
oxime ether compounds such as cyflufenamid;
anthraquinone compounds such as dithianon;
crotonic acid compounds such as meptyldinocap;
antibiotics such as validamycin, kasugamycin, streptomycin and polyoxins;
guanidine compounds such as iminoctadine and dodine;
quinoline compounds such as tebufloquin, quinoxyfen, quinofumelin and ipflufenoquin;
thiazolidine compounds such as flutianil;
carbamate compounds such as propamocarb hydrochloride, pyribencarb and tolprocarb;
tetrazole compounds such as picarbutrazox and metyltetraprole;
sulfonamide compounds such as amisulbrom and cyazofamid;
allyl phenyl ketone compounds such as metrafenone and pyriofenone;
benzothiazole compounds such as probenazole and dichlobentiazox;
phenylpyrazole compounds such as fenpyrazamine;
dithiolane compounds such as isoprothiolane;
picolinamide compounds such as fenpicoxamid and florylpicoxamid;
sulfur compounds such as sulfur and lime sulfur;
other compounds such as pyroquilon, diclomezine, chloropicrin, dazomet, metam-sodium, proquinazid, spiroxamine and dipymetitrone;
Microbial fungicides such as Bacillus amyloliqefaciens strain QST713, Bacillus amyloliqefaciens strain FZB24, Bacillus amyloliqefaciens strain MBI600, Bacillus amyloliqefaciens strain D747, Pseudomonas fluorescens, Bacillus subtilis and Trichoderma atroviride SKT-1; and
Plant extracts such as tea tree oil.

Preferred embodiments of the present invention will be described below. However, it should be understood that the present invention is by no means restricted thereto.
[1] A nematicidal composition comprising as an active ingredient a compound represented by the formula (I) or its salt.
[2] A method for controlling nematodes, which comprises applying a nematicidal composition containing an effective amount of a compound represented by the formula (I) or its salt to soil, to nematodes or to a plant body.
[3] The nematicidal composition according to the above [1], wherein in the compound represented by the formula (I) or its salt, A is phenyl substituted by one to five R¹, R¹ is halogen, alkyl, haloalkyl, alkenyl, alkynyl, cycloalkyl, alkoxy or haloalkoxy, provided that when there are two R¹, the two R¹ together may form a ring which may be substituted by one or two Z¹.
[4] The method for controlling nematodes according to the above [2], wherein in the compound represented by the formula (I) or its salt, A is phenyl substituted by one to five R¹, R¹ is halogen, alkyl, haloalkyl, alkenyl, alkynyl, cycloalkyl, alkoxy or haloalkoxy, provided that when there are two R¹, the two R¹ together may form a ring which may be substituted by one or two Z¹.
[5] A compound represented by the formula (I) or its salt.
[6] A compound represented by the formula (IA) or its salt.
[7] A compound represented by the formula (IB) or its salt.
[8] The compound or its salt according to the above [6], wherein R^{1A} is halogen, (C₁-C₆)-alkyl, (C₁-C₆)-haloalkyl, (C₂-C₆)-alkenyl, (C₂-C₆)-alkynyl, (C₃-C₆)-cycloalkyl, (C₁-C₆)-alkoxy or (C₁-C₆)-haloalkoxy.
[9] The compound or its salt according to the above [6], wherein R^{1A} is halogen, (C₁-C₆)-alkyl, (C₁-C₆)-haloalkyl, (C₁-C₆)-alkoxy or (C₁-C₆)-haloalkoxy.
[10] The compound or its salt according to the above [6], wherein R^{1A} is halogen, (C₁-C₆)-alkyl or (C₁-C₆)-haloalkyl.
[11] The compound or its salt according to the above [6], wherein two R^{1A} together form a ring which may be substituted by one or two Z^{1A}.
[12] The compound or its salt according to any one of the above [6] to [11], wherein R^{2A} and R^{3A} are (C₁-C₃)-alkyl.
[13] The compound or its salt according to any one of the above [6] to [11], wherein R^{2A} and R^{3A} are both methyl.
[14] The compound or its salt according to any one of the above [6] to [13], wherein R^{4A} is halogen, (C₁-C₆)-haloalkyl, (C₁-C₆)-alkylsulfinyl, (C₁-C₆)-alkylsulfonyl, nitro or cyano.
[15] The compound or its salt according to any one of the above [6] to [13], wherein R^{4A} is fluorine, chlorine, bromine, difluoromethyl, trifluoromethyl, nitro or cyano.
[16] The compound or its salt according to any one of the above [6] to [15], wherein R^{6A} is hydrogen, (C₁-C₆)-alkyl, (C₂-C₆)-alkenyl or (C₂-C₆)-alkynyl.
[17] The compound or its salt according to any one of the above [6] to [15], wherein R^{6A} is hydrogen.
[18] The compound or its salt according to any one of the above [6] and [8] to [17], wherein n is 1 or 2.
[19] The compound or its salt according to any one of the above [6] and [8] to [17], wherein n is 1, and the phenyl is substituted by one R^{1A} at 2- or 3-position.
[20] The compound or its salt according to any one of the above [6] and [8] to [17], wherein n is 1, and the phenyl is substituted by one R^{1A} at 2-position.
[21] The compound or its salt according to any one of the above [6] and [8] to [17], wherein n is 1, and the phenyl is substituted by one R^{1A} at 3-position.
[22] The compound or its salt according to any one of the above [6] and [8] to [17], wherein n is 2, and the phenyl is substituted by two R^{1A} at 2- and 3-positions.
[23] The compound or its salt according to any one of the above [6] and [8] to [17], wherein n is 2, and the phenyl is substituted by two R^{1A} at 2- and 6-positions.
[24] The compound or its salt according to any one of the above [6] and [8] to [17], wherein n is 2, and the phenyl is substituted by two R^{1A} at 2- and 5-positions.
[25] A method for controlling nematodes, which comprises applying a nematicidal composition containing an effective amount of the compound or its salt as defined in any one of the above [6] to [24], to soil, to nematodes or to a plant body.
[26] An agricultural and horticultural nematicide comprising as an active ingredient the compound or its salt as defined in any one of the above [6] to [24].

The compound of the present invention is preferably the compound of the formula (IA) or the compound of the formula (IB), more preferably the compound of the formula (IA).

### EXAMPLES

Now, Test Examples of the present invention will be described, however, it should be understood that the present invention is by no means restricted to such specific Examples.

### Synthesis Example 1: Synthesis of N'-(2-methyl-1-phenyl-2-(3-trifluoromethyl-1H-pyrazol-1-yl)propylidene) formhydrazide (No. A-1)

### (1) Synthesis of 2-methyl-1-phenyl-2-(3-trifluoromethyl-1H-pyrazol-1-yl)propan-1-one

Potassium carbonate (0.92 g) was added to an acetonitrile (12 ml) solution of 3-trifluoromethyl-1H-pyrazole (1.80 g) and stirred at room temperature. 30 minutes later, 2-bromo-2-methyl-1-phenylpropan-1-one (3.01 g) was added and stirred at room temperature to obtain a mixture. 10 minutes later, the mixture was heated to 70°C and reacted for 20.5 hours to obtain a reaction mixture. Water was added to the reaction mixture, followed by extraction with ethyl acetate. The obtained extract was concentrated under reduced pressure. The obtained residue was purified by column chromatography (eluent: ethyl acetate/heptane) to obtain the desired product (1.23 g). ¹H NMR (CDCl₃/300 MHz):δ (ppm)= 7.50-7.40 (m, 2H), 7.35-7.23 (m, 4H), 6.57 (d, 1H), 1.93 (s, 6H).

### (2) Synthesis of (2-methyl-1-phenyl-2-(3-trifluoromethyl-1H-pyrazol-1-yl)propylidene) hydrazide

Hydrazine monohydrate (0.38 g) was added to an ethanol (2.51 ml) solution of 2-methyl-1-phenyl-2-(3-trifluoromethyl-1H-pyrazol-1-yl)propan-1-one (0.84 g), followed by reflux for 19.5 hours to obtain a reaction mixture. Water was added to the reaction mixture, followed by extraction with ethyl acetate. Then, the obtained extract was concentrated under reduced pressure. The obtained residue was purified by column chromatography (eluent: ethyl acetate/heptane) to obtain the desired product (0.37 g). ¹H NMR (CDCl₃/300 MHz):δ (ppm)= 7.45-7.42 (m, 1H), 7.37-7.28 (m, 3H), 6.70-6.62 (m, 2H), 6.45 (d, 1H), 5.10 (s, 2H), 1.82 (s, 6H).

### (3) Synthesis of (N'-(2-methyl-1-phenyl-2-(3-trifluoromethyl-1H-pyrazol-1-yl)propylidene) formhydrazide (No. A-1)

Formic acid (0.04 g) was added to an ethyl formate (2 ml) solution of (2-methyl-1-phenyl-2-(3-trifluoromethyl-1H-pyrazol-1-yl)propylidene) hydrazide (0.20 g), followed by reflux for 2 hours to obtain a reaction mixture. Water was added to the reaction mixture, and a saturated aqueous sodium bicarbonate solution was added to obtain an alkaline reaction solution. The reaction solution was subjected to extraction with ethyl acetate, and the obtained extract was concentrated under reduced pressure. The obtained residue was purified by column chromatography (eluent: ethyl acetate/heptane) to obtain the desired product (No. A-1, 0.20 g).
¹H NMR (CDCl₃/300 MHz):δ (ppm)= 8.73 (d, 1H), 8.03 (d, 1H), 7.43-7.29 (m, 4H), 6.67-6.61 (m, 2H), 6.45 (d, 1H), 1.89 (s, 6H).

### Synthesis Example 2: Synthesis of two geometric isomers of N'-(phenyl(1-(3-trifluoromethyl-1H-pyrazol-1-yl)cyclopropyl)methylene) formhydrazide (No. B-1 and No. B-2)

### (1) Synthesis of 2-bromo-4-chloro-1-phenylbutan-1-one

Trimethylphenylammonium tribromide (1.28 g) was added to a tetrahydrofuran (6.07 ml) solution of 4-chloro-1-phenylbutan-1-one (0.61 g), followed by reaction at room temperature for 15.5 hours to obtain a reaction mixture. Water was added to the reaction mixture, followed by extraction with ethyl acetate, and the obtained extract was concentrated under reduced pressure. The obtained residue was purified by column chromatography (eluent: ethyl acetate/heptane) to obtain the desired product (0.86 g).
¹H NMR (CDCl₃/300 MHz):δ (ppm)= 8.08-8.01 (m, 2H), 7.66-7.59 (m, 1H), 7.55-7.48 (m, 2H), 5.49 (dd, 1H), 3.90-3.72 (m, 2H), 2.67-2.49 (m, 2H).

### (2) Synthesis of phenyl(1-(3-trifluoromethyl-1H-pyrazol-1-yl)cyclopropyl) methanone

Potassium carbonate (0.51 g) was added to an acetonitrile (2.58 ml) solution of 3-trifluoromethyl-1H-pyrazole (0.50 g), followed by stirring at room temperature. 50 minutes later, 2-bromo-4-chloro-1-phenylbutan-1-one (0.86 g) was added, and the mixture was heated to 90°C and reacted for 6 hours to obtain a reaction mixture. Water was added to the reaction mixture, followed by extraction with ethyl acetate, and the obtained extract was concentrated under reduced pressure. The obtained residue was purified by column chromatography (eluent: ethyl acetate/heptane) to obtain the desired product (0.86 g).
¹H NMR (CDCl₃/300 MHz):δ (ppm)= 7.48-7.39 (m, 4H), 7.32-7.24 (m, 2H), 6.43 (d, 1H), 2.08-2.01 (m, 2H), 1.89-1.83 (m, 2H).

### (3) Synthesis of two types of geometric isomers of (phenyl(1-(3-trifluoromethyl-1H-pyrazol-1-yl)cyclopropyl)methylene) hydrazide

Hydrazine monohydrate (0.51 g) was added to an ethanol (4.29 ml) solution of phenyl(1-(3-trifluoromethyl-1H-pyrazol-1-yl)cyclopropyl) methanone (1.07 g), followed by reflux for 25.5 hours to obtain a reaction mixture. Water was added to the reaction mixture, followed by extraction with ethyl acetate, and the obtained extract was concentrated under reduced pressure. The obtained residue was purified by column chromatography (eluent: ethyl acetate/heptane) to obtain a main product (0.41 g) and its geometric isomer (0.23 g).

### Main product:

¹H NMR (CDCl₃/300 MHz):δ (ppm)= 7.41-7.29 (m, 4H), 7.11-7.03 (m, 2H), 6.33 (d, 1H), 5.15 (s, 2H), 1.65-1.58 (m, 2H), 1.56-1.49 (m, 2H).

### Geometric isomer of main product:

¹H NMR (CDCl₃/300 MHz):δ (ppm)= 7.80-7.74 (m, 2H), 7.72-7.68 (m, 1H), 7.40-7.27 (m, 3H), 6.64 (s, 2H). 6.50 (d, 1H), 1.92-1.86 (m, 2H), 1.41-1.35 (m, 2H).

### (4) Synthesis of two geometric isomers of N'-(phenyl(1-(3-trifluoromethyl-1H-pyrazol-1-yl)cyclopropyl)methylene) formhydrazide (No. B-1, No. B-2)

Formic acid (0.05 g) was added to an ethyl formate (2.01 ml) solution of (phenyl(1-(3-trifluoromethyl-1H-pyrazol-1-yl)cyclopropyl)methylene) hydrazide (0.20 g), followed by reflux for 2.5 hours to obtain a reaction mixture. Water was added to the reaction mixture, and a saturated aqueous sodium bicarbonate solution was added to obtain an alkaline reaction solution. The reaction solution was subjected to extraction with ethyl acetate, and the obtained extract was concentrated under reduced pressure. The obtained residue was purified by column chromatography (eluent: ethyl acetate/heptane) to obtain main product (No. B-1, 0.18 g) and its geometric isomer (No. B-2, 0.02 g).

### Main product:

¹H NMR (CDCl₃/300 MHz):δ (ppm)= 8.61 (d, 1H), 8.17 (d, 1H), 7.43-7.37 (m, 3H), 7.37-7.34 (m, 1H), 7.06-6.99 (m, 2H), 6.33 (d, 1H), 1.80-1.61 (m, 4H).

### Geometric isomer of main product:

¹H NMR (CDCl₃/300 MHz):δ (ppm)= 10.67 (d, 1H), 8.84 (d, 1H), 7.93-7.86 (m, 2H), 7.74-7.70 (m, 1H), 7.46-7.38 (m, 3H), 6.55 (d, 1H), 1.96-1.89 (m, 2H), 1.45-1.38 (m, 2H).

### Synthesis Example 3: Synthesis of N'-(2-methyl-1-(3-methylphenyl)-2-(3-trifluoromethyl-1H-triazol-1-yl)propylidene) formhydrazide (No.A-323)

### (1) Synthesis of (2-methyl-1-(3-methylphenyl)-propylidene)hydrazone

A mixture of 2-methyl-1-(3-methylphenyl)-propan-1-one (0.35 g), ethanol (1.05 ml) and hydrazine monohydrate (0.27 g) was degassed, followed by reflux for 13.5 hours to obtain a reaction mixture. The reaction mixture was diluted with ethyl acetate and heptane, and subjected to filtration through a pad containing celite and silica gel to obtain a filtrate. The filtrate was concentrated under reduced pressure, and the obtained residue was purified by column chromatography (eluent: ethyl acetate/heptane) to obtain the desired product (0.35 g).
¹H NMR (CDCl₃/500 MHz):δ (ppm)= 7.34 (t, 1H), 7.18 (d, 1H), 6.98 (s, 1H), 6.97 (d, 1H), 4.93 (brs, 2H), 2.71 (m, 1H), 2.38 (s, 3H), 1.09 (d, 6H).

### (2) Synthesis of N' -(2-methyl-1-(3-methylphenyl)-propylidene) formhydrazide

A mixture of (2-methyl-1-(3-methylphenyl)-propylidene)hydrazone (0.35 g), ethyl formate (1.4 ml) and formic acid (0.12 g) was subjected to nitrogen replacement, followed by reflux for 5 hours to obtain a reaction solution. The reaction solution was concentrated under reduced pressure and purified by column chromatography (eluent: ethyl acetate/heptane) to obtain the desired product (0.29 g).
¹H NMR (CDCl₃/500 MHz):δ (ppm)= 8.70 (d, 1H), 8.22 (d, 1H), 7.36 (t, 1H), 7.24 (d, 1H), 6.90 (s, 1H), 6.90 (d, 1H), 2.78 (m, 1H), 2.39 (s, 3H), 1.13 (d, 6H).

### (3) Synthesis of N'-(2-chloro-2-methyl-1-(3-methylphenyl)-propylidene) formhydrazide

A mixture of N'-(2-methyl-1-(3-methylphenyl)-propylidene) formhydrazide (0.26 g), toluene (2.5 ml) and sulfuryl chloride (0.2 g) was stirred at room temperature for 4 hours to obtain a reaction mixture. While the obtained reaction mixture was cooled in an ice bath, ice water and a saturated aqueous sodium hydrogen carbonate were added sequentially and stirred, and the mixture was subjected to extraction with ethyl acetate three times. The resulting organic layer was subjected to filtration through a celite pad to obtain a filtrate. The filtrate was concentrated under reduced pressure, and the obtained residue was purified by column chromatography (eluent: ethyl acetate/heptane) to obtain the desired product (0.24 g).
¹H NMR (CDCl₃/500 MHz):δ (ppm)= 8.70 (d, 1H), 8.05 (d, 1H), 7.39 (t, 1H), 7.29 (d, 1H), 7.03 (s, 1H), 7.03 (d, 1H), 2.41 (s, 3H), 1.82 (s, 6H).

### (4) Synthesis of N'-(2-methyl-1-(3-methylphenyl)-2-(3-trifluoromethyl-1H-triazol-1-yl)propylidene) formhydrazide (No. A-323)

A mixture of 3-trifluoromethyl-1H-triazole (0.05 g), DMF(0.3 ml) and potassium carbonate (0.05 g) was subjected to nitrogen replacement, followed by stirring at room temperature overnight. To the obtained mixture, a DMF (0.3 ml) solution of N-(2-chloro-2-methyl-1-(3-methylphenyl)-propylidene) formhydrazide (0.07 g) was added dropwise little by little, followed by stirring at room temperature for 1 hour to obtain a reaction mixture. The obtained reaction mixture was diluted with water, followed by extraction with ethyl acetate/heptane three times. The resulting organic layer was subjected to filtration through a celite pad to obtain a filtrate. The filtrate was concentrated under reduced pressure, and the obtained residue was purified by column chromatography (eluent: ethyl acetate/heptane) to obtain the desired product (No. A-323, 0.09 g).
¹H NMR (CDCl₃/500 MHz):δ (ppm)= 8.68 (d, 1H), 8.04 (d, 1H), 8.03 (s, 1H), 7.28 (t, 1H), 7.22 (d, 1H), 6.55 (d, 1H), 6.49 (s, 1H), 2.28 (s, 3H), 1.94 (s, 6H).

Now, representative examples of the compound (I) of the present invention are specifically shown in Tables 1 and 2. These compounds may be synthesized by the above production method or the above Synthesis Example, or a method known in this technical field.

In Tables, No. represents Compound No. Of compounds indicated with "NMR" in the column "Physical Properties" in Tables 1 and 2, ¹H-NMR spectrum data are shown in Tables 3 and 4. Further, in a case where the compound in Table 1 or 2 is a salt of the compound (I) of the present invention, the type of the salt is also shown in the column "Physical Properties" in Table 1 or 2.

In Tables 1 and 2, abbreviations used in the columns A, R², R³, R⁴, R⁶ and X are as follows. Me represents a methyl group, Et an ethyl group, nPr a normal-propyl group, iPr an isopropyl group, cPr a cyclopropyl group, tBu a tert-butyl group, cBu a cyclobutyl group, nHexyl a normal-hexyl group, cHexyl a cyclohexyl group, Ph a phenyl group, Na salt a sodium salt, K salt a potassium salt, di two, bis two, and tri three, respectively.

In Tables 1 and 2, the compounds (I) of the present invention wherein R⁵ is CHO are shown. In Tables 1 and 2, the positions of the substituents identified in columns A and R⁴ are as follows. "*" means the binding site.

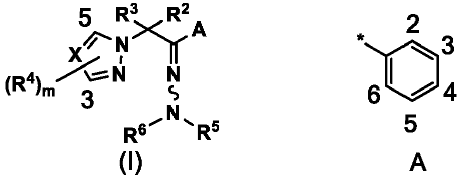

For example, a compound indicated with "4-CI-Ph" in the column A, means a compound in which A is phenyl substituted by one R¹ group at the substitution position in the above chemical structure, that is the phenyl is substituted by a chloro group only at the 4-position.

Further, a compound indicated with "3-CF₃" in the column R⁴ and with "1" in the column m, means a compound in which the hetero 5-membered ring is substituted by one R⁴ group at the substitution position in the above chemical structure, that is the hetero 5-membered ring is substituted by a trifluoromethyl group only at the 3-position. A compound indicated with "3-CF₃-5-Me" in the column R⁴ and with "2" in the column m, means a compound in which the hetero 5-membered ring is substituted by two R⁴ groups at the substitution positions in the above chemical structure, that is the hetero 5-membered ring is substituted by a trifluoromethyl group at the 3-position and by a methyl group at the 5-position. A compound indicated with "-" in the column R⁴ and with "0" in the column m, means a compound in which the hetero 5-membered ring in the above chemical structure has no R⁴ group at the 3- and 5-positions.

In Tables 1 and 2, Compound Nos. A-188, A-189, B-186 and B-187 are sodium salts of A-1, A-2, B-1 and B-3, and Compound Nos. A-190, A-191, B-188 and B-189 are potassium salts of A-1, A-2, B-1 and B-3.

In ¹H-NMR data in Tables 3 and 4, s means singlet, brs broad singlet, d doublet, t triplet, q quartet, dd double doublet, dt double triplet, ddd double double doublet, and m multiplet.

**TABLE 1**

| 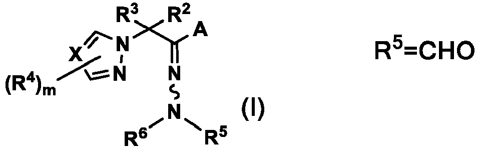 | | | | | | | | |
|---|---|---|---|---|---|---|---|---|
| No. | A | R² | R³ | R⁴ | R⁶ | X | m | Physical properties |
| A-1 | Ph | Me | Me | 3-CF₃ | H | CH | 1 | NMR |
| A-2 | Ph | Me | Me | 3-CF₃ | H | N | 1 | NMR |
| A-3 | Ph | Me | Me | 3-CF₃ | Me | CH | 1 | |
| A-4 | Ph | Me | Me | 3-CF₃ | Me | N | 1 | |
| A-5 | Ph | Me | Me | 3-CF₃ | Et | CH | 1 | |
| A-6 | Ph | Me | Me | 3-CF₃ | Et | N | 1 | |
| A-7 | Ph | Me | Me | 3-CF₃ | nPr | CH | 1 | |
| A-8 | Ph | Me | Me | 3-CF₃ | nPr | N | 1 | |
| A-9 | Ph | Me | Me | 3-CF₃ | nHexyl | CH | 1 | |
| A-10 | Ph | Me | Me | 3-CF₃ | nHexyl | N | 1 | |
| A-11 | Ph | Me | Me | 3-CF₃ | iPr | CH | 1 | |
| A-12 | Ph | Me | Me | 3-CF₃ | iPr | N | 1 | |
| A-13 | Ph | Me | Me | 3-CF₃ | CH₂CH=CH₂ | CH | 1 | |
| A-14 | Ph | Me | Me | 3-CF₃ | CH₂CH=CH₂ | N | 1 | |
| A-15 | Ph | Me | Me | 3-CF₃ | CH₂CH=C(Me)₂ | CH | 1 | |
| A-16 | Ph | Me | Me | 3-CF₃ | CH₂CH=C(Me)₂ | N | 1 | |
| A-17 | Ph | Me | Me | 3-CF₃ | CH₂C≡CH | CH | 1 | |
| A-18 | Ph | Me | Me | 3-CF₃ | CH₂C≡CH | N | 1 | |
| A-19 | Ph | Me | Me | 3-CF₃ | CH₂C≡CMe | CH | 1 | |
| A-20 | Ph | Me | Me | 3-CF₃ | CH₂C≡CMe | N | 1 | |
| A-21 | Ph | Me | Me | 3-CF₃ | CH₂OMe | CH | 1 | |
| A-22 | Ph | Me | Me | 3-CF₃ | CH₂OMe | N | 1 | |
| A-23 | Ph | Me | Me | 3-CF₃ | CH₂OEt | CH | 1 | |
| A-24 | Ph | Me | Me | 3-CF₃ | CH₂OEt | N | 1 | |
| A-25 | Ph | Me | Me | 3-CF₃ | C₂H₄OMe | CH | 1 | |
| A-26 | Ph | Me | Me | 3-CF₃ | C₂H₄OMe | N | 1 | |

| No. | A | R² | R³ | R⁴ | R⁶ | X | m | Physical properties |
|---|---|---|---|---|---|---|---|---|
| A-27 | Ph | Me | Me | 3-CF₃ | SO₂CF₃ | CH | 1 | |
| A-28 | Ph | Me | Me | 3-CF₃ | SO₂CF₃ | N | 1 | |
| A-29 | Ph | Me | Me | 3-CHF₂ | H | CH | 1 | |
| A-30 | Ph | Me | Me | 3-CF₂CF₃ | H | CH | 1 | |
| A-31 | Ph | Me | Me | 3-OMe | H | CH | 1 | |
| A-32 | Ph | Me | Me | 3-OMe | H | N | 1 | |
| A-33 | Ph | Me | Me | 3-O(iPr) | H | CH | 1 | |
| A-34 | Ph | Me | Me | 3-O(nHexyl) | H | CH | 1 | |
| A-35 | Ph | Me | Me | 3-OCHF₂ | H | CH | 1 | |
| A-36 | Ph | Me | Me | 3-OCF₃ | H | CH | 1 | |
| A-37 | Ph | Me | Me | 3-OCF₂CF₃ | H | CH | 1 | |
| A-38 | Ph | Me | Me | 3-OCH(CF₃)₂ | H | CH | 1 | |
| A-39 | Ph | Me | Me | 3-OCH₂CF₃ | H | CH | 1 | |
| A-40 | Ph | Me | Me | 3-F | H | CH | 1 | |
| A-41 | Ph | Me | Me | 3-Cl | H | CH | 1 | |
| A-42 | Ph | Me | Me | 3-Cl | H | N | 1 | |
| A-43 | Ph | Me | Me | 3-Br | H | CH | 1 | |
| A-44 | Ph | Me | Me | 3-Br | H | N | 1 | |
| A-45 | Ph | Me | Me | 3-I | H | CH | 1 | |
| A-46 | Ph | Me | Me | 3-NO₂ | H | CH | 1 | |
| A-47 | Ph | Me | Me | 3-NO₂ | H | N | 1 | |
| A-48 | Ph | Me | Me | 3-CN | H | CH | 1 | |
| A-49 | Ph | Me | Me | 3-CN | H | N | 1 | |
| A-50 | Ph | Me | Me | 3-SMe | H | CH | 1 | |
| A-51 | Ph | Me | Me | 3-SMe | H | N | 1 | |
| A-52 | Ph | Me | Me | 3-S(iPr) | H | N | 1 | |
| A-53 | Ph | Me | Me | 3-S(nHexyl) | H | N | 1 | |
| A-54 | Ph | Me | Me | 3-SOMe | H | CH | 1 | |
| A-55 | Ph | Me | Me | 3-SOMe | H | N | 1 | |
| A-56 | Ph | Me | Me | 3-SO₂Me | H | CH | 1 | |
| A-57 | Ph | Me | Me | 3-SO₂Me | H | N | 1 | |
| A-58 | Ph | Me | Me | 3-CF₃ | H | CCl | 1 | |
| A-59 | Ph | Me | Me | 3-CF₃ | H | CBr | 1 | |
| A-60 | Ph | Me | Me | 3-CF₃ | H | CMe | 1 | |
| A-61 | Ph | Me | Me | 3-CF₃ | H | C-C≡CH | 1 | |
| A-62 | Ph | Me | Me | 3-CF₃ | H | C-cPr | 1 | |
| A-63 | Ph | Me | Me | 3-CF₃-5-Me | H | CH | 2 | |
| A-64 | Ph | Me | Me | 3-Cl | H | CNO₂ | 1 | |
| A-65 | Ph | Me | Me | 3-Cl | H | CCl | 1 | |
| A-66 | Ph | Me | Me | 3-Cl | H | CBr | 1 | |
| A-67 | Ph | Me | Me | 3-Cl | H | CMe | 1 | |
| A-68 | Ph | Me | Me | 3-Cl | H | CEt | 1 | |
| A-69 | Ph | Me | Me | 3-Cl | H | C-iPr | 1 | |
| A-70 | Ph | Me | Me | 3-Cl | H | C-nHexyl | 1 | |
| A-71 | Ph | Me | Me | 3-Cl | H | C-CH=CH₂ | 1 | |
| A-72 | Ph | Me | Me | 3-Cl | H | C-C(Me)=CH₂ | 1 | |
| A-73 | Ph | Me | Me | 3-Cl | H | C-C≡CH | 1 | |
| A-74 | Ph | Me | Me | 3-Cl | H | C-cPr | 1 | |
| A-75 | Ph | Me | Me | 3-Cl | H | CCF₃ | 1 | |
| A-76 | Ph | Me | Me | - | H | CCF₃ | 0 | |
| A-77 | Ph | Me | Me | - | H | CCl | 0 | |
| A-78 | Ph | Me | Me | - | H | CF | 0 | |
| A-79 | Ph | Me | Et | 3-CF₃ | H | CH | 1 | |
| A-80 | Ph | Me | Et | 3-CF₃ | H | N | 1 | |
| A-81 | Ph | Et | Et | 3-CF₃ | H | CH | 1 | |
| A-82 | Ph | Et | Et | 3-CF₃ | H | N | 1 | |
| A-83 | Ph | Me | nPr | 3-CF₃ | H | CH | 1 | |
| A-84 | Ph | Me | nPr | 3-CF₃ | H | N | 1 | |
| A-85 | Ph | Me | iPr | 3-CF₃ | H | CH | 1 | |
| A-86 | Ph | Me | iPr | 3-CF₃ | H | N | 1 | |
| A-87 | Ph | Me | nHexyl | 3-CF₃ | H | CH | 1 | |
| A-88 | Ph | Me | nHexyl | 3-CF₃ | H | N | 1 | |
| A-89 | 4-Cl-Ph | Me | Me | 3-CF₃ | H | CH | 1 | NMR |
| A-90 | 4-Cl-Ph | Me | Me | 3-CF₃ | H | N | 1 | NMR |
| A-91 | 4-Cl-Ph | Me | Me | 3-CF₃ | Me | CH | 1 | NMR |
| A-92 | 4-Cl-Ph | Me | Me | 3-CF₃ | Et | CH | 1 | NMR |
| A-93 | 4-Cl-Ph | Me | Me | 3-CF₃ | CH₂OMe | CH | 1 | |
| A-94 | 4-Cl-Ph | Me | Me | 3-CF₃ | Me | N | 1 | NMR |
| A-95 | 4-Cl-Ph | Me | Me | 3-CF₃ | Et | N | 1 | NMR |
| A-96 | 4-Cl-Ph | Me | Me | 3-CF₃ | nPr | N | 1 | NMR |
| A-97 | 4-Cl-Ph | Me | Me | 3-CF₃ | nHexyl | N | 1 | |
| A-98 | 4-Cl-Ph | Me | Me | 3-CF₃ | iPr | N | 1 | |
| A-99 | 4-Cl-Ph | Me | Me | 3-CF₃ | CH₂OMe | N | 1 | |
| A-100 | 4-Cl-Ph | Me | Me | 3-CF₃ | CH₂OEt | N | 1 | |
| A-101 | 4-Cl-Ph | Me | Me | 3-CF₃ | C₂H₄OMe | N | 1 | NMR |
| A-102 | 4-Cl-Ph | Me | Me | 3-CF₃ | SO₂CF₃ | N | 1 | |
| A-103 | 4-Cl-Ph | Me | Me | 3-OMe | H | CH | 1 | NMR |
| A-104 | 4-Cl-Ph | Me | Me | 3-OMe | H | N | 1 | |
| A-105 | 4-Cl-Ph | Me | Me | 3-CI | H | CH | 1 | |
| A-106 | 4-Cl-Ph | Me | Me | 3-CI | H | N | 1 | |
| A-107 | 4-F-Ph | Me | Me | 3-CF₃ | H | CH | 1 | NMR |
| A-108 | 4-F-Ph | Me | Me | 3-CF₃ | H | N | 1 | |
| A-109 | 4-F-Ph | Me | Me | 3-CF₃ | CH₂OMe | CH | 1 | NMR |
| A-110 | 4-F-Ph | Me | Me | 3-CF₃ | CH₂OMe | N | 1 | |
| A-111 | 4-F-Ph | Me | Me | 3-CF₃ | CH₂Ph | CH | 1 | |
| A-112 | 4-F-Ph | Me | Me | 3-CF₃ | CH₂Ph | N | 1 | |
| A-113 | 4-F-Ph | Me | Me | 3-CF₃ | CH₂-(4-OMe-Ph) | CH | 1 | |
| A-114 | 4-F-Ph | Me | Me | 3-CF₃ | CH₂-(4-OMe-Ph) | N | 1 | |
| A-115 | 4-F-Ph | Me | Me | 3-CF₃ | CO₂tBu | CH | 1 | |
| A-116 | 4-F-Ph | Me | Me | 3-CF₃ | CO₂tBu | N | 1 | |
| A-117 | 4-F-Ph | Me | Me | 3-CF₃ | CO₂CH₂Ph | CH | 1 | |
| A-118 | 4-F-Ph | Me | Me | 3-CF₃ | CO₂CH₂Ph | N | 1 | |
| A-119 | 4-F-Ph | Me | Me | 3-OMe | H | CH | 1 | |
| A-120 | 4-F-Ph | Me | Me | 3-OMe | H | N | 1 | |
| A-121 | 4-F-Ph | Me | Me | 3-CI | H | CH | 1 | |
| A-122 | 4-F-Ph | Me | Me | 3-CI | H | N | 1 | |
| A-123 | 4-F-Ph | Me | Me | 3-CF₃ | H | CCl | 1 | |
| A-124 | 4-F-Ph | Me | Me | 3-CI | H | CB r | 1 | |
| A-125 | 4-F-Ph | Me | Me | - | H | CF | 0 | |
| A-126 | 4-Br-Ph | Me | Me | 3-CF₃ | H | CH | 1 | |
| A-127 | 4-Br-Ph | Me | Me | 3-CF₃ | H | N | 1 | |
| A-128 | 4-Br-Ph | Me | Me | 3-CF₃ | CH₂OMe | CH | 1 | |
| A-129 | 4-Br-Ph | Me | Me | 3-CF₃ | CH₂OMe | N | 1 | |
| A-130 | 4-I-Ph | Me | Me | 3-CF₃ | H | CH | 1 | |
| A-131 | 4-I-Ph | Me | Me | 3-CF₃ | H | N | 1 | |
| A-132 | 4-CF₃-Ph | Me | Me | 3-CF₃ | H | CH | 1 | |
| A-133 | 4-CF₃-Ph | Me | Me | 3-CF₃ | H | N | 1 | |
| A-134 | 4-OMe-Ph | Me | Me | 3-CF₃ | H | CH | 1 | |
| A-135 | 4-OMe-Ph | Me | Me | 3-CF₃ | H | N | 1 | |
| A-136 | 4-O(iPr)-Ph | Me | Me | 3-CF₃ | H | CH | 1 | |
| A-137 | 4-O(iPr)-Ph | Me | Me | 3-CF₃ | H | N | 1 | |
| A-138 | 4-O(nHexyl)-Ph | Me | Me | 3-CF₃ | H | CH | 1 | |
| A-139 | 4-O(nHexyl)-Ph | Me | Me | 3-CF₃ | H | N | 1 | |
| A-140 | 4-OCHF₂-Ph | Me | Me | 3-CF₃ | H | CH | 1 | |
| A-141 | 4-OCHF₂-Ph | Me | Me | 3-CF₃ | H | N | 1 | |
| A-142 | 4-OCF₃-Ph | Me | Me | 3-CF₃ | H | CH | 1 | |
| A-143 | 4-OCF₃-Ph | Me | Me | 3-CF₃ | H | N | 1 | |
| A-144 | 4-OCF₂CF₃-Ph | Me | Me | 3-CF₃ | H | CH | 1 | |
| A-145 | 4-OCF₂CF₃-Ph | Me | Me | 3-CF₃ | H | N | 1 | |
| A-146 | 4-OCH(CF₃)₂-Ph | Me | Me | 3-CF₃ | H | CH | 1 | |
| A-147 | 4-OCH(CF₃)₂-Ph | Me | Me | 3-CF₃ | H | N | 1 | |
| A-148 | 4-OCH₂CF₃-Ph | Me | Me | 3-CF₃ | H | CH | 1 | |
| A-149 | 4-OCH₂CF₃-Ph | Me | Me | 3-CF₃ | H | N | 1 | |
| A-150 | 2-Cl-Ph | Me | Me | 3-CF₃ | H | CH | 1 | NMR |
| A-151 | 2-Cl-Ph | Me | Me | 3-CF₃ | H | N | 1 | NMR |
| A-152 | 2-F-Ph | Me | Me | 3-CF₃ | H | CH | 1 | NMR |
| A-153 | 2-F-Ph | Me | Me | 3-CF₃ | H | N | 1 | NMR |
| A-154 | 2,4-diCl-Ph | Me | Me | 3-CF₃ | H | CH | 1 | NMR |
| A-155 | 2,4-diCl-Ph | Me | Me | 3-CF₃ | H | N | 1 | |
| A-156 | 2,4-diF-Ph | Me | Me | 3-CF₃ | H | CH | 1 | NMR |
| A-157 | 2,4-diF-Ph | Me | Me | 3-CF₃ | H | N | 1 | NMR |
| A-158 | 3,5-diF-Ph | Me | Me | 3-CF₃ | H | CH | 1 | |
| A-159 | 3,5-diF-Ph | Me | Me | 3-CF₃ | H | N | 1 | |
| A-160 | 2,6-diF-Ph | Me | Me | 3-CF₃ | H | CH | 1 | NMR |
| A-161 | 2,6-diF-Ph | Me | Me | 3-CF₃ | H | N | 1 | NMR |
| A-162 | 3,4,5-triF-Ph | Me | Me | 3-CF₃ | H | CH | 1 | |
| A-163 | 3,4,5-triF-Ph | Me | Me | 3-CF₃ | H | N | 1 | |
| A-164 | 4-Et-Ph | Me | Me | 3-CF₃ | H | CH | 1 | |
| A-165 | 4-Et-Ph | Me | Me | 3-CF₃ | H | N | 1 | |
| A-166 | 4-iPr-Ph | Me | Me | 3-CF₃ | H | CH | 1 | |
| A-167 | 4-iPr-Ph | Me | Me | 3-CF₃ | H | N | 1 | |
| A-168 | 4-CH₂CH(Me)₂-Ph | Me | Me | 3-CF₃ | H | CH | 1 | |
| A-169 | 4-CH₂CH(Me)₂-Ph | Me | Me | 3-CF₃ | H | N | 1 | |
| A-170 | 4-nHexyl-Ph | Me | Me | 3-CF₃ | H | CH | 1 | |
| A-171 | 4-nHexyl-Ph | Me | Me | 3-CF₃ | H | N | 1 | |
| A-172 | 4-CH=CH₂-Ph | Me | Me | 3-CF₃ | H | CH | 1 | |
| A-173 | 4-CH=CH₂-Ph | Me | Me | 3-CF₃ | H | N | 1 | |
| A-174 | 4-C(Me)=CH₂-Ph | Me | Me | 3-CF₃ | H | CH | 1 | |
| A-175 | 4-C(Me)=CH₂-Ph | Me | Me | 3-CF₃ | H | N | 1 | |
| A-176 | 4-CH=C(Me)₂-Ph | Me | Me | 3-CF₃ | H | CH | 1 | |
| A-177 | 4-CH=C(Me)₂-Ph | Me | Me | 3-CF₃ | H | N | 1 | |
| A-178 | 4-C≡CH-Ph | Me | Me | 3-CF₃ | H | CH | 1 | |
| A-179 | 4-C≡CH-Ph | Me | Me | 3-CF₃ | H | N | 1 | |
| A-180 | 4-cPr-Ph | Me | Me | 3-CF₃ | H | CH | 1 | |
| A-181 | 4-cPr-Ph | Me | Me | 3-CF₃ | H | N | 1 | |
| A-182 | 2-Me-4-Cl-Ph | Me | Me | 3-CF₃ | H | CH | 1 | NMR |
| A-183 | 2-Me-4-Cl-Ph | Me | Me | 3-CF₃ | H | N | 1 | |
| A-184 | 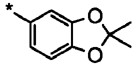 | Me | Me | 3-CF₃ | H | CH | 1 | |
| A-185 | 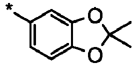 | Me | Me | 3-CF₃ | H | N | 1 | |
| A-186 | 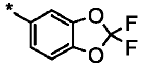 | Me | Me | 3-CF₃ | H | CH | 1 | |
| A-187 | 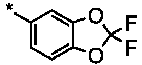 | Me | Me | 3-CF₃ | H | N | 1 | |
| A-188 | Ph | Me | Me | 3-CF₃ | H | CH | 1 | Na salt |
| A-189 | Ph | Me | Me | 3-CF₃ | H | N | 1 | Na salt |
| A-190 | Ph | Me | Me | 3-CF₃ | H | CH | 1 | K salt |
| A-191 | Ph | Me | Me | 3-CF₃ | H | N | 1 | K salt |
| A-192 | 2-Cl-Ph | Me | Me | 3-CF₃ | Me | CH | 1 | |
| A-193 | 2-Cl-Ph | Me | Me | 3-CF₃ | Et | CH | 1 | NMR |
| A-194 | 2-Cl-Ph | Me | Me | 3-CF₃ | nPr | CH | 1 | |
| A-195 | 2-Cl-Ph | Me | Me | 3-CF₃ | CH₂C≡CH | CH | 1 | NMR |
| A-196 | 2-Cl-Ph | Me | Me | 3-CF₃ | CH₂CH=CH₂ | CH | 1 | |
| A-197 | 2-Cl-Ph | Me | Me | 3-CF₃ | CH₂OMe | CH | 1 | |
| A-198 | 2-Cl-Ph | Me | Me | 3-CHF₂ | H | N | 1 | NMR |
| A-199 | 2-Cl-Ph | Me | Me | 3-Cl | H | CH | 1 | NMR |
| A-200 | 2-Cl-Ph | Me | Me | 3-Cl | H | N | 1 | NMR |
| A-201 | 2-Cl-Ph | Me | Me | 3-Br | H | CH | 1 | NMR |
| A-202 | 2-Cl-Ph | Me | Me | 3-Br | H | N | 1 | NMR |
| A-203 | 2-Cl-Ph | Me | Me | 3-I | H | CH | 1 | NMR |
| A-204 | 2-Cl-Ph | Me | Me | 3-I | H | N | 1 | |
| A-205 | 2-Cl-Ph | Me | Me | 3-NO₂ | H | CH | 1 | NMR |
| A-206 | 2-Cl-Ph | Me | Me | 3-NO₂ | H | N | 1 | NMR |
| A-207 | 2-Cl-Ph | Me | Me | 3-CN | H | CH | 1 | |
| A-208 | 2-Cl-Ph | Me | Me | 3-CN | H | N | 1 | |
| A-209 | 2-Cl-Ph | Me | Me | 3-Me | H | CH | 1 | |
| A-210 | 2-Cl-Ph | Me | Me | 3-Me | H | N | 1 | NMR |
| A-211 | 2-Cl-Ph | Me | Me | 3-cPr | H | CH | 1 | |
| A-212 | 2-Cl-Ph | Me | Me | 3-cBu | H | CH | 1 | |
| A-213 | 2-Cl-Ph | Me | Me | 3-cHexyl | H | CH | 1 | |
| A-214 | 2-Cl-Ph | Me | Me | 3-OMe | H | CH | 1 | NMR |
| A-215 | 2-Cl-Ph | Me | Me | 3-S(nPr) | H | CH | 1 | NMR |
| A-216 | 2-Cl-Ph | Me | Me | - | H | CCF₃ | 0 | NMR |
| A-217 | 2-Cl-Ph | Me | Me | - | H | CF | 0 | NMR |
| A-218 | 2-Cl-Ph | Me | Me | - | H | CCl | 0 | |
| A-219 | 2-Cl-Ph | Me | Me | - | H | CNO₂ | 0 | |
| A-220 | 2-Cl-Ph | Me | Me | 3-CF₃ | H | CCN | 1 | |
| A-221 | 2-Cl-Ph | Me | Me | 3-CF₃-5-Me | H | CH | 2 | NMR |
| A-222 | 2-Cl-Ph | Me | Me | 3-CF₃-5-CF₃ | H | CH | 2 | NMR |
| A-223 | 2-Cl-Ph | Me | Me | 3-Br-5-Br | H | CBr | 2 | NMR |
| A-224 | 2-Cl-Ph | Me | Me | 3-Br-5-Br | H | N | 2 | NMR |
| A-225 | 2-Cl-Ph | Et | Et | 3-CF₃ | H | CH | 1 | NMR |
| A-226 | 2-Cl-Ph | Et | Et | 3-CF₃ | H | N | 1 | NMR |
| A-227 | 2-Cl-Ph | Et | Et | 3-Cl | H | CH | 1 | |
| A-228 | 2-Cl-Ph | Et | Et | 3-Cl | H | N | 1 | |
| A-229 | 2-Cl-Ph | Et | Et | 3-Br | H | CH | 1 | |
| A-230 | 2-Cl-Ph | Et | Et | 3-Br | H | N | 1 | |
| A-231 | 2-Cl-Ph | nPr | nPr | 3-CF₃ | H | CH | 1 | |
| A-232 | 2-Cl-Ph | nPr | nPr | 3-CF₃ | H | N | 1 | |
| A-233 | 2-Br-Ph | Me | Me | 3-CF₃ | H | CH | 1 | NMR |
| A-234 | 2-Br-Ph | Me | Me | 3-CF₃ | H | N | 1 | NMR |
| A-235 | 2-Br-Ph | Me | Me | 3-CF₃ | CH₂OMe | CH | 1 | NMR |
| A-236 | 2-Br-Ph | Me | Me | 3-Cl | H | CH | 1 | |
| A-237 | 2-Br-Ph | Me | Me | 3-Cl | H | N | 1 | |
| A-238 | 2-Br-Ph | Me | Me | 3-Br | H | CH | 1 | |
| A-239 | 2-Br-Ph | Me | Me | 3-Br | H | N | 1 | |
| A-240 | 2-I-Ph | Me | Me | 3-CF₃ | H | CH | 1 | NMR |
| A-241 | 2-I-Ph | Me | Me | 3-CF₃ | H | N | 1 | NMR |
| A-242 | 2-I-Ph | Me | Me | 3-CF₃ | CH₂OMe | CH | 1 | |
| A-243 | 2-I-Ph | Me | Me | 3-CF₃ | CO₂tBu | CH | 1 | NMR |
| A-244 | 2-Me-Ph | Me | Me | 3-CF₃ | H | CH | 1 | NMR |
| A-245 | 2-Me-Ph | Me | Me | 3-CF₃ | H | N | 1 | NMR |
| A-246 | 2-Me-Ph | Me | Me | 3-CHF₂ | H | N | 1 | |
| A-247 | 2-Me-Ph | Me | Me | 3-Cl | H | CH | 1 | NMR |
| A-248 | 2-Me-Ph | Me | Me | 3-Cl | H | N | 1 | NMR |
| A-249 | 2-Me-Ph | Me | Me | 3-Br | H | CH | 1 | NMR |
| A-250 | 2-Me-Ph | Me | Me | 3-Br | H | N | 1 | |
| A-251 | 2-Me-Ph | Me | Me | 3-NO₂ | H | CH | 1 | NMR |
| A-252 | 2-Me-Ph | Me | Me | 3-NO₂ | H | N | 1 | NMR |
| A-253 | 2-Me-Ph | Me | Me | 3-CN | H | CH | 1 | NMR |
| A-254 | 2-Me-Ph | Me | Me | 3-CN | H | N | 1 | |
| A-255 | 2-Me-Ph | Me | Me | - | H | CF | 0 | NMR |
| A-256 | 2-Me-Ph | Me | Me | - | H | CNO₂ | 0 | NMR |
| A-257 | 2-Me-Ph | Me | Me | 3-CF₃ | H | CCN | 1 | NMR |
| A-258 | 2-Me-Ph | Me | Me | 3-Br-5-Br | H | CBr | 2 | |
| A-259 | 2-Me-Ph | Me | Me | 3-Br-5-Br | H | N | 2 | |
| A-260 | 2-CF₃-Ph | Me | Me | 3-CF₃ | H | CH | 1 | NMR |
| A-261 | 2-CF₃-Ph | Me | Me | 3-CF₃ | H | N | 1 | NMR |
| A-262 | 2-CHF₂-Ph | Me | Me | 3-CF₃ | H | CH | 1 | |
| A-263 | 2-CHF₂-Ph | Me | Me | 3-CF₃ | H | N | 1 | |
| A-264 | 2-OMe-Ph | Me | Me | 3-CF₃ | H | CH | 1 | NMR |
| A-265 | 2-OMe-Ph | Me | Me | 3-CF₃ | H | N | 1 | NMR |
| A-266 | 2-OMe-Ph | Me | Me | 3-Cl | H | CH | 1 | |
| A-267 | 2-OMe-Ph | Me | Me | 3-Cl | H | N | 1 | |
| A-268 | 2-OMe-Ph | Me | Me | 3-Br | H | CH | 1 | |
| A-269 | 2-OMe-Ph | Me | Me | 3-Br | H | N | 1 | |
| A-270 | 2-OCF₃-Ph | Me | Me | 3-CF₃ | H | CH | 1 | NMR |
| A-271 | 2-OCF₃-Ph | Me | Me | 3-CF₃ | H | N | 1 | NMR |
| A-272 | 2-OCF₃-Ph | Me | Me | 3-Br | H | CH | 1 | NMR |
| A-273 | 2-OCF₃-Ph | Me | Me | 3-CN | H | N | 1 | NMR |
| A-274 | 3-F-Ph | Me | Me | 3-CF₃ | H | CH | 1 | NMR |
| A-275 | 3-F-Ph | Me | Me | 3-CF₃ | H | N | 1 | NMR |
| A-276 | 3-F-Ph | Me | Me | 3-CHF₂ | H | N | 1 | NMR |
| A-277 | 3-F-Ph | Me | Me | 3-Cl | H | CH | 1 | NMR |
| A-278 | 3-F-Ph | Me | Me | 3-Cl | H | N | 1 | NMR |
| A-279 | 3-F-Ph | Me | Me | 3-Br | H | CH | 1 | NMR |
| A-280 | 3-F-Ph | Me | Me | 3-Br | H | N | 1 | |
| A-281 | 3-F-Ph | Me | Me | 3-I | H | CH | 1 | |
| A-282 | 3-F-Ph | Me | Me | 3-I | H | N | 1 | |
| A-283 | 3-F-Ph | Me | Me | 3-NO₂ | H | CH | 1 | |
| A-284 | 3-F-Ph | Me | Me | 3-NO₂ | H | N | 1 | |
| A-285 | 3-F-Ph | Me | Me | 3-CN | H | CH | 1 | |
| A-286 | 3-F-Ph | Me | Me | 3-CN | H | N | 1 | |
| A-287 | 3-F-Ph | Me | Me | 3-Me | H | CH | 1 | NMR |
| A-288 | 3-F-Ph | Me | Me | 3-Me | H | N | 1 | |
| A-289 | 3-F-Ph | Me | Me | 3-cPr | H | CH | 1 | NMR |
| A-290 | 3-F-Ph | Me | Me | 3-cBu | H | CH | 1 | |
| A-291 | 3-F-Ph | Me | Me | 3-cHexyl | H | CH | 1 | NMR |
| A-292 | 3-F-Ph | Me | Me | 3-OMe | H | CH | 1 | |
| A-293 | 3-F-Ph | Me | Me | 3-S(nPr) | H | CH | 1 | |
| A-294 | 3-F-Ph | Me | Me | 3-SOMe | H | CH | 1 | NMR |
| A-295 | 3-F-Ph | Me | Me | 3-SO₂Me | H | CH | 1 | NMR |
| A-296 | 3-F-Ph | Me | Me | - | H | CCF₃ | 0 | |
| A-297 | 3-F-Ph | Me | Me | - | H | CF | 0 | |
| A-298 | 3-F-Ph | Me | Me | - | H | CCl | 0 | |
| A-299 | 3-F-Ph | Me | Me | - | H | CBr | 0 | |
| A-300 | 3-F-Ph | Me | Me | - | H | Cl | 0 | |
| A-301 | 3-F-Ph | Me | Me | - | H | CNO₂ | 0 | |
| A-302 | 3-F-Ph | Me | Me | 3-CF₃-5-Me | H | CH | 2 | |
| A-303 | 3-F-Ph | Me | Me | 3-CF₃-5-CF₃ | H | CH | 2 | |
| A-304 | 3-F-Ph | Me | Me | 3-Br-5-Br | H | CBr | 2 | |
| A-305 | 3-F-Ph | Me | Me | 3-Br-5-Br | H | N | 2 | |
| A-306 | 3-F-Ph | Me | Me | 3-CF₃ | Me | CH | 1 | |
| A-307 | 3-F-Ph | Me | Me | 3-CF₃ | Et | CH | 1 | |
| A-308 | 3-F-Ph | Me | Me | 3-CF₃ | nPr | CH | 1 | |
| A-309 | 3-F-Ph | Me | Me | 3-CF₃ | nHexyl | CH | 1 | |
| A-310 | 3-F-Ph | Me | Me | 3-CF₃ | iPr | CH | 1 | |
| A-311 | 3-F-Ph | Me | Me | 3-CF₃ | CH₂CH=CH₂ | CH | 1 | |
| A-312 | 3-F-Ph | Me | Me | 3-CF₃ | CH₂CH=C(Me)₂ | CH | 1 | |
| A-313 | 3-F-Ph | Me | Me | 3-CF₃ | CH₂C≡CH | CH | 1 | |
| A-314 | 3-F-Ph | Me | Me | 3-CF₃ | CH₂C≡CMe | CH | 1 | |
| A-315 | 3-F-Ph | Me | Me | 3-CF₃ | CH₂OMe | CH | 1 | |
| A-316 | 3-F-Ph | Me | Me | 3-CF₃ | CH₂OEt | CH | 1 | |
| A-317 | 3-F-Ph | Me | Me | 3-CF₃ | C₂H₄OMe | CH | 1 | |
| A-318 | 3-Cl-Ph | Me | Me | 3-CF₃ | H | CH | 1 | NMR |
| A-319 | 3-Cl-Ph | Me | Me | 3-CF₃ | H | N | 1 | NMR |
| A-320 | 3-Cl-Ph | Me | Me | 3-Cl | H | CH | 1 | |
| A-321 | 3-Cl-Ph | Me | Me | 3-Cl | H | N | 1 | |
| A-322 | 3-Me-Ph | Me | Me | 3-CF₃ | H | CH | 1 | NMR |
| A-323 | 3-Me-Ph | Me | Me | 3-CF₃ | H | N | 1 | NMR |
| A-324 | 3-Me-Ph | Me | Me | 3-Cl | H | CH | 1 | NMR |
| A-325 | 3-Me-Ph | Me | Me | 3-CHF₂ | H | N | 1 | |
| A-326 | 3-CF₃-Ph | Me | Me | 3-CF₃ | H | CH | 1 | |
| A-327 | 3-CF₃-Ph | Me | Me | 3-CF₃ | H | N | 1 | NMR |
| A-328 | 3-OMe-Ph | Me | Me | 3-CF₃ | H | CH | 1 | NMR |
| A-329 | 3-OMe-Ph | Me | Me | 3-CF₃ | H | N | 1 | NMR |
| A-330 | 3-OMe-Ph | Me | Me | 3-Cl | H | CH | 1 | NMR |
| A-331 | 3-OMe-Ph | Me | Me | 3-CHF₂ | H | N | 1 | |
| A-332 | 2,3-diCl-Ph | Me | Me | 3-CF₃ | H | CH | 1 | NMR |
| A-333 | 2,3-diCl-Ph | Me | Me | 3-CF₃ | H | N | 1 | NMR |
| A-334 | 2,5-diCl-Ph | Me | Me | 3-CF₃ | H | CH | 1 | NMR |
| A-335 | 2,5-diCl-Ph | Me | Me | 3-CF₃ | H | N | 1 | NMR |
| A-336 | 2,3-diF-Ph | Me | Me | 3-CF₃ | H | CH | 1 | NMR |
| A-337 | 2,3-diF-Ph | Me | Me | 3-CF₃ | H | N | 1 | NMR |
| A-338 | 2,5-diF-Ph | Me | Me | 3-CF₃ | H | CH | 1 | |
| A-339 | 2,5-diF-Ph | Me | Me | 3-CF₃ | H | N | 1 | |
| A-340 | 2,3-diMe-Ph | Me | Me | 3-CF₃ | H | CH | 1 | NMR |
| A-341 | 2,3-diMe-Ph | Me | Me | 3-CF₃ | H | N | 1 | NMR |
| A-342 | 2,5-diMe-Ph | Me | Me | 3-CF₃ | H | CH | 1 | |
| A-343 | 2,5-diMe-Ph | Me | Me | 3-CF₃ | H | N | 1 | |
| A-344 | 2,4-diF-Ph | Me | Me | 3-CI | H | CH | 1 | NMR |
| A-345 | 2,6-diCl-Ph | Me | Me | 3-CF₃ | H | CH | 1 | |
| A-346 | 2,6-diCl-Ph | Me | Me | 3-CF₃ | H | N | 1 | |
| A-347 | 2,6-diMe-Ph | Me | Me | 3-CF₃ | H | CH | 1 | |
| A-348 | 2,6-diMe-Ph | Me | Me | 3-CF₃ | H | N | 1 | |
| A-349 | 2-Cl-3-F-Ph | Me | Me | 3-CF₃ | H | CH | 1 | NMR |
| A-350 | 2-Cl-3-F-Ph | Me | Me | 3-CF₃ | H | N | 1 | NMR |
| A-351 | 2-Cl-5-F-Ph | Me | Me | 3-CF₃ | H | CH | 1 | NMR |
| A-352 | 2-Cl-5-F-Ph | Me | Me | 3-CF₃ | H | N | 1 | NMR |
| A-353 | 2-Me-3-F-Ph | Me | Me | 3-CF₃ | H | CH | 1 | |
| A-354 | 2-Me-3-F-Ph | Me | Me | 3-CF₃ | H | N | 1 | |
| A-355 | 2-Me-5-F-Ph | Me | Me | 3-CF₃ | H | CH | 1 | |
| A-356 | 2-Me-5-F-Ph | Me | Me | 3-CF₃ | H | N | 1 | |
| A-357 | 2-CF₃-3-F-Ph | Me | Me | 3-CF₃ | H | CH | 1 | |
| A-358 | 2-CF₃-3-F-Ph | Me | Me | 3-CF₃ | H | N | 1 | |
| A-359 | 3,5-bis(CF₃)-Ph | Me | Me | 3-CF₃ | H | CH | 1 | |
| A-360 | 3,5-bis(CF₃)-Ph | Me | Me | 3-CF₃ | H | N | 1 | NMR |
| A-361 | 2-Et-Ph | Me | Me | 3-CF₃ | H | CH | 1 | |
| A-362 | 2-Et-Ph | Me | Me | 3-CF₃ | H | N | 1 | |
| A-363 | 2-iPr-Ph | Me | Me | 3-CF₃ | H | CH | 1 | NMR |
| A-364 | 2-iPr-Ph | Me | Me | 3-CF₃ | H | N | 1 | NMR |
| A-365 | 2-CH₂CH(Me)₂-Ph | Me | Me | 3-CF₃ | H | CH | 1 | |
| A-366 | 2-CH₂CH(Me)₂-Ph | Me | Me | 3-CF₃ | CH₂OMe | CH | 1 | |
| A-367 | 2-CH₂CH(Me)₂-Ph | Me | Me | 3-CF₃ | CO₂tBu | CH | 1 | |
| A-368 | 2-nBu-Ph | Me | Me | 3-CF₃ | H | CH | 1 | |
| A-369 | 2-nBu-Ph | Me | Me | 3-CF₃ | CH₂OMe | CH | 1 | |
| A-370 | 2-nBu-Ph | Me | Me | 3-CF₃ | CO₂tBu | CH | 1 | |
| A-371 | 2-CH=CH₂-Ph | Me | Me | 3-CF₃ | H | CH | 1 | |
| A-372 | 2-CH=CH₂-Ph | Me | Me | 3-CF₃ | CH₂OMe | CH | 1 | |
| A-373 | 2-CH=CH₂-Ph | Me | Me | 3-CF₃ | CO₂tBu | CH | 1 | |
| A-374 | 2-C(Me)=CH₂-Ph | Me | Me | 3-CF₃ | H | CH | 1 | |
| A-375 | 2-C(Me)=CH₂-Ph | Me | Me | 3-CF₃ | CH₂OMe | CH | 1 | |
| A-376 | 2-C(Me)=CH₂-Ph | Me | Me | 3-CF₃ | CO₂tBu | CH | 1 | |
| A-377 | 2-CH=C(Me)₂-Ph | Me | Me | 3-CF₃ | H | CH | 1 | |
| A-378 | 2-CH=C(Me)₂-Ph | Me | Me | 3-CF₃ | CH₂OMe | CH | 1 | |
| A-379 | 2-CH=C(Me)₂-Ph | Me | Me | 3-CF₃ | CO₂tBu | CH | 1 | |
| A-380 | 2-C≡CH-Ph | Me | Me | 3-CF₃ | H | CH | 1 | |
| A-381 | 2-C≡CH-Ph | Me | Me | 3-CF₃ | CH₂OMe | CH | 1 | |
| A-382 | 2-C≡CH-Ph | Me | Me | 3-CF₃ | CO₂tBu | CH | 1 | |
| A-383 | 2-C=C(Me)-Ph | Me | Me | 3-CF₃ | H | CH | 1 | |
| A-384 | 2-C=C(Me)-Ph | Me | Me | 3-CF₃ | CH₂OMe | CH | 1 | |
| A-385 | 2-C=C(Me)-Ph | Me | Me | 3-CF₃ | CO₂tBu | CH | 1 | |
| A-386 | 2-C=C(tBu)-Ph | Me | Me | 3-CF₃ | H | CH | 1 | |
| A-387 | 2-C=C(tBu)-Ph | Me | Me | 3-CF₃ | CH₂OMe | CH | 1 | |
| A-388 | 2-C=C(tBu)-Ph | Me | Me | 3-CF₃ | CO₂tBu | CH | 1 | |
| A-389 | 2-cPr-Ph | Me | Me | 3-CF₃ | H | CH | 1 | NMR |
| A-390 | 2-cPr-Ph | Me | Me | 3-CF₃ | CH₂OMe | CH | 1 | |
| A-391 | 2-cPr-Ph | Me | Me | 3-CF₃ | CO₂tBu | CH | 1 | |
| A-392 | 2-OEt-Ph | Me | Me | 3-CF₃ | H | CH | 1 | |
| A-393 | 2-OEt-Ph | Me | Me | 3-CF₃ | H | N | 1 | |
| A-394 | 2-O(iPr)-Ph | Me | Me | 3-CF₃ | H | CH | 1 | |
| A-395 | 2-O(iPr)-Ph | Me | Me | 3-CF₃ | H | N | 1 | |
| A-396 | 2-O(nPr)-Ph | Me | Me | 3-CF₃ | H | CH | 1 | |
| A-397 | 2-O(nPr)-Ph | Me | Me | 3-CF₃ | H | N | 1 | |
| A-398 | 2-OCH₂CH(Me)₂-Ph | Me | Me | 3-CF₃ | H | CH | 1 | |
| A-399 | 2-OCH₂CH(Me)₂-Ph | Me | Me | 3-CF₃ | H | N | 1 | |
| A-400 | 2-OCH₂CH=CH₂-Ph | Me | Me | 3-CF₃ | H | CH | 1 | |
| A-401 | 2-OCH₂CH=CH₂-Ph | Me | Me | 3-CF₃ | H | N | 1 | |
| A-402 | 2-OCH₂C≡CH-Ph | Me | Me | 3-CF₃ | H | CH | 1 | |
| A-403 | 2-OCH₂C≡CH-Ph | Me | Me | 3-CF₃ | H | N | 1 | |
| A-404 | 2-OCHF₂-Ph | Me | Me | 3-CF₃ | H | CH | 1 | |
| A-405 | 2-OCHF₂-Ph | Me | Me | 3-CF₃ | H | N | 1 | |
| A-406 | 2-OCH₂CHF₂-Ph | Me | Me | 3-CF₃ | H | CH | 1 | |
| A-407 | 2-OCH₂CHF₂-Ph | Me | Me | 3-CF₃ | H | N | 1 | |
| A-408 | 2-OCH₂CF₃-Ph | Me | Me | 3-CF₃ | H | CH | 1 | |
| A-409 | 2-OCH₂CF₃-Ph | Me | Me | 3-CF₃ | H | N | 1 | |
| A-410 | 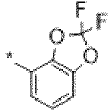 | Me | Me | 3-CF₃ | H | CH | 1 | NMR |
| A-411 | 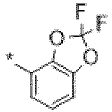 | Me | Me | 3-CF₃ | H | N | 1 | NMR |
| A-412 | 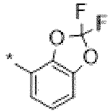 | Me | Me | 3-Cl | H | CH | 1 | NMR |
| A-413 | 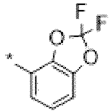 | Me | Me | 3-Cl | H | N | 1 | NMR |
| A-414 | 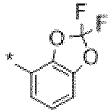 | Me | Me | 3-Br | H | CH | 1 | NMR |
| A-415 | 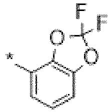 | Me | Me | 3-Br | H | N | 1 | |
| A-416 | 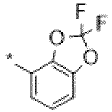 | Me | Me | 3-CN | H | CH | 1 | NMR |
| A-417 | 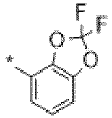 | Me | Me | 3-CN | H | N | 1 | NMR |
| A-418 | 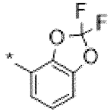 | Me | Me | 3-NO₂ | H | CH | 1 | |
| A-419 | 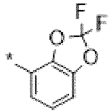 | Me | Me | 3-NO₂ | H | N | 1 | NMR |
| A-420 | 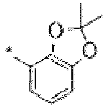 | Me | Me | 3-CF₃ | H | CH | 1 | |
| A-421 | 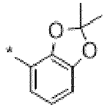 | Me | Me | 3-CF₃ | H | N | 1 | |
| A-422 | 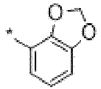 | Me | Me | 3-CF₃ | H | CH | 1 | |
| A-423 | 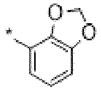 | Me | Me | 3-CF₃ | H | N | 1 | |
| A-424 | 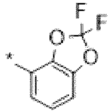 | Me | Me | 3-CHF₂ | H | N | 1 | NMR |
| A-425 | 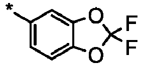 | Me | Me | 3-Cl | H | CH | 1 | |
| A-426 | 2-Cl-Ph | Me | Me | 3-CF₃ | CH₂Ph | CH | 1 | |
| A-427 | 2-Cl-Ph | Me | Me | 3-NO₂ | H | CCN | 1 | |
| A-428 | 2-Cl-Ph | Me | Me | 3-CHF₂-5-CHF₂ | H | CH | 2 | |
| A-429 | 2-Me-Ph | Me | Me | 3-CF₃-5-CF₃ | H | CH | 2 | |
| A-430 | 3-F-Ph | Me | Me | 3-SMe | H | CH | 1 | NMR |
| A-431 | 3-F-Ph | Me | Me | 3-Et | H | CH | 1 | |
| A-432 | 3-F-Ph | Me | Me | 3-CH=CH₂ | H | CH | 1 | |
| A-433 | 3-F-Ph | Me | Me | 3-trans-CH=CHCH₃ | H | CH | 1 | |
| A-434 | 3-F-Ph | Me | Me | 3-nPr | H | CH | 1 | |
| A-435 | 3-F-Ph | Me | Me | 3-C≡CH | H | CH | 1 | |
| A-436 | 3-F-Ph | Me | Me | 3-C≡C(CH₂)₃CH₃ | H | CH | 1 | |
| A-437 | 3-F-Ph | Me | Me | 3-nHex | H | CH | 1 | |

**TABLE 2**

| 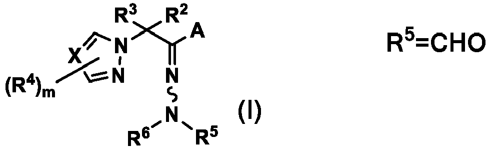 | | | | | | | | |
|---|---|---|---|---|---|---|---|---|
| No. | A | R² | R³ | R⁴ | R⁶ | X | m | Physical properties |
| B-1 | Ph | Cyclopropane | | 3-CF₃ | H | CH | 1 | NMR |
| B-2 | Ph | Cyclopropane | | 3-CF₃ | H | CH | 1 | NMR |
| B-3 | Ph | Cyclopropane | | 3-CF₃ | H | N | 1 | NMR |
| B-4 | Ph | Cyclopropane | | 3-CF₃ | H | N | 1 | NMR |
| B-5 | Ph | Cyclopropane | | 3-CF₃ | Me | CH | 1 | |
| B-6 | Ph | Cyclopropane | | 3-CF₃ | Me | N | 1 | NMR |
| B-7 | Ph | Cyclopropane | | 3-CF₃ | Et | CH | 1 | |
| B-8 | Ph | Cyclopropane | | 3-CF₃ | Et | N | 1 | |
| B-9 | Ph | Cyclopropane | | 3-CF₃ | nPr | CH | 1 | |
| B-10 | Ph | Cyclopropane | | 3-CF₃ | nPr | N | 1 | |
| B-11 | Ph | Cyclopropane | | 3-CF₃ | nHexyl | CH | 1 | |
| B-12 | Ph | Cyclopropane | | 3-CF₃ | nHexyl | N | 1 | |
| B-13 | Ph | Cyclopropane | | 3-CF₃ | iPr | CH | 1 | |
| B-14 | Ph | Cyclopropane | | 3-CF₃ | iPr | N | 1 | |
| B-15 | Ph | Cyclopropane | | 3-CF₃ | CH₂CH=CH₂ | CH | 1 | |
| B-16 | Ph | Cyclopropane | | 3-CF₃ | CH₂CH=CH₂ | N | 1 | |
| B-17 | Ph | Cyclopropane | | 3-CF₃ | CH₂CH=C(Me)₂ | CH | 1 | |
| B-18 | Ph | Cyclopropane | | 3-CF₃ | CH₂CH=C(Me)₂ | N | 1 | |
| B-19 | Ph | Cyclopropane | | 3-CF₃ | CH₂C≡CH | CH | 1 | |
| B-20 | Ph | Cyclopropane | | 3-CF₃ | CH₂C≡CH | N | 1 | NMR |
| B-21 | Ph | Cyclopropane | | 3-CF₃ | CH₂C≡CMe | CH | 1 | |
| B-22 | Ph | Cyclopropane | | 3-CF₃ | CH₂C≡CMe | N | 1 | |
| B-23 | Ph | Cyclopropane | | 3-CF₃ | CH₂OMe | CH | 1 | |
| B-24 | Ph | Cyclopropane | | 3-CF₃ | CH₂OMe | N | 1 | NMR |
| B-25 | Ph | Cyclopropane | | 3-CF₃ | CH₂OEt | CH | 1 | |
| B-26 | Ph | Cyclopropane | | 3-CF₃ | CH₂OEt | N | 1 | |
| B-27 | Ph | Cyclopropane | | 3-CF₃ | C₂H₄OMe | CH | 1 | |
| B-28 | Ph | Cyclopropane | | 3-CF₃ | C₂H₄OMe | N | 1 | NMR |
| B-29 | Ph | Cyclopropane | | 3-CF₃ | SO₂CF₃ | CH | 1 | |
| B-30 | Ph | Cyclopropane | | 3-CF₃ | SO₂CF₃ | N | 1 | |
| B-31 | Ph | Cyclopropane | | 3-CHF₂ | H | CH | 1 | |
| B-32 | Ph | Cyclopropane | | 3-CF₂CF₃ | H | CH | 1 | |
| B-33 | Ph | Cyclopropane | | 3-OMe | H | CH | 1 | |

| No. | A | R² | R³ | R⁴ | R⁶ | X | m | Physical properties |
|---|---|---|---|---|---|---|---|---|
| B-34 | Ph | cyclopropane | | 3-OMe | H | N | 1 | |
| B-35 | Ph | cyclopropane | | 3-O(iPr) | H | CH | 1 | |
| B-36 | Ph | cyclopropane | | 3-O(nHexyl) | H | CH | 1 | |
| B-37 | Ph | cyclopropane | | 3-OCHF₂ | H | CH | 1 | |
| B-38 | Ph | cyclopropane | | 3-OCF₃ | H | CH | 1 | |
| B-39 | Ph | cyclopropane | | 3-OCF₂CF₃ | H | CH | 1 | |
| B-40 | Ph | cyclopropane | | 3-OCH(CF₃)₂ | H | CH | 1 | |
| B-41 | Ph | cyclopropane | | 3-OCH₂CF₃ | H | CH | 1 | |
| B-42 | Ph | cyclopropane | | 3-F | H | CH | 1 | |
| B-43 | Ph | cyclopropane | | 3-Cl | H | CH | 1 | |
| B-44 | Ph | cyclopropane | | 3-Cl | H | N | 1 | |
| B-45 | Ph | cyclopropane | | 3-Br | H | CH | 1 | |
| B-46 | Ph | cyclopropane | | 3-Br | H | N | 1 | |
| B-47 | Ph | cyclopropane | | 3-I | H | CH | 1 | |
| B-48 | Ph | cyclopropane | | 3-NO₂ | H | CH | 1 | |
| B-49 | Ph | cyclopropane | | 3-NO₂ | H | N | 1 | |
| B-50 | Ph | cyclopropane | | 3-CN | H | CH | 1 | |
| B-51 | Ph | cyclopropane | | 3-CN | H | N | 1 | |
| B-52 | Ph | cyclopropane | | 3-SMe | H | CH | 1 | |
| B-53 | Ph | cyclopropane | | 3-SMe | H | N | 1 | |
| B-54 | Ph | cyclopropane | | 3-S(iPr) | H | N | 1 | |
| B-55 | Ph | cyclopropane | | 3-S(nHexyl) | H | N | 1 | |
| B-56 | Ph | cyclopropane | | 3-SOMe | H | CH | 1 | |
| B-57 | Ph | cyclopropane | | 3-SOMe | H | N | 1 | |
| B-58 | Ph | cyclopropane | | 3-SO₂Me | H | CH | 1 | |
| B-59 | Ph | cyclopropane | | 3-SO₂Me | H | N | 1 | |
| B-60 | Ph | Cyclopropane | | 3-CF₃ | H | CCl | 1 | |
| B-61 | Ph | Cyclopropane | | 3-CF₃ | H | CBr | 1 | |
| B-62 | Ph | Cyclopropane | | 3-CF₃ | H | CMe | 1 | |
| B-63 | Ph | Cyclopropane | | 3-CF₃ | H | C-C≡CH | 1 | |
| B-64 | Ph | Cyclopropane | | 3-CF₃ | H | C-cPr | 1 | |
| B-65 | Ph | Cyclopropane | | 3-CF₃-5-Me | H | CH | 2 | |
| B-66 | Ph | Cyclopropane | | 3-Cl | H | CNO₂ | 1 | |
| B-67 | Ph | Cyclopropane | | 3-Cl | H | CCl | 1 | |
| B-68 | Ph | Cyclopropane | | 3-Cl | H | CBr | 1 | |
| B-69 | Ph | Cyclopropane | | 3-C l | H | CMe | 1 | |
| B-70 | Ph | Cyclopropane | | 3-Cl | H | CEt | 1 | |
| B-71 | Ph | Cyclopropane | | 3-Cl | H | C-iPr | 1 | |
| B-72 | Ph | Cyclopropane | | 3-Cl | H | C-nHexyl | 1 | |
| B-73 | Ph | Cyclopropane | | 3-Cl | H | C-CH=CH₂ | 1 | |
| B-74 | Ph | Cyclopropane | | 3-Cl | H | C-C(Me)=CH₂ | 1 | |
| B-75 | Ph | Cyclopropane | | 3-C l | H | C-C≡CH | 1 | |
| B-76 | Ph | Cyclopropane | | 3-C l | H | C-cPr | 1 | |
| B-77 | Ph | Cyclopropane | | 3-C l | H | CCF₃ | 1 | |
| B-78 | Ph | Cyclopropane | | - | H | CCF₃ | 0 | |
| B-79 | Ph | Cyclopropane | | - | H | CCl | 0 | |
| B-80 | Ph | Cyclopropane | | - | H | CF | 0 | |
| B-81 | Ph | Cyclobutane | | 3-CF₃ | H | CH | 1 | NMR |
| B-82 | Ph | Cyclobutane | | 3-CF₃ | H | N | 1 | |
| B-83 | Ph | Cyclopentane | | 3-CF₃ | H | CH | 1 | NMR |
| B-84 | Ph | Cyclopentane | | 3-CF₃ | H | N | 1 | |
| B-85 | Ph | Cyclohexane | | 3-CF₃ | H | CH | 1 | |
| B-86 | Ph | Cyclohexane | | 3-CF₃ | H | N | 1 | |
| B-87 | 4-Cl-Ph | Cyclopropane | | 3-CF₃ | H | CH | 1 | NMR |
| B-88 | 4-Cl-Ph | Cyclopropane | | 3-CF₃ | H | N | 1 | |
| B-89 | 4-Cl-Ph | Cyclopropane | | 3-CF₃ | Me | CH | 1 | |
| B-90 | 4-Cl-Ph | Cyclopropane | | 3-CF₃ | Et | CH | 1 | |
| B-91 | 4-Cl-Ph | Cyclopropane | | 3-CF₃ | CH₂OMe | CH | 1 | NMR |
| B-92 | 4-Cl-Ph | Cyclopropane | | 3-CF₃ | Me | N | 1 | |
| B-93 | 4-Cl-Ph | Cyclopropane | | 3-CF₃ | Et | N | 1 | |
| B-94 | 4-Cl-Ph | Cyclopropane | | 3-CF₃ | nPr | N | 1 | |
| B-95 | 4-Cl-Ph | Cyclopropane | | 3-CF₃ | nHexyl | N | 1 | |
| B-96 | 4-Cl-Ph | Cyclopropane | | 3-CF₃ | iPr | N | 1 | |
| B-97 | 4-Cl-Ph | Cyclopropane | | 3-CF₃ | CH₂OMe | N | 1 | |
| B-98 | 4-Cl-Ph | Cyclopropane | | 3-CF₃ | CH₂OEt | N | 1 | |
| B-99 | 4-Cl-Ph | Cyclopropane | | 3-CF₃ | C₂H₄OMe | N | 1 | |
| B-100 | 4-Cl-Ph | Cyclopropane | | 3-CF₃ | SO₂CF₃ | N | 1 | |
| B-101 | 4-Cl-Ph | Cyclopropane | | 3-OMe | H | CH | 1 | |
| B-102 | 4-Cl-Ph | Cyclopropane | | 3-OMe | H | N | 1 | |
| B-103 | 4-Cl-Ph | Cyclopropane | | 3-CI | H | CH | 1 | |
| B-104 | 4-Cl-Ph | Cyclopropane | | 3-CI | H | N | 1 | |
| B-105 | 4-F-Ph | Cyclopropane | | 3-CF₃ | H | CH | 1 | NMR |
| B-106 | 4-F-Ph | Cyclopropane | | 3-CF₃ | H | N | 1 | NMR |
| B-107 | 4-F-Ph | Cyclopropane | | 3-CF₃ | CH₂OMe | CH | 1 | |
| B-108 | 4-F-Ph | Cyclopropane | | 3-CF₃ | CH₂OMe | N | 1 | |
| B-109 | 4-F-Ph | Cyclopropane | | 3-CF₃ | CH₂Ph | CH | 1 | |
| B-110 | 4-F-Ph | Cyclopropane | | 3-CF₃ | CH₂Ph | N | 1 | |
| B-111 | 4-F-Ph | Cyclopropane | | 3-CF₃ | CH₂-(4-OMe-Ph) | CH | 1 | |
| B-112 | 4-F-Ph | Cyclopropane | | 3-CF₃ | CH₂-(4-OMe-Ph) | N | 1 | |
| B-113 | 4-F-Ph | Cyclopropane | | 3-CF₃ | CO₂tBu | CH | 1 | |
| B-114 | 4-F-Ph | Cyclopropane | | 3-CF₃ | CO₂tBu | N | 1 | |
| B-115 | 4-F-Ph | Cyclopropane | | 3-CF₃ | CO₂CH₂Ph | CH | 1 | |
| B-116 | 4-F-Ph | Cyclopropane | | 3-CF₃ | CO₂CH₂Ph | N | 1 | |
| B-117 | 4-F-Ph | Cyclopropane | | 3-OMe | H | CH | 1 | |
| B-118 | 4-F-Ph | Cyclopropane | | 3-OMe | H | N | 1 | |
| B-119 | 4-F-Ph | Cyclopropane | | 3-CI | H | CH | 1 | |
| B-120 | 4-F-Ph | Cyclopropane | | 3-CI | H | N | 1 | |
| B-121 | 4-F-Ph | cyclopropane | | 3-CF₃ | H | CCl | 1 | |
| B-122 | 4-F-Ph | cyclopropane | | 3-Cl | H | CBr | 1 | |
| B-123 | 4-F-Ph | cyclopropane | | - | H | CF | 0 | |
| B-124 | 4-Br-Ph | cyclopropane | | 3-CF₃ | H | CH | 1 | |
| B-125 | 4-Br-Ph | cyclopropane | | 3-CF₃ | H | N | 1 | |
| B-126 | 4-Br-Ph | cyclopropane | | 3-CF₃ | CH₂OMe | CH | 1 | |
| B-127 | 4-Br-Ph | cyclopropane | | 3-CF₃ | CH₂OMe | N | 1 | |
| B-128 | 4-I-Ph | cyclopropane | | 3-CF₃ | H | CH | 1 | |
| B-129 | 4-I-Ph | cyclopropane | | 3-CF₃ | H | N | 1 | |
| B-130 | 4-CF₃-Ph | cyclopropane | | 3-CF₃ | H | CH | 1 | |
| B-131 | 4-CF₃-Ph | cyclopropane | | 3-CF₃ | H | N | 1 | |
| B-132 | 4-OMe-Ph | cyclopropane | | 3-CF₃ | H | CH | 1 | |
| B-133 | 4-OMe-Ph | cyclopropane | | 3-CF₃ | H | N | 1 | |
| B-134 | 4-O(iPr)-Ph | cyclopropane | | 3-CF₃ | H | CH | 1 | |
| B-135 | 4-O(iPr)-Ph | cyclopropane | | 3-CF₃ | H | N | 1 | |
| B-136 | 4-O(nHexyl)-Ph | cyclopropane | | 3-CF₃ | H | CH | 1 | |
| B-137 | 4-O(nHexyl)-Ph | cyclopropane | | 3-CF₃ | H | N | 1 | |
| B-138 | 4-OCHF₂-Ph | cyclopropane | | 3-CF₃ | H | CH | 1 | |
| B-139 | 4-OCHF₂-Ph | cyclopropane | | 3-CF₃ | H | N | 1 | |
| B-140 | 4-OCF₃-Ph | cyclopropane | | 3-CF₃ | H | CH | 1 | |
| B-141 | 4-OCF₃-Ph | cyclopropane | | 3-CF₃ | H | N | 1 | |
| B-142 | 4-OCF₂CF₃-Ph | cyclopropane | | 3-CF₃ | H | CH | 1 | |
| B-143 | 4-OCF₂CF₃-Ph | cyclopropane | | 3-CF₃ | H | N | 1 | |
| B-144 | 4-OCH(CF₃)₂-Ph | cyclopropane | | 3-CF₃ | H | CH | 1 | |
| B-145 | 4-OCH(CF₃)₂-Ph | cyclopropane | | 3-CF₃ | H | N | 1 | |
| B-146 | 4-OCH₂CF₃-Ph | cyclopropane | | 3-CF₃ | H | CH | 1 | |
| B-147 | 4-OCH₂CF₃-Ph | cyclopropane | | 3-CF₃ | H | N | 1 | |
| B-148 | 2-Cl-Ph | cyclopropane | | 3-CF₃ | H | CH | 1 | NMR |
| B-149 | 2-Cl-Ph | cyclopropane | | 3-CF₃ | H | N | 1 | |
| B-150 | 2-F-Ph | cyclopropane | | 3-CF₃ | H | CH | 1 | |
| B-151 | 2-F-Ph | cyclopropane | | 3-CF₃ | H | N | 1 | |
| B-152 | 2,4-diCl-Ph | cyclopropane | | 3-CF₃ | H | CH | 1 | |
| B-153 | 2,4-diCl-Ph | cyclopropane | | 3-CF₃ | H | N | 1 | |
| B-154 | 2,4-diF-Ph | cyclopropane | | 3-CF₃ | H | CH | 1 | |
| B-155 | 2,4-diF-Ph | cyclopropane | | 3-CF₃ | H | N | 1 | |
| B-156 | 3,5-diF-Ph | cyclopropane | | 3-CF₃ | H | CH | 1 | |
| B-157 | 3,5-diF-Ph | cyclopropane | | 3-CF₃ | H | N | 1 | |
| B-158 | 2,6-diF-Ph | cyclopropane | | 3-CF₃ | H | CH | 1 | |
| B-159 | 2,6-diF-Ph | cyclopropane | | 3-CF₃ | H | N | 1 | |
| B-160 | 3,4,5-triF-Ph | cyclopropane | | 3-CF₃ | H | CH | 1 | |
| B-161 | 3,4,5-triF-Ph | cyclopropane | | 3-CF₃ | H | N | 1 | |
| B-162 | 4-Et-Ph | cyclopropane | | 3-CF₃ | H | CH | 1 | |
| B-163 | 4-Et-Ph | cyclopropane | | 3-CF₃ | H | N | 1 | |
| B-164 | 4-iPr-Ph | cyclopropane | | 3-CF₃ | H | CH | 1 | |
| B-165 | 4-iPr-Ph | cyclopropane | | 3-CF₃ | H | N | 1 | |
| B-166 | 4-CH₂CH(Me)₂-Ph | cyclopropane | | 3-CF₃ | H | CH | 1 | |
| B-167 | 4-CH₂CH(Me)₂-Ph | cyclopropane | | 3-CF₃ | H | N | 1 | |
| B-168 | 4-nHexyl-Ph | cyclopropane | | 3-CF₃ | H | CH | 1 | |
| B-169 | 4-nHexyl-Ph | cyclopropane | | 3-CF₃ | H | N | 1 | |
| B-170 | 4-CH=CH₂-Ph | cyclopropane | | 3-CF₃ | H | CH | 1 | |
| B-171 | 4-CH=CH₂-Ph | cyclopropane | | 3-CF₃ | H | N | 1 | |
| B-172 | 4-C(Me)=CH₂-Ph | cyclopropane | | 3-CF₃ | H | CH | 1 | |
| B-173 | 4-C(Me)=CH₂-Ph | cyclopropane | | 3-CF₃ | H | N | 1 | |
| B-174 | 4-CH=C(Me)₂-Ph | cyclopropane | | 3-CF₃ | H | CH | 1 | |
| B-175 | 4-CH=C(Me)₂-Ph | cyclopropane | | 3-CF₃ | H | N | 1 | |
| B-176 | 4-C≡CH-Ph | cyclopropane | | 3-CF₃ | H | CH | 1 | |
| B-177 | 4-C≡CH-Ph | cyclopropane | | 3-CF₃ | H | N | 1 | |
| B-178 | 4-cPr-Ph | cyclopropane | | 3-CF₃ | H | CH | 1 | |
| B-179 | 4-cPr-Ph | cyclopropane | | 3-CF₃ | H | N | 1 | |
| B-180 | 2-Me-4-Cl-Ph | cyclopropane | | 3-CF₃ | H | CH | 1 | |
| B-181 | 2-Me-4-Cl-Ph | cyclopropane | | 3-CF₃ | H | N | 1 | |
| B-182 | 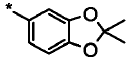 | Cyclopropane | | 3-CF₃ | H | CH | 1 | |
| B-183 | 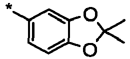 | Cyclopropane | | 3-CF₃ | H | N | 1 | |
| B-184 | 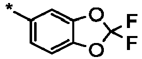 | Cyclopropane | | 3-CF₃ | H | CH | 1 | |
| B-185 | 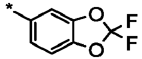 | Cyclopropane | | 3-CF₃ | H | N | 1 | |
| B-186 | Ph | Cyclopropane | | 3-CF₃ | H | CH | 1 | Na salt |
| B-187 | Ph | Cyclopropane | | 3-CF₃ | H | N | 1 | Na salt |
| B-188 | Ph | Cyclopropane | | 3-CF₃ | H | CH | 1 | K salt |
| B-189 | Ph | Cyclopropane | | 3-CF₃ | H | N | 1 | K salt |
| B-190 | 4-Cl-Ph | Cyclopropane | | 3-CF₃ | H | CH | 1 | NMR |
| B-191 | 4-Cl-Ph | Cyclopropane | | 3-CF₃ | nPr | CH | 1 | NMR |
| B-192 | 4-F-Ph | Cyclopropane | | 3-CF₃ | H | CH | 1 | NMR |
| B-193 | 4-F-Ph | Cyclopropane | | 3-CF₃ | H | N | 1 | NMR |
| B-194 | 2-Cl-Ph | Cyclopropane | | 3-CI | H | CH | 1 | |
| B-195 | 2-Cl-Ph | Cyclopropane | | 3-CF₃ | H | CH | 1 | NMR |
| B-196 | 2-Me-Ph | Cyclopropane | | 3-CF₃ | H | CH | 1 | NMR |
| B-197 | 2-Me-Ph | Cyclopropane | | 3-CF₃ | H | CH | 1 | NMR |
| B-198 | 2-CF₃-Ph | Cyclopropane | | 3-CF₃ | H | CH | 1 | NMR |
| B-199 | 3-F-Ph | Cyclopropane | | 3-CF₃ | H | CH | 1 | NMR |
| B-200 | 3-F-Ph | Cyclopropane | | 3-CF₃ | CH₂C≡CH | CH | 1 | |
| B-201 | 3-F-Ph | Cyclopropane | | 3-Cl | H | CH | 1 | NMR |
| B-202 | 3-F-Ph | Cyclopropane | | 3-Cl | CH₂C≡CH | CH | 1 | |
| B-203 | 3-Cl-Ph | Cyclopropane | | 3-CF₃ | H | CH | 1 | |
| B-204 | 3-Cl-Ph | Cyclopropane | | 3-CF₃ | CH₂C≡CH | CH | 1 | |

**TABLE 3**

| No. | ¹H-NMR (300MHz or 500MHz/solvent) δ (ppm) |
|---|---|
| A-1 | (300MHz/CDCl₃) 8.73 (d, 1H), 8.03 (d, 1H), 7.43-7.29 (m, 4H), 6.67-6.61 (m, 2H), 6.45 (d, 1H), 1.89 (s, 6H) |
| A-2 | (300MHz/CDCl₃) 8.69 (d, 1H), 8.07-7.99 (m, 1H), 8.05 (d, 1H), 7.47-7.36 (m, 3H), 6.77-6.71 (m, 2H), 1.94 (s, 6H) |
| A-89 | (300MHz/CDCl₃) 8.73 (d, 1H), 7.97 (d, 1H), 7.41-7.38 (m, 1H), 7.36-7.30 (m, 2H), 6.63-6.57 (m, 2H), 6.46 (d, 1H), 1.88 (s, 6H) |
| A-90 | (300MHz/CDCl₃) 8.69 (d, 1H), 8.07 (d, 1H), 7.99 (d, 1H), 7.43-7.38 (m, 2H), 6.74-6.69 (m, 2H), 1.93 (s, 6H) |
| A-91 | (300MHz/CDCl₃) 8.69 (s, 1H), 7.39-7.37 (m, 1H), 7.24-7.18 (m, 2H), 6.61-6.55 (m, 2H), 6.46 (d, 1H), 2.59 (s, 3H), 1.85 (s, 6H) |
| A-92 | (300MHz/CDCl₃) 8.39 (s, 0.58H), 7.59 (s, 0.42H), 7.56 (d, 0.42H), 7.43 (d, 0.58H), 7.27-7.10 (m, 3H), 6.75-6.67 (m, 0.84H), 6.58-6.51 (m, 1.16H), 6.50 (d, 0.58H), 6.46 (d, 0.42H), 3.46 (q, 0.84H), 3.31 (q, 1.16H), 1.93 (s, 2.52H), 1.85 (s, 3.48H), 1.35-1.20 (m, 1.26H), 0.95-0.83 (m, 1.74H) |
| A-94 | (300MHz/CDCl₃) 8.64 (s, 1H), 8.09 (d, 1H), 7.33-7.25 (m, 2H), 6.80-6.74 (m, 2H), 2.59 (s, 3H), 1.92 (s, 6H) |
| A-95 | (300MHz/CDCl₃) 8.40 (s, 0.53H), 8.26 (s, 0.47H), 8.11 (s, 0.53H), 7.56 (s, 0.47H), 7.34-7.27 (m, 1.06H), 7.25-7.17 (m, 0.94H), 6.91-6.83 (m, 0.94H), 6.77-6.69 (m, 1.06H), 3.46 (q, 0.94H), 3.29 (q, 1.06H), 1.98 (s, 2.82H), 1.92 (s, 3.18H), 1.21 (t, 1.41H), 0.84 (t, 1.59H) |
| A-96 | (300MHz/CDCl₃) 8.46 (s, 0.54H), 8.24 (s, 0.46H), 8.09 (s, 0.54H), 7.54 (s, 0.46H), 7.34-7.28 (m, 1.08H), 7.25-7.18 (m, 0.92H), 6.92-6.83 (m, 0.92H), 6.76-6.66 (m, 1.08H), 3.32 (t, 0.92), 3.15 (t, 1.08H), 1.97 (s, 2.76H), 1.91 (s, 3.24H), 1.67-1.54 (m, 0.92H), 1.34-1.20 (m, 1.08H), 0.87 (t, 1.38H), 0.59 (t, 1.62H) |
| A-101 | (300MHz/CDCl₃) 8.32 (s, 0.45H), 8.25 (s, 0.55H), 8.15 (s, 0.45H), 7.54 (s, 0.55H), 7.31 (d, 0.90H), 7.22 (d, 1.10H), 6.90 (d, 1.10H), 6.71 (d, 0.90H), 3.58-3.24 (m, 7H), 1.97 (s, 3.30H), 1.90 (s, 2.70H) |
| A-103 | (300MHz/CDCl₃) 8.72 (d, 1H), 8.02-7.94 (m, 1H), 7.54-7.49 (m, 2H), 7.43 (d, 1H), 7.14-7.08 (m, 2H), 5.77 (d, 1H), 3.95 (s, 3H), 1.40 (s, 6H) |
| A-107 | (300MHz/CDCl₃) 8.73 (d, 1H), 8.08-7.92 (d, 1H), 7.41-7.37 (m, 1H), 7.09-7.00 (m, 2H), 6.70-6.61 (m, 2H), 6.46 (d, 1H), 1.88 (s, 6H) |
| A-109 | (300MHz/CDCl₃) 8.48 (s, 0.52H), 7.71 (s, 0.48H), 7.60 (d, 0.48H), 7.41 (d, 0.52H), 6.99-6.72 (m, 3H), 6.70-6.58 (m, 1H), 6.48 (s, 1H), 4.78 (s, 0.96H), 4.57 (s, 1.04H), 3.21 (s, 1.44H), 3.11 (s, 1.56H), 1.94 (s, 3H), 1.87 (s, 3H) |
| A-150 | (300MHz/CDCl₃) 8.76 (d, 1H), 7.95 (d, 1H), 7.52-7.51 (m, 1H), 7.46 (dd, 1H), 7.35 (ddd, 1H), 7.17 (ddd, 1H), 6.50 (d, 1H), 6.26 (dd, 1H), 2.02 (s, 3H), 1.83 (s, 3H) |
| A-151 | (300MHz/CDCl₃) 8.71 (d, 1H), 8.19 (d, 1H), 7.97 (d, 1H), 7.50 (dd, 1H), 7.42 (ddd, 1H), 7.28 (ddd, 1H), 6.57 (dd, 1H), 2.05 (s, 3H), 1.90 (s, 3H) |
| A-152 | (300MHz/CDCl₃) 8.76 (d, 1H), 7.99 (d, 1H), 7.49-7.45 (m, 1H), 7.45-7.36 (m, 1H), 7.18-7.01 (m, 2H), 6.47 (d, 1H), 6.37-6.30 (m, 1H), 1.99 (s, 3H), 1.82 (s, 3H) |
| A-153 | (300MHz/CDCl₃) 8.72 (d, 1H), 8.14 (d, 1H), 8.03 (d, 1H), 7.51-7.43 (m, 1H), 7.21-7.13 (m, 2H), 6.58-6.53 (m, 1H), 2.01 (s, 3H), 1.90 (s, 3H) |
| A-154 | (300MHz/CDCl₃) 8.75 (d, 1H), 7.93 (d, 1H), 7.53-7.50 (m, 1H), 7.49 (d, 1H), 7.17 (dd, 1H), 6.51 (d, 1H), 6.22 (d, 1H), 2.01 (s, 3H), 1.81 (s, 3H) |
| A-156 | (500MHz/CDCl₃) 8.76 (d, 1H), 7.98 (d, 1H), 7.47 (s, 1H), 6.90 (m, 1H), 6.81 (m, 1H), 6.48 (d, 1H), 6.34 (m, 1H), 1.99 (s, 3H), 1.81 (s, 3H) |
| A-157 | (500MHz/CDCl₃) 8.72 (d, 1H), 8.15 (s, 1H), 8.00 (d, 1H), 6.95 (m, 1H), 6.92 (m, 1H), 6.58 (m, 1H), 2.01 (s, 3H), 1.88 (s, 3H) |
| A-160 | (500MHz/CDCl₃) 8.74 (d, 1H), 8.03 (d, 1H), 7.57 (s, 1H), 7.42-7.36 (m, 1H), 6.89 (t, 2H), 6.34 (d, 1H), 1.97 (s, 6H) |
| A-161 | (500MHz/CDCl₃) 8.71 (d, 1H), 8.24 (s, 1H), 8.10 (d, 1H), 7.50-7.44 (m, 1H), 6.97 (t, 2H), 1.99 (s, 6H) |
| A-182 | (300MHz/CDCl₃) 8.75 (d, 1H), 7.90 (d, 1H), 7.48-7.44 (m, 1H), 7.27-7.24 (m, 1H), 7.09 (dd, 1H), 6.48 (d, 1H), 6.19 (d, 1H), 2.03 (s, 3H), 1.96 (s, 3H), 1.83 (s, 3H) |
| A-193 | (300MHz/CDCl₃) 8.44 (s, 1H), 7.54-7.51 (m, 1H), 7.36 (dd, 1H), 7.28 (ddd, 1H), 7.09 (ddd, 1H), 6.51 (d, 1H), 6.26 (dd, 1H), 3.84-3.72 (m, 1H), 2.90-2.78 (m, 1H), 2.01 (s, 3H), 1.76 (s, 3H), 0.85 (t, 3H) |
| A-195 | (300MHz/CDCl₃) 8.60 (s, 1H), 7.58 (d, 1H), 7.36-7.26 (m, 2H), 7.12-7.06 (m, 1H), 6.52 (d, 1H), 6.37 (dd, 1H), 4.74 (dd, 1H), 3.16 (dd, 1H), 2.08 (t, 1H), 2.02 (s, 3H), 1.79 (s, 3H) |
| A-198 | (300MHz/CDCl₃) 8.73 (d, 1H), 8.15 (s, 1H), 7.96 (d, 1H), 7.50 (dd, 1H), 7.41 (ddd, 1H), 7.26 (ddd, 1H), 6.71 (t, 1H), 6.46 (d, 1H), 2.03 (s, 3H), 1.87 (s, 3H) |
| A-199 | (300MHz/CDCl₃) 8.76 (d, 1H), 7.95 (d, 1H), 7.47 (dd, 1H), 7.36 (ddd, 1H), 7.35 (d, 1H), 7.19 (ddd, 1H), 6.26 (dd, 1H), 6.16 (d, 1H), 1.97 (s, 3H), 1.75 (s, 3H) |
| A-200 | (300MHz/CDCl₃) 8.73 (d, 1H), 7.98 (s, 1H), 7.97 (d, 1H), 7.51 (dd, 1H), 7.42 (ddd, 1H), 7.29 (ddd, 1H), 6.53 (dd, 1H), 2.00 (s, 3H), 1.81 (s, 3H) |
| A-201 | (300MHz/CDCl₃) 8.76 (d, 1H), 7.95 (d, 1H), 7.46 (dd, 1H), 7.36 (ddd, 1H), 7.32 (d, 1H), 7.18 (ddd, 1H), 6.25 (d, 1H), 6.23 (dd, 1H), 1.98 (s, 3H), 1.75 (s, 3H) |
| A-202 | (300MHz/CDCl₃) 8.73 (d, 1H), 7.97 (d, 1H), 7.96 (s, 1H), 7.51 (dd, 1H), 7.42 (ddd, 1H), 7.28 (ddd, 1H), 6.51 (dd, 1H), 2.00 (s, 3H), 1.82 (s, 3H) |
| A-203 | (300MHz/CDCl₃) 8.76 (d, 1H), 7.94 (d, 1H), 7.46 (dd, 1H), 7.35 (ddd, 1H), 7.26 (d, 1H), 7.17 (ddd, 1H), 6.39 (d, 1H), 6.20 (dd, 1H), 1.98 (s, 3H), 1.76 (s, 3H) |
| A-205 | (300MHz/CDCl₃) 8.73 (d, 1H), 7.96 (d, 1H), 7.60 (d, 1H), 7.49 (dd, 1H), 7.40 (ddd, 1H), 7.25 (ddd, 1H), 6.90 (d, 1H), 6.51 (dd, 1H), 2.03 (s, 3H), 1.86 (s, 3H) |
| A-206 | (300MHz/CDCl₃) 8.68 (d, 1H), 8.25 (s, 1H), 7.97 (d, 1H), 7.53 (dd, 1H), 7.46 (ddd, 1H), 7.36 (ddd, 1H), 6.77 (dd, 1H), 2.07 (s, 3H), 1.93 (s, 3H) |
| A-210 | (300MHz/CDCl₃) 8.77 (d, 0.34H), 8.76 (d, 0.66H), 8.03 (d, 0.34H), 7.96 (d, 0.66H), 7.90 (s, 0.66H), 7.72 (s, 0.34H), 7.52-7.47 (m, 1H), 7.43-7.35 (m, 1H), 7.24-7.17 (m, 1H), 6.31 (dd, 0.66H), 6.20 (dd, 0.34H), 2.56 (s, 1.02H), 2.43 (s, 1.98H), 1.99 (s, 1.02H), 1.98 (s, 1.98H), 1.88 (s, 1.02H), 1.78 (s, 1.98H) |
| A-214 | (300MHz/CDCl₃) 8.77 (d, 1H), 7.94 (d, 1H), 7.45 (dd, 1H), 7.34 (ddd, 1H), 7.18 (d, 1H), 7.15 (ddd, 1H), 6.29 (dd, 1H), 5.62 (d, 1H), 3.86 (s, 3H), 1.93 (s, 3H), 1.68 (s, 3H) |
| A-215 | (300MHz/CDCl₃) 8.77 (d, 1H), 7.94 (d, 1H), 7.45 (dd, 1H), 7.37 (d, 1H), 7.33 (ddd, 1H), 7.12 (ddd, 1H), 6.21-6.16 (m, 2H), 2.87 (ddd, 2H), 1.97 (s, 3H), 1.75 (s, 3H), 1.73-1.60 (m, 2H), 1.00 (t, 3H) |
| A-216 | (300MHz/CDCl₃) 8.77 (d, 1H), 7.98 (d, 1H), 7.78 (s, 1H), 7.70 (d, 1H), 7.49 (dd, 1H), 7.38 (ddd, 1H), 7.18 (ddd, 1H), 6.18 (dd, 1H), 2.01 (s, 3H), 1.80 (s, 3H) |
| A-217 | (300MHz/CDCl₃) 8.78 (d, 1H), 7.96 (d, 1H), 7.47 (dd, 1H), 7.41 (d, 1H), 7.40-7.32 (m, 2H), 7.18 (ddd, 1H), 6.18 (dd, 1H), 1.94 (s, 3H), 1.73 (s, 3H) |
| A-221 | (300MHz/CDCl₃) 8.78 (d, 1H), 8.03 (d, 1H), 7.48 (dd, 1H), 7.37 (ddd, 1H), 7.15 (ddd, 1H), 6.37 (s, 1H), 6.00 (dd, 1H), 2.47 (d, 3H), 2.00 (s, 3H), 1.85 (s, 3H) |
| A-222 | (300MHz/CDCl₃) 8.67 (d, 1H), 7.99 (d, 1H), 7.51 (dd, 1H), 7.41 (ddd, 1H), 7.23 (ddd, 1H), 7.04 (s, 1H), 6.47 (dd, 1H), 2.07 (s, 3H), 1.88 (s, 3H) |
| A-223 | (300MHz/CDCl₃) 8.74 (d, 1H), 8.04 (d, 1H), 7.52 (dd, 1H), 7.41 (ddd, 1H), 7.27 (ddd, 1H), 6.49 (dd, 1H), 2.04 (s, 3H), 1.85 (s, 3H) |
| A-224 | (300MHz/CDCl₃) 8.71 (d, 1H), 8.03 (d, 1H), 7.54 (dd, 1H), 7.45 (ddd, 1H), 7.33 (ddd, 1H), 6.67 (dd, 1H), 2.06 (s, 3H), 1.90 (s, 3H) |
| A-225 | (300MHz/CDCl₃) 8.77 (d, 1H), 7.97 (d, 1H), 7.47-7.46 (m, 1H), 7.41 (dd, 1H), 7.32 (ddd, 1H), 7.13 (ddd, 1H), 6.44 (d, 1H), 6.30 (dd, 1H), 2.69-2.38 (m, 2H), 2.30-2.17 (m, 2H), 1.02 (t, 3H), 0.87 (t, 3H) |
| A-226 | (300MHz/CDCl₃) 8.75 (d, 1H), 8.13 (d, 1H), 8.00 (d, 1H), 7.45 (dd, 1H), 7.38 (ddd, 1H), 7.24 (ddd, 1H), 6.59 (dd, 1H), 2.70-2.57 (m, 1H), 2.46-2.34 (m, 2H), 2.31-2.19 (m, 1H), 1.02 (t, 3H), 0.94 (t, 3H) |
| A-233 | (500MHz/CDCl₃) 8.75 (d, 1H), 7.97 (d, 1H), 7.63 (d, 1H), 7.53 (s, 1H), 7.28-7.19 (m, 2H), 6.49 (d, 1H), 6.25 (dd, 1H), 2.01 (s, 3H), 1.84 (s, 3H) |
| A-234 | (500MHz/CDCl₃) 8.71 (d, 1H), 8.21 (s, 1H), 7.98 (d, 1H), 7.67-7.65 (m, 1H), 7.34-7.32 (m, 2H), 6.58-6.56 (m, 1H), 2.05 (s, 3H), 1.90 (s, 3H) |
| A-235 | (500MHz/CDCl₃) 8.17 (s, 1H), 7.54-7.50 (m, 2H), 7.29-7.12 (m, 2H), 6.50 (s, 1H), 6.31 (d, 1H), 4.95 (d, 1H), 4.01 (d, 1H), 3.03 (s, 3H), 2.01 (s, 3H), 1.79 (s, 3H) |
| A-240 | (300MHz/CDCl₃) 8.77 (d, 1H), 7.97 (d, 1H), 7.90 (dd, 1H), 7.59-7.57 (m, 1H), 7.28 (ddd, 1H), 7.10 (ddd, 1H), 6.52 (d, 1H), 6.22 (dd, 1H), 2.05 (s, 3H), 1.89 (s, 3H) |
| A-241 | (300MHz/CDCl₃) 8.72 (d, 1H), 8.26 (s, 1H), 7.98 (d, 1H), 7.93 (dd, 1H), 7.40 (ddd, 1H), 7.17 (ddd, 1H), 6.58 (dd, 1H), 2.10 (s, 3H), 1.93 (s, 3H) |
| A-243 | (300MHz/CDCl₃) 8.86 (s, 0.44H), 7.86-7.84 (m, 0.56H), 7.80 (dd, 0.56H), 7.75 (dd, 0.44H), 7.62-7.61 (m, 0.44H), 7.32 (s, 0.56H), 7.17-7.11 (m, 1H), 7.00-6.91 (m, 1H), 6.52 (d, 0.56H), 6.49 (d, 0.44H), 6.17-6.10 (m, 1H), 2.08 (s, 1.68H), 2.06 (s, 1.32H), 1.92-1.91 (m, 3H), 1.53 (s, 5.04H), 1.50 (s, 3.96H) |
| A-244 | (300MHz/CDCl₃) 8.75 (d, 1H), 7.96 (d, 1H), 7.46-7.44 (m, 1H), 7.32-7.21 (m, 2H), 7.12-7.06 (m, 1H), 6.47 (d, 1H), 6.25 (d, 1H), 2.04 (s, 3H), 1.97 (s, 3H), 1.85 (s, 3H) |
| A-245 | (300MHz/CDCl₃) 8.70 (d, 1H), 8.12 (d, 1H), 7.98 (d, 1H), 7.38-7.32 (m, 1H), 7.30-7.26 (m, 1H), 7.21-7.15 (m, 1H), 6.48 (d, 1H), 2.07 (s, 3H), 1.99 (s, 3H), 1.92 (s, 3H) |
| A-247 | (300MHz/CDCl₃) 8.75 (d, 1H), 7.95 (d, 1H), 7.32-7.23 (m, 3H), 7.13-7.08 (m, 1H), 6.23 (d, 1H), 6.14 (d, 1H), 2.07 (s, 3H), 1.92 (s, 3H), 1.76 (s, 3H) |
| A-248 | (300MHz/CDCl₃) 8.71 (d, 1H), 7.97 (d, 1H), 7.92 (s, 1H), 7.38-7.28 (m, 2H), 7.19 (ddd, 1H), 6.45 (d, 1H), 2.10 (s, 3H), 1.94 (s, 3H), 1.83 (s, 3H) |
| A-249 | (300MHz/CDCl₃) 8.75 (d, 1H), 7.94 (d, 1H), 7.32-7.23 (m, 3H), 7.13-7.08 (m, 1H), 6.24-6.22 (m, 2H), 2.06 (s, 3H), 1.93 (s, 3H), 1.77 (s, 3H) |
| A-251 | (300MHz/CDCl₃) 8.73 (d. 1H), 7.98 (d, 1H), 7.54 (d, 1H), 7.35-7.25 (m, 2H), 7.14 (ddd, 1H), 6.88 (d, 1H), 6.38 (d, 1H), 2.09 (s, 3H), 1.98 (s, 3H), 1.88 (s, 3H) |
| A-252 | (300MHz/CDCl₃) 8.66 (d, 1H), 8.19 (s, 1H), 7.98 (d, 1H), 7.41-7.30 (m, 2H), 7.27-7.21 (m, 1H), 6.61 (d, 1H), 2.13 (s, 3H), 2.00 (s, 3H), 1.95 (s, 3H) |
| A-253 | (300MHz/CDCl₃) 8.73 (d, 1H), 7.96 (d, 1H), 7.52 (d, 1H), 7.35-7.25 (m, 2H), 7.13 (ddd, 1H), 6.65 (d, 1H), 6.25 (d, 1H), 2.06 (s, 3H), 1.95 (s, 3H), 1.83 (s, 3H) |
| A-255 | (300MHz/CDCl₃) 8.77 (d, 1H), 7.98 (d, 1H), 7.40 (dd, 1H), 7.33-7.24 (m, 3H), 7.13-7.08 (m, 1H), 6.13 (d, 1H), 2.07 (s, 3H), 1.91 (s, 3H), 1.72 (s, 3H) |
| A-256 | (300MHz/CDCl₃) 8.73 (d, 1H), 8.16 (d, 1H), 8.13 (s, 1H), 7.98 (d, 1H), 7.37-7.26 (m, 2H), 7.18-7.12 (m, 1H), 6.36 (d, 1H), 2.11 (s, 3H), 1.96 (s, 3H), 1.83 (s, 3H) |
| A-257 | (300MHz/CDCl₃) 8.70 (d, 1H), 7.97 (d, 1H), 7.94 (d, 1H), 7.39-7.33 (m, 1H), 7.31-7.27 (m, 1H), 7.20 (t, 1H), 6.46 (d, 1H), 2.07 (s, 3H), 1.96 (s, 3H), 1.89 (s, 3H) |
| A-260 | (300MHz/CDCl₃) 8.69 (d, 1H), 7.82-7.73 (m, 2H), 7.64-7.50 (m, 3H), 6.65 (dd, 1H), 6.50 (d, 1H), 2.05 (s, 3H), 1.74 (s, 3H) |
| A-261 | (300MHz/CDCl₃) 8.63 (d, 1H), 8.30 (s, 1H), 7.85-7.77 (m, 2H), 7.71-7.60 (m, 2H), 7.05-6.97 (m, 1H), 2.09 (s, 3H), 1.77 (s, 3H) |
| A-264 | (300MHz/CDCl₃) 8.73 (d, 1H), 8.00 (d, 1H), 7.46-7.45 (m, 1H), 7.38-7.31 (m, 1H), 6.90 (d, 1H), 6.84 (ddd, 1H), 6.45 (d, 1H), 6.25 (dd, 1H), 3.72 (s, 3H), 1.95 (s, 3H), 1.80 (s, 3H) |
| A-265 | (300MHz/CDCl₃) 8.70 (d, 1H), 8.12 (d, 1H), 8.01 (d, 1H), 7.44-7.37 (m, 1H), 6.98-6.89 (m, 2H), 6.56 (dd, 1H), 3.67 (s, 3H), 1.94 (s, 3H), 1.91 (s, 3H) |
| A-270 | (300MHz/CDCl₃) 8.74 (d, 1H), 7.93 (d, 1H), 7.50-7.43 (m, 2H), 7.34-7.30 (m, 1H), 7.19 (ddd, 1H), 6.49 (dd, 1H), 6.46 (d, 1H), 2.00 (s, 3H), 1.82 (s, 3H) |
| A-271 | (300MHz/CDCl₃) 8.70 (d, 1H), 8.17 (d, 1H), 7.96 (d, 1H), 7.56-7.50 (m, 1H), 7.39-7.34 (m, 1H), 7.31 (ddd, 1H), 6.74 (dd, 1H), 2.01 (s, 3H), 1.89 (s, 3H) |
| A-272 | (300MHz/CDCl₃) 8.74 (d, 1H), 7.91 (d, 1H), 7.50-7.43 (m, 1H), 7.36-7.31 (m, 1H), 7.30 (d, 1H), 7.20 (ddd, 1H), 6.45 (dd, 1H), 6.22 (d, 1H), 1.96 (s, 3H), 1.74 (s, 3H) |
| A-273 | (300MHz/CDCl₃) 8.67 (d, 1H), 8.22 (s, 1H), 7.97 (d, 1H), 7.60-7.54 (m, 1H), 7.42-7.34 (m, 2H), 6.78 (dd, 1H), 2.00 (s, 3H), 1.87 (s, 3H) |
| A-274 | (500MHz/CDCl₃) 8.73 (d, 1H), 8.01 (d, 1H), 7.42 (s, 1H), 7.34 (dd, 1H), 7.10 (dt, 1H), 6.47 (d, 1H), 6.46 (d, 1H), 6.38 (d, 1H), 1.90 (s, 6H) |
| A-275 | (500MHz/CDCl₃) 8.69 (d, 1H), 8.09 (s, 1H), 8.01 (d, 1H), 7.41 (dd, 1H), 7.16 (dt, 1H), 6.56 (d, 1H), 6.54 (d, 1H), 1.95 (s, 6H) |
| A-276 | (500MHz/CDCl₃) 8.70 (d, 1H), 8.05 (s, 1H), 8.00 (d, 1H), 7.39 (m, 1H), 7.15 (m, 1H), 6.72 (t, 1H), 6.53 (d, 1H), 6.50 (d, 1H), 1.93 (s, 6H) |
| A-277 | (500MHz/CDCl₃) 8.73 (d, 1H), 8.02 (d, 1H), 7.36 (dd, 1H), 7.27 (d, 1H), 7.11 (dt, 1H), 6.48 (d, 1H), 6.40 (d, 1H), 6.14 (d, 1H), 1.83 (s, 6H) |
| A-278 | (500MHz/CDCl₃) 8.70 (d, 1H), 8.00 (d, 1H), 7.88 (s, 1H), 7.42 (dd, 1H), 7.16 (dt, 1H), 6.57 (d, 1H), 6.55 (d, 1H), 1.88 (s, 6H) |
| A-279 | (500MHz/CDCl₃) 8.73 (d, 1H), 8.01 (d, 1H), 7.35 (dd, 1H), 7.24 (d, 1H), 7.10 (dt, 1H), 6.47 (d, 1H), 6.39 (d, 1H), 6.22 (d, 1H), 1.84 (s, 6H) |
| A-287 | (300MHz/CDCl₃) 8.79 (d, 0.3H), 8.77 (d, 0.7H), 8.18 (d, 0.3H), 8.04 (d, 0.7H), 7.40-7.30 (m, 1.3H), 7.22 (d, 0.7H), 7.15-7.05 (m, 1H), 6.51-6.42 (m, 1H), 6.29 (ddd, 0.7H), 6.24 (ddd, 0.3H), 6.12 (d, 0.3H), 5.99 (d, 0.7H), 2.41 (s, 0.9H), 2.33 (s, 2.1H), 1.91 (s, 1.8H), 1.83 (s, 4.2H) |
| A-289 | (300MHz/CDCl₃) 8.77 (d, 0.21H), 8.76 (d, 0.79H), 8.18 (d, 0.21H), 8.02 (d, 0.79H), 7.40-7.28 (m, 1.21H), 7.19 (d, 0.79H), 7.15-7.04 (m, 1H), 6.49 (dt, 1H), 6.29 (ddd, 1H), 5.91 (d, 0.21H), 5.82 (d, 0.79H), 1.97 (s, 1.26H), 1.83 (s, 4.74H), 1.04-0.87 (m, 3H), 0.74-0.65 (m, 2H) |
| A-291 | (300MHz/CDCl₃) 8.83 (d, 0.35H), 8.76 (d, 0.65H), 8.30 (d, 0.35H), 8.01 (d, 0.65H), 7.38 (d, 0.35H), 7.38-7.28 (m, 1H), 7.22 (d, 0.65H), 7.15-7.03 (m, 1H), 6.50 (dt, 0.65H), 6.35 (dt, 0.35H), 6.26 (ddd, 0.65H), 6.23 (d, 0.35H), 6.10 (ddd, 0.35H), 6.00 (d, 0.65H), 3.21-3.09 (m, 0.35H), 2.78-2.67 (m, 0.65H), 1.87 (s, 2.10H), 1.85 (s, 3.90H), 2.00-0.85 (m, 10H) |
| A-294 | (300MHz/CDCl₃) 8.75 (d, 1H), 8.05 (d, 1H), 7.54 (d, 1H), 7.42-7.33 (m, 1H), 7.13 (ddd, 1H), 6.76 (ddd, 1H), 6.49-6.45 (ddd, 1H), 6.36 (ddd, 1H), 2.88 (s, 3H), 1.91 (s, 3H), 1.90 (s, 3H) |
| A-295 | (300MHz/CDCl₃) 8.74 (d, 1H), 8.05 (d, 1H), 7.53 (d, 1H), 7.43-7.34 (m, 1H), 7.13 (tdd, 1H), 6.78 (d, 1H), 6.52 (ddd, 1H), 6.39 (ddd, 1H), 3.15 (s, 3H), 1.94 (s, 6H) |
| A-318 | (500MHz/CDCl₃) 8.73 (d, 1H), 7.99 (d, 1H), 7.41 (s, 1H), 7.37 (d, 1H), 7.28 (t, 1H), 6.66 (s, 1H), 6.54 (d, 1H), 6.47 (d, 1H), 1.90 (s, 6H) |
| A-319 | (500MHz/CDCl₃) 8.69 (d, 1H), 8.08 (s, 1H), 8.00 (d, 1H), 7.43 (d, 1H), 7.35 (t, 1H), 6.84 (s, 1H), 6.62 (d, 1H), 1.95 (s, 6H) |
| A-322 | (500MHz/CDCl₃) 8.72 (d, 1H), 8.05 (d, 1H), 7.39 (d, 1H), 7.21 (t, 1H), 7.17 (d, 1H), 6.45 (d, 1H), 6.44 (s, 1H), 6.38 (s, 1H), 2.24 (s, 3H), 1.89 (s, 6H) |
| A-323 | (500MHz/CDCl₃) 8.68 (d, 1H), 8.04 (d, 1H), 8.03 (s, 1H), 7.28 (t, 1H), 7.22 (d, 1H), 6.55 (d, 1H), 6.49 (s, 1H), 2.28 (s, 3H), 1.94 (s, 6H) |
| A-324 | (500MHz/CDCl₃) 8.71 (d, 1H), 8.05 (d, 1H), 7.25 (d, 1H), 7.23 (t, 1H), 7.18 (d, 1H), 6.44 (s, 1H), 6.44 (d, 1H), 6.11 (d, 1H), 2.28 (s, 3H), 1.82 (s, 6H) |
| A-327 | (500MHz/CDCl₃) 8.71 (d, 1H), 8.07 (s, 1H), 7.94 (d, 1H), 7.72 (d, 1H), 7.57 (t, 1H), 7.07 (s, 1H), 7.01 (d, 1H), 1.97 (s, 6H) |
| A-328 | (500MHz/CDCl₃) 8.73 (d, 1H), 8.09 (d, 1H), 7.41 (s, 1H), 7.29 (t, 1H), 6.89 (dd, 1H), 6.46 (d, 1H), 6.31 (d, 1H), 6.02 (s, 1H), 3.65 (s, 3H), 1.89 (s, 6H) |
| A-329 | (500MHz/CDCl₃) 8.70 (d, 1H), 8.08 (d, 1H), 8.06 (s, 1H), 7.32 (t, 1H), 6.94 (dd, 1H), 6.35 (d, 1H), 6.14 (s, 1H), 3.70 (s, 3H), 1.95 (s, 6H) |
| A-330 | (500MHz/CDCl₃) 8.73 (d, 1H), 8.08 (d, 1H), 7.27 (t, 1H), 6.90 (d, 1H), 6.33 (d, 1H), 6.12 (d, 1H), 6.07 (s, 1H), 3.68 (s, 3H), 1.84 (s, 6H) |
| | One proton peak overlaps the peak attributable to the measurement solvent |
| A-332 | (500MHz/CDCl₃) 8.75 (d, 1H), 7.92 (d, 1H), 7.52-7.51 (m, 2H), 7.11 (t, 1H), 6.49 (d, 1H), 6.19 (d, 1H), 2.01 (s, 3H), 1.82 (s, 3H) |
| A-333 | (500MHz/CDCl₃) 8.70 (d, 1H), 8.20 (s, 1H), 7.96 (d, 1H), 7.57 (dd, 1H), 7.24-7.21 (m, 1H), 6.47 (dd, 1H), 2.05 (s, 3H), 1.87 (s, 3H) |
| A-334 | (500MHz/CDCl₃) 8.75 (d, 1H), 7.95 (d, 1H), 7.52 (d, 1H), 7.38-7.31 (m, 2H), 6.52 (d, 1H), 6.23 (d, 1H), 2.01 (s, 3H), 1.82 (s, 3H) |
| A-335 | (500MHz/CDCl₃) 8.69 (d, 1H), 8.21 (s, 1H), 7.98 (d, 1H), 7.43-7.37 (m, 2H), 6.65 (d, 1H), 2.03 (s, 3H), 1.90 (s, 3H) |
| A-336 | (500MHz/CDCl₃) 8.75 (d, 1H), 8.00 (d, 1H), 7.47 (s, 1H), 7.23-7.20 (m, 1H), 7.02-6.98 (m, 1H), 6.46 (s, 1H), 6.13 (m, 1H), 1.99 (s, 3H), 1.82 (s, 3H) |
| A-337 | (500MHz/CDCl₃) 8.72 (d, 1H), 8.15 (s, 1H), 8.01 (d, 1H), 7.32-7.23 (m, 1H), 7.13-7.09 (m, 1H), 6.32-6.29 (m, 1H), 2.01 (s, 3H), 1.89 (s, 3H) |
| A-340 | (500MHz/CDCl₃) 8.73 (d, 1H), 7.95 (d, 1H), 7.43 (d, 1H), 7.15 (d, 1H), 7.00 (t, 1H), 6.44 (d, 1H), 6.11 (d, 1H), 2.23 (s, 3H), 1.95 (s, 3H), 1.89 (s, 3H), 1.84 (s, 3H) |
| A-341 | (500MHz/CDCl₃) 8.68 (d, 1H), 8.08 (s, 1H), 7.96 (d, 1H), 7.20 (d, 1H), 7.09 (t, 1H), 6.33 (d, 1H), 2.25 (s, 3H), 1.96 (s, 3H), 1.91 (s, 6H) |
| A-344 | (500MHz/CDCl₃) 8.76 (d, 1H), 7.98 (d, 1H), 7.31 (d, 1H), 6.91 (m, 1H), 6.83 (m, 1H), 6.34 (m, 1H), 6.14 (d, 1H), 1.94 (s, 3H), 1.72 (s, 3H) |
| A-349 | (500MHz/CDCl₃) 8.75 (d, 1H), 7.94 (d, 1H), 7.51 (s, 1H), 7.22-7.13 (m, 2H), 6.49 (d, 1H), 6.09 (d, 1H), 2.01 (s, 3H), 1.82 (s, 3H) |
| A-350 | (500MHz/CDCl₃) 8.70 (d, 1H), 8.19 (s, 1H), 7.97 (d, 1H), 7.28-7.23 (m, 2H), 6.37-6.35 (m, 1H), 2.04 (s, 3H), 1.88 (s, 3H) |
| A-351 | (500MHz/CDCl₃) 8.75 (d, 1H), 7.97 (d, 1H), 7.53 (s, 1H), 7.43-7.40 (m, 1H), 7.09-7.04 (m, 1H), 6.52 (d, 1H), 6.05-6.02 (m, 1H), 2.00 (s, 3H), 1.82 (s, 3H) |
| A-352 | (500MHz/CDCl₃) 8.70 (d, 1H), 8.22 (s, 1H), 7.98 (d, 1H), 7.48-7.45 (m, 1H), 7.16-7.12 (m, 1H), 6.45-6.43 (m, 1H), 2.03 (s, 3H), 1.89 (s, 3H) |
| A-360 | (500MHz/CDCl₃) 8.66 (d, 1H), 8.14 (d, 1H), 8.09 (s, 1H), 7.97 (s, 1H), 7.31 (s, 2H), 1.98 (s, 6H) |
| A-363 | (300MHz/CDCl₃) 8.71 (d, 1H), 7.94 (d, 1H), 7.54-7.52 (m, 1H), 7.40-7.35 (m, 2H), 7.13-7.06 (m, 1H), 6.51 (d, 1H), 6.14 (d, 1H), 2.56-2.44 (m, 1H), 1.97 (s, 3H), 1.81 (s, 3H), 1.19 (d, 3H), 1.16 (d, 3H) |
| A-364 | (300MHz/CDCl₃) 8.65 (d, 1H), 8.20 (s, 1H), 7.95 (d, 1H), 7.45-7.42 (m, 2H), 7.23-7.17 (m, 1H), 6.45 (d, 1H), 2.54-2.45 (m, 1H), 1.97 (s, 3H), 1.89 (s, 3H), 1.20 (d, 3H), 1.16 (d, 3H) |
| A-389 | (300MHz/CDCl₃) 8.74 (d, 1H), 8.07 (d, 1H), 7.47-7.45 (m, 1H), 7.27 (ddd, 1H), 7.03 (ddd, 1H), 6.75 (d, 1H), 6.47 (d, 1H), 6.22 (dd, 1H), 1.99 (s, 3H), 1.88 (s, 3H), 1.44-1.35 (m, 1H), 1.03-0.89 (m, 2H), 0.76-0.65 (m, 2H) |
| A-410 | (500MHz/CDCl₃) 8.77 (d, 1H), 8.04 (d, 1H), 7.48 (s, 1H), 7.11 (d, 1H), 7.01 (t, 1H), 6.46 (s, 1H), 6.23 (d, 1H), 1.93 (brs, 6H) |
| A-411 | (500MHz/CDCl₃) 8.73 (d, 1H), 8.16 (s, 1H), 8.12 (d, 1H), 7.17 (d, 1H), 7.11 (t, 1H), 6.37 (d, 1H), 1.98 (brs, 6H) |
| A-412 | (500MHz/CDCl₃) 8.76 (d, 1H), 8.02 (d, 1H), 7.33 (d, 1H), 7.12 (d, 1H), 7.04 (t, 1H), 6.22 (d, 1H), 6.12 (d, 1H), 1.87 (brs, 6H) |
| A-413 | (500MHz/CDCl₃) 8.73 (d, 1H), 8.10 (d, 1H), 7.95 (s, 1H), 7.18 (d, 1H), 7.12 (t, 1H), 6.37 (d, 1H), 1.91 (brs, 6H) |
| A-414 | (500MHz/CDCl₃) 8.76 (d, 1H), 8.02 (d, 1H), 7.30 (d, 1H), 7.12 (d, 1H), 7.03 (t, 1H), 6.21 (d, 1H), 6.21 (s, 1H), 1.87 (brs, 6H) |
| A-416 | (500MHz/CDCl₃) 8.76 (d, 1H), 8.06 (d, 1H), 7.54 (d, 1H), 7.15 (dd, 1H), 7.07 (t, 1H), 6.64 (d, 1H), 6.24 (dd, 1H), 1.92 (brs, 6H) |
| A-417 | (500MHz/CDCl₃) 8.71 (d, 1H), 8.21 (s, 1H), 8.11 (d, 1H), 7.21 (dd, 1H), 7.16 (t, 1H), 6.41 (dd, 1H), 1.96 (brs, 6H) |
| A-419 | (500MHz/CDCl₃) 8.70 (d, 1H), 8.22 (s, 1H), 8.11 (d, 1H), 7.22 (dd, 1H), 7.18 (t, 1H), 6.52 (dd, 1H), 2.01 (brs, 6H) |
| A-424 | (500MHz/CDCl₃) 8.74 (d, 1H), 8.12 (s, 1H), 8.08 (d, 1H), 7.16 (d, 1H), 7.09 (t, 1H), 6.68 (t, 1H), 6.31 (d, 1H), 1.96 (brs, 6H) |
| A-430 | (300MHz/CDCl₃) 8.76 (d, 1H), 8.02 (d, 1H), 7.40-7.30 (m, 1H), 7.32 (d, 1H), 7.10 (ddd, 1H), 6.51 (ddd, 1H), 6.37 (ddd, 1H), 6.18 (d, 1H), 2.49 (s, 3H), 1.86 (s, 6H) |

**TABLE 4**

| No. | ¹H-NMR (300MHz or 500MHz/solvent) δ (ppm) |
|---|---|
| B-1 | (300MHz/CDCl₃) 8.61 (d, 1H), 8.17 (d, 1H), 7.43-7.37 (m, 3H), 7.37-7.34 (m, 1H), 7.06-6.99 (m, 2H), 6.33 (d, 1H), 1.80-1.61 (m, 4H) |
| B-2 | Geometric isomer of Compound No. B-1 (300MHz/CDCl₃) 10.67 (d, 1H), 8.84 (d, 1H), 7.93-7.86 (m, 2H), 7.74-7.70 (m, 1H), 7.46-7.38 (m, 3H), 6.55 (d, 1H), 1.96-1.89 (m, 2H), 1.45-1.38 (m, 2H) |
| B-3 | (300MHz/CDCl₃) 8.57 (d, 1H), 8.18 (d, 1H), 8.12 (d, 1H), 7.50-7.44 (m, 3H), 7.12-7.05 (m, 2H), 1.79-1.64 (m, 4H) |
| B-4 | Geometric isomer of Compound No.B-3 (300MHz/CDCl₃) 10.13 (d, 1H), 8.86 (d, 1H), 8.37 (s, 1H), 7.92-7.85 (m, 2H), 7.48-7.41 (m, 3H), 2.03-1.97 (m, 2H), 1.53-1.49 (m, 2H) |
| B-6 | (300MHz/CDCl₃) 8.55 (s, 1H), 8.01 (d, 1H), 7.42-7.29 (m, 3H), 7.17-7.08 (m, 2H), 2.62 (s, 3H), 1.83-1.77 (m, 2H), 1.68-1.62 (m, 2H) |
| B-20 | (300MHz/CDCl₃) 8.42 (s, 0.8H), 8.13 (s, 0.2H), 8.05 (s, 0.8H), 7.61 (d, 0.2H), 7.44-7.18 (m, 5H), 4.20 (s, 0.4H), 4.01 (d, 1.6H), 2.40 (s, 0.2H), 2.15 (t, 0.8H), 2.03-1.87 (m, 2H), 1.82-1.68 (m, 2H) |
| B-24 | (300MHz/CDCl₃) 8.38 (s, 0.48H), 8.15 (s, 0.52H), 8.05 (s, 0.48H), 7.68 (s, 0.52H), 7.40-7.12 (m, 5H), 4.72 (s, 1.04H), 4.55 (s, 0.96H), 3.13 (s, 1.56H), 3.05 (s, 1.44H), 2.00-1.65 (m, 4H) |
| B-28 | (300MHz/CDCl₃) 8.22 (s, 0.43H), 8.15 (s, 0.47H), 8.06 (s, 0.43H), 7.52 (s, 0.47H), 7.39-7.21 (m, 5H), 3.60-3.45 (m, 3H), 3.34-3.25 (m, 4H), 1.97-1.83 (m, 2H), 1.80-1.67 (m, 2H) |
| B-81 | (300MHz/CDCl₃) 8.79 (d, 1H), 8.18 (d, 1H), 7.40-7.29 (m, 4H), 6.71-6.64 (m, 2H), 6.44 (d, 1H), 3.11-2.99 (m, 2H), 2.82-2.68 (m, 2H), 2.18-1.98 (m, 2H) |
| B-83 | (300MHz/CDCl₃) 8.73 (d, 1H), 8.07 (d, 1H), 7.42-7.29 (m, 4H), 6.66-6.59 (m, 2H), 6.44 (d, 1H), 2.65-2.41 (m, 4H), 1.98-1.66 (m, 4H) |
| B-87 | (300MHz/CDCl₃) 8.62 (d, 1H), 8.12 (d, 1H), 7.40-7.34 (m, 3H), 7.00-6.95 (m, 2H), 6.34 (d, 1H), 1.80-1.63 (m, 4H) |
| B-91 | (300MHz/CDCl₃) 8.37 (s, 0.38H), 7.67 (s, 0.62H), 7.39 (d, 0.62H), 7.31 (d, 0.38H), 7.28-7.03 (m, 4H), 6.31-6.29 (m, 1H), 4.75 (s, 1.24H), 4.60 (s, 0.76H), 3.18 (s, 1.86H), 3.09 (s, 1.14H), 2.02-1.67 (m, 4H) |
| B-105 | (500MHz/CDCl₃) 10.61 (d, 1H), 8.83 (d, 1H), 7.90 (m, 2H), 7.68 (d, 1H), 7.11 (m, 2H), 6.55 (d, 1H), 1.94 (m, 2H), 1.40 (m, 2H) |
| B-106 | (500MHz/CDCl₃) 10.08 (d, 1H), 8.85 (d, 1H), 8.34 (s, 1H), 7.89 (m, 2H), 7.10 (m, 2H), 2.01 (m, 2H), 1.52 (m, 2H) |
| B-148 | (300MHz/CDCl₃) 8.62 (d, 1H), 7.96 (d, 1H), 7.44-7.40 (m, 2H), 7.35 (ddd, 1H), 7.24 (ddd, 1H), 6.92 (dd, 1H), 6.30 (d, 1H), 1.95-1.62 (m, 4H) |
| B-190 | Geometric isomer of Compound No.B-87 (300MHz/CDCl₃) 10.64 (d, 1H), 8.83 (d, 1H), 7.87-7.81 (m, 2H), 7.69-7.67 (m, 1H), 7.42-7.37 (m, 2H), 6.55 (d, 1H), 1.96-1.90 (m, 2H), 1.42-1.37 (m, 2H) |
| B-191 | (300MHz/CDCl₃) 8.32 (s, 0.50H), 7.53 (s, 0.50H), 7.37 (d, 0.50H), 7.31 (s, 0.50H), 7.28-7.16 (m, 2H), 7.10-7.00 (m, 2H), 6.30 (d, 0.50H), 6.28 (d, 0.50H), 3.32-3.22 (m, 2H), 2.01-1.59 (m, 5H), 1.37-1.24 (m, 1H), 0.86 (t, 1.50H), 0.64 (t, 1.50H) |
| B-192 | Geometric isomer of Compound No.B-105 (500MHz/CDCl₃) 8.62 (d, 1H), 8.14 (d, 1H), 7.35 (d, 1H), 7.09 (m, 2H), 7.03 (m, 2H), 6.33 (d, 1H), 1.77 (m, 2H), 1.66 (m, 2H) |
| B-193 | Geometric isomer of Compound No.B-106 (500MHz/CDCl₃) 8.58 (d, 1H), 8.17 (d, 1H), 8.12 (s, 1H), 7.17 (m, 2H), 7.11 (m, 2H), 1.75 (m, 2H), 1.69 (m, 2H) |
| B-195 | Geometric isomer of Compound No.B-148 (300MHz/CDCl₃) 8.75 (s, 1H), 7.36-7.32 (m, 2H), 7.27 (ddd, 1H), 7.22-7.15 (m, 2H), 6.36 (d, 1H), 5.55 (t, 1H), 4.16-3.87 (m, 2H), 2.64-2.36 (m, 2H) |
| B-196 | (300MHz/CDCl₃) 8.60 (d, 1H), 7.98 (s, 1H), 7.34-7.32 (m, 1H), 7.31-7.27 (m, 1H), 7.22-7.17 (m, 2H), 6.94-6.90 (dd, 1H), 6.32 (d, 1H), 2.14 (s, 3H), 1.87-1.57 (m, 4H) |
| B-197 | Geometric isomer of Compound No.B-196 (300MHz/CDCl₃) 8.76 (s, 1H), 7.26-7.23 (m, 1H), 7.22-7.10 (m, 3H), 7.08-7.05 (m, 1H), 6.41 (d, 1H), 5.30 (t, 1H), 4.29-4.20 (m, 1H), 3.79-3.69 (m, 1H), 2.63-2.53 (m, 1H), 2.41-2.29 (m, 1H), 2.35 (s, 3H) |
| B-198 | (300MHz/CDCl₃) 8.72 (s, 1H), 7.69-7.66 (m, 1H), 7.49-7.38 (m, 2H), 7.29-7.25 (m, 1H), 7.01 (d, 1H), 6.41 (d, 1H), 5.19 (t, 1H), 4.19-3.93 (m, 2H), 2.61-2.32 (m, 2H) |
| B-199 | (300MHz/CDCl₃) 8.63 (d, 1H), 8.18 (d, 1H), 7.46-7.35 (m, 2H), 7.17-7.09 (m, 1H), 6.83 (ddd, 1H), 6.78 (ddd, 1H), 6.36 (d, 1H), 1.84-1.65 (m, 4H) |
| B-201 | (300MHz/CDCl₃) 8.64 (d, 1H), 8.16 (d, 1H), 7.48-7.38 (m, 1H), 7.25 (d, 1H), 7.18-7.10 (m, 1H), 6.84 (ddd, 1H), 6.79 (ddd, 1H), 6.02 (d, 1H), 1.78-1.59 (m, 4H) |

### Test Example 1: Test on effects against southern root-knot nematode (Meloidogyne incognita)

To 200 ml of soil contaminated with southern root-knot nematode, 10 ml of the present composition having the concentration of the compound (I) adjusted to be 400 ppm or 200 ppm with water, was poured, followed by mixing so that the present composition was uniformly dispersed. The treated soil was put into a pot, three cucumber seeds were sown, and the pot was placed in a greenhouse. Two to three weeks after the sowing of the cucumber seeds, the root knot index was determined based on the degree of formation of root knots shown in Table 5. Each composition containing Compound No. A-1, A-2, A-89, A-90, A-94, A-95, A-96, A-107, A-150, A-151, A-152, A-156, A-157, A-160, A-161, A-193, A-195, A-198, A-199, A-200, A-201, A-202, A-203, A-205, A-206, A-216, A-217, A-224, A-233, A-234, A-235, A-240, A-241, A-244, A-245, A-247, A-248, A-249, A-251, A-252, A-253, A-255, A-256, A-257, A-260, A-261, A-264, A-265, A-270, A-271, A-272, A-273, A-274, A-275, A-277, A-278, A-279, A-318, A-319, A-322, A-323, A-324, A-327, A-328, A-329, A-330, A-332, A-333, A-334, A-335, A-336, A-337, A-344, A-349, A-350, A-351, A-352, A-410, A-411, A-412, A-413, A-414, A-416, A-417, A-419, A-424, A-430, B-1, B-2, B-3, B-4, B-81, B-87, B-105, B-106, B-148, B-190, B-192 or B-196 at a concentration of 400 ppm and the composition containing Compound No. A-153 at a concentration of 200 ppm showed high control effects with a root knot index of 1 or less.

**TABLE 5**

| Root knot index | Degree of formation of root knots |
|---|---|
| 0 | No knot formed on the root at all |
| 1 | Knots formed to a slight degree |
| 2 | Knots formed to a moderate degree |
| 3 | Knots formed to a heavy degree |
| 4 | Knots formed to the heaviest degree |

### Test Example 2: Test on effects against Cobb root-lesion nematode (Pratylenchus penetrans)

To 200 ml of soil contaminated with Cobb root-lesion nematode, 10 ml of the present composition having the concentration of the compound (I) adjusted to be 800 ppm with water, was poured, followed by mixing so that the present composition was uniformly dispersed. The treated soil was put into a pot, three burdock seeds were sown, and the pot was placed in a greenhouse. About one month after the sowing of the burdock seeds, the injury index was determined based on the root injury level shown in Table 6. Each composition containing Compound No. A-1, A-318, B-1 or B-192 showed high control effects with an injury index of 1 or less.

**TABLE 6**

| Injury index | Root injury level |
|---|---|
| 0 | No injury |
| 1 | Slight injury |
| 2 | Moderate injury |
| 3 | Heavy injury |
| 4 | Heaviest injury |

### Test Example 3: Test on effects against soybean cyst nematode (Heterodera glycines)

To 200 ml of soil contaminated with soybean cyst nematode, 10 ml of the present composition having the concentration of the compound (I) adjusted to be 800 ppm or 400 ppm with water, was poured, followed by mixing so that the present composition was uniformly dispersed. The treated soil was put into a pot, three soybean seeds were sown, and the pot was placed in a greenhouse. About one month after the sowing of the soybean seeds, the parasite index was determined based on the cyst parasite level on the roots shown in Table 7. Each composition containing Compound No. A-1, A-2, A-89, A-91, A-107, A-109, A-271, A-274, A-279, A-318, A-319, A-322, A-323, A-328, A-333, A-352, A-411, A-416, B-1, B-3, B-4, B-6, B-24, B-192 or B-196 at 800 ppm and each composition containing Compound No. A-260, B-106 or B-193 at 400 ppm showed high control effects with a parasite index of 1 or less.

**TABLE 7**

| Parasite index | Parasite level |
|---|---|
| 0 | No parasitic cyst observed on the roots at all |
| 1 | Parasitic cyst observed to a slight degree |
| 2 | Parasitic cyst observed to a moderate degree |
| 3 | Parasitic cyst observed to a heavy degree |
| 4 | Parasitic cyst observed densely on the entire roots |

Now, effects of the present composition and a composition containing the compound disclosed in WO2020/179910 on southern root-knot nematode are shown in the following Comparative Test.

### Comparative Test

The test on effects against southern root-knot nematode was conducted in the same manner as in Test Example 1 except that Compound No. A-1, A-2 or A-318 was used as the active ingredient in the present composition, compound No. A-1, A-3 or A-58 disclosed in WO2020/179910 was used as the active ingredient in comparative composition, and the active ingredient concentration was adjusted to 50 ppm. The test results are shown in Tables 8 and 9.

As a result, each composition containing the compound disclosed in WO2020/179910 (compound No. A-1, A-3 or A-58) was at a level of root knot index of 2. Whereas each present composition containing the compounds of the present invention (Compound No. A-1, A-2 or A-318) was at a level of root knot index of 1 or 0.

Root knot index of 2 corresponds to a state where the proportion of healthy roots is 50% or less to the entire roots, and indicates control effects to such an extent that cucumber as a crop plant to be tested cannot healthily grow. Whereas root knot index of 1 or 0 corresponds to a state where the proportion of healthy roots is 75% or more to the entire roots, and indicates high control effects such that cucumber can healthily grow.

**TABLE 8**

| Compound No. | Test compound | Root knot index |
|---|---|---|
| No. A-1 | 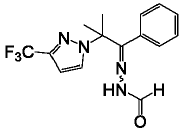 | 1 |
| No. A-2 | 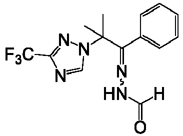 | 0 |
| Compound A-1 (WO2020/179910) | 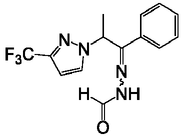 | 2 |
| Compound A-3 (WO2020/179910) | 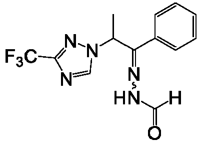 | 2 |

**TABLE 9**

| Compound No. | Test compound | Root knot index |
|---|---|---|
| No. A-318 | 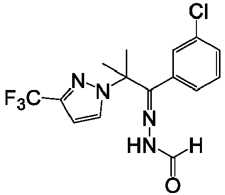 | 1 |
| Compound A-58 (WO2020/179910) | 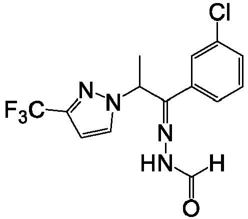 | 2 |

Now, formulation of the present composition will be specifically described. However, the mix ratio, the dosage form, etc. are not limited to the following Formulation Examples.

Formulation Examples are described below.

### FORMULATION EXAMPLE 1

| | |
|---|---|
| (1) Compound (I) | 20 parts by weight |
| (2) Clay | 70 parts by weight |
| (3) White carbon | 5 parts by weight |
| (4) Sodium polycarboxylate | 3 parts by weight |
| (5) Sodium alkylnaphthalene sulfonate | 2 parts by weight |

The above components are uniformly mixed to obtain a wettable powder.

### FORMULATION EXAMPLE 2

| | |
|---|---|
| (1) Compound (I) | 5 parts by weight |
| (2) Talc | 60 parts by weight |
| (3) Calcium carbonate | 34.5 parts by weight |
| (4) Liquid paraffin | 0.5 part by weight |

The above components are uniformly mixed to obtain a dust.

### FORMULATION EXAMPLE 3

| | |
|---|---|
| (1) Compound (I) | 20 parts by weight |
| (2) N,N-dimethylacetamide | 20 parts by weight |
| (3) Polyoxyethylene tristyryl phenyl ether | 10 parts by weight |
| (4) Calcium dodecylbenzene sulfonate | 2 parts by weight |
| (5) Xylene | 48 parts by weight |

The above components are uniformly mixed and dissolved to obtain an emulsifiable concentrate.

### FORMULATION EXAMPLE 4

| | |
|---|---|
| (1) Clay | 68 parts by weight |
| (2) Sodium lignin sulfonate | 2 parts by weight |
| (3) Polyoxyethylenealkylaryl sulfate | 5 parts by weight |
| (4) White carbon | 25 parts by weight |

The mixture of the above components is mixed with Compound (I) in a weight ratio of 4:1 to obtain a wettable powder.

### FORMULATION EXAMPLE 5

| | |
|---|---|
| (1) Compound (I) | 50 parts by weight |
| (2) Sodium alkylnaphthalene sulfonate condensed with formaldehyde | 2 parts by weight |
| (3) Silicone oil | 0.2 part by weight |
| (4) Water | 47.8 parts by weight |

The above components are uniformly mixed and pulverized to obtain a base liquid, and

| | |
|---|---|
| (5) Sodium polycarboxylate | 5 parts by weight |
| (6) Anhydrous sodium sulfate | 42.8 parts by weight |

are added, and the mixture is uniformly mixed, granulated and dried to obtain water-dispersible granules.

### FORMULATION EXAMPLE 6

| | |
|---|---|
| (1) Compound (I) | 5 parts by weight |
| (2) Polyoxyethylene octylphenyl ether | 1 part by weight |
| (3) Polyoxyethylene alkyl ether phosphoric acid ester | 0.1 part by weight |
| (4) Granular calcium carbonate | 93.9 parts by weight |

The above components (1) to (3) are preliminarily uniformly mixed and diluted with a proper amount of acetone, and then the mixture is sprayed onto the component (4), and acetone is removed to obtain granules.

### FORMULATION EXAMPLE 7

| | |
|---|---|
| (1) Compound (I) | 2.5 parts by weight |
| (2) N,N-dimethylacetamide | 2.5 parts by weight |
| (3) Soybean oil | 95 parts by weight |

The above components are uniformly mixed and dissolved to obtain an ultra low volume formulation.

### FORMULATION EXAMPLE 8

| | |
|---|---|
| (1) Compound (I) | 40 parts by weight |
| (2) Potassium polyoxyethylene tristyryl phenyl ether phosphate | 4 parts by weight |
| (3) Silicone oil | 0.2 part by weight |
| (4) Xanthan gum | 0.1 part by weight |
| (5) Ethylene glycol | 5 parts by weight |
| (6) Water | 50.7 parts by weight |

The above components are uniformly mixed and pulverized to obtain a water-based suspension concentrate.

### FORMULATION EXAMPLE 9

| | |
|---|---|
| (1) Compound (I) | 10 parts by weight |
| (2) Diethylene glycol monoethyl ether | 80 parts by weight |
| (3) Polyoxyethylene alkyl ether | 10 parts by weight |

The above components are uniformly mixed to obtain a liquid.

The entire disclosure of Japanese Patent Application No. 2021-113233 filed on July 8, 2021 including specification, claims and abstract is incorporated herein by reference in its entirety.

## Claims

1. A nematicidal composition comprising as an active ingredient a compound represented by the formula (I) or its salt: 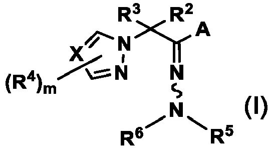 wherein X is N, CH or CR⁴,
A is an unsubstituted phenyl, or a phenyl substituted by one to five R¹,
R¹ is halogen, alkyl, haloalkyl, alkenyl, alkynyl, cycloalkyl, alkoxy or haloalkoxy, provided that when there are two R¹, the two R¹ together may form a ring which may be substituted by one or two Z¹,
Z¹ is halogen or alkyl,
R² and R³ are alkyl, or R² and R³ together form a (C₃-C₆)-carbon ring,
R⁴ is halogen, alkyl, haloalkyl, alkenyl, alkynyl, cycloalkyl, alkoxy, haloalkoxy, alkylthio, alkylsulfinyl, alkylsulfonyl, nitro or cyano,
R⁵ is CHO,
R⁶ is H, -SO₂CF₃, -C(=O)OZ³, alkyl, alkenyl, alkynyl, alkoxyalkyl, or aralkyl which may be substituted by Z²,
Z² is alkoxy,
Z³ is aralkyl which may be substituted by Z², or alkyl, and
m is an integer of from 0 to 2.

2. A method for controlling nematodes, which comprises applying a nematicidal composition containing an effective amount of a compound represented by the formula (I) or its salt to soil, to nematodes or to a plant body: 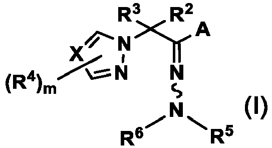 wherein X is N, CH or CR⁴,
A is an unsubstituted phenyl, or a phenyl substituted by one to five R¹,
R¹ is halogen, alkyl, haloalkyl, alkenyl, alkynyl, cycloalkyl, alkoxy or haloalkoxy, provided that when there are two R¹, the two R¹ together may form a ring which may be substituted by one or two Z¹,
Z¹ is halogen or alkyl,
R² and R³ are alkyl, or R² and R³ together form a (C₃-C₆)-carbon ring,
R⁴ is halogen, alkyl, haloalkyl, alkenyl, alkynyl, cycloalkyl, alkoxy, haloalkoxy, alkylthio, alkylsulfinyl, alkylsulfonyl, nitro or cyano,
R⁵ is CHO,
R⁶ is H, -SO₂CF₃, -C(=O)OZ³, alkyl, alkenyl, alkynyl, alkoxyalkyl, or aralkyl which may be substituted by Z²,
Z² is alkoxy,
Z³ is aralkyl which may be substituted by Z², or alkyl, and
m is an integer of from 0 to 2.

3. A compound selected from the group consisting of compounds of the following formulae, or its salt.

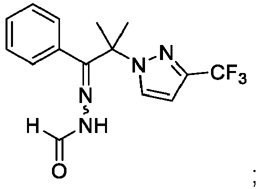

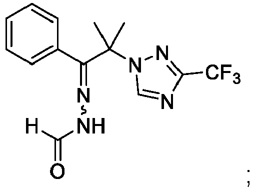

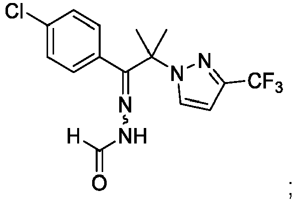

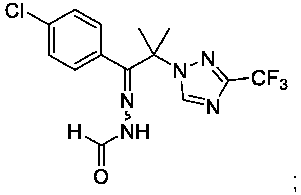

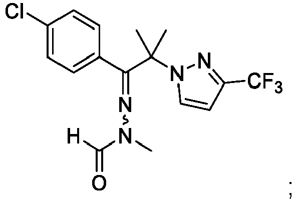

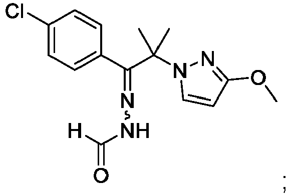

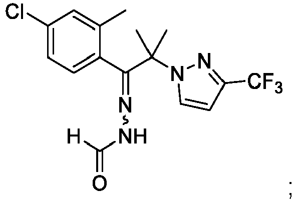

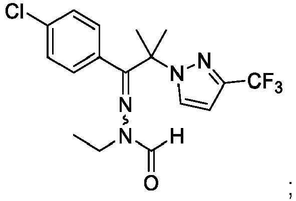

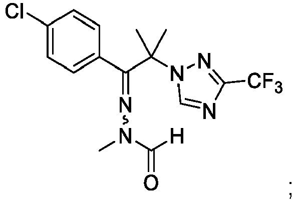

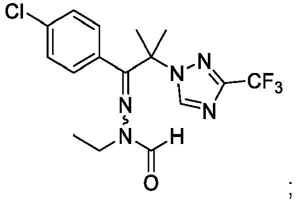

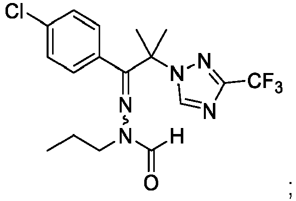

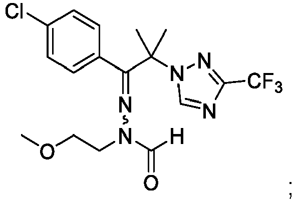

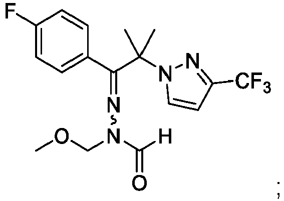

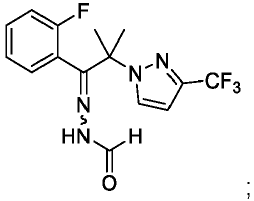

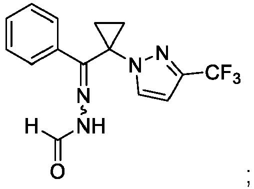

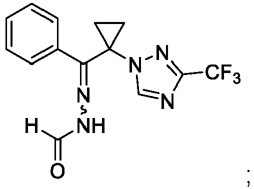

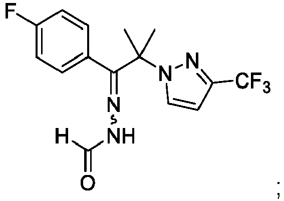

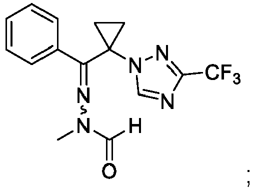

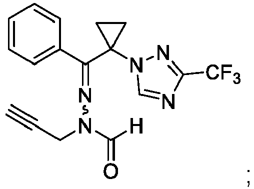

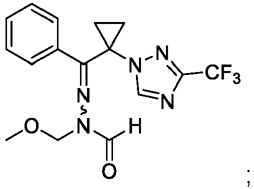

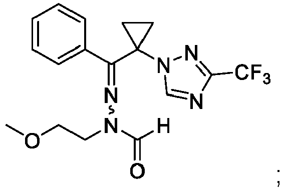

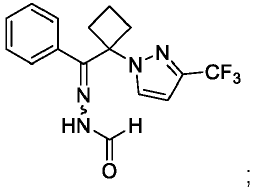

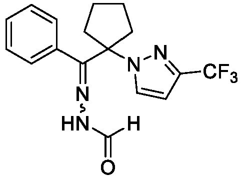

4. The nematicidal composition according to Claim 1, wherein in the compound represented by the formula (I) or its salt, A is phenyl substituted by one to five R¹, R¹ is halogen, alkyl, haloalkyl, alkenyl, alkynyl, cycloalkyl, alkoxy or haloalkoxy, provided that when there are two R¹, the two R¹ together may form a ring which may be substituted by one or two Z¹.

5. The method for controlling nematodes according to Claim 2, wherein in the compound represented by the formula (I) or its salt, A is phenyl substituted by one to five R¹, R¹ is halogen, alkyl, haloalkyl, alkenyl, alkynyl, cycloalkyl, alkoxy, or haloalkoxy, provided that when there are two R¹, the two R¹ together may form a ring which may be substituted by one or two Z¹.

6. A compound represented by the formula (IA) or its salt: 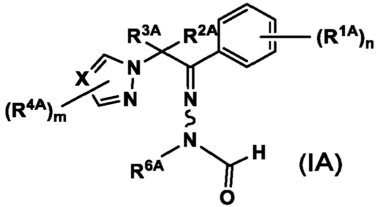
wherein X is N, CH or CR^{4A},
R^{1A} is halogen, (C₁-C₆)-alkyl, (C₁-C₆)-haloalkyl, (C₂-C₆)-alkenyl, (C₂-C₆)-alkynyl, (C₃-C₆)-cycloalkyl, (C₁-C₆)-alkoxy or (C₁-C₆)-haloalkoxy, provided that when there are two R^{1A}, the two R^{1A} together may form a ring which may be substituted by one or two Z^{1A},
Z^{1A} is halogen or (C₁-C₆)-alkyl,
R^{2A} and R^{3A} are (C₁-C₆)-alkyl, or R^{2A} and R^{3A} together may form a (C₃-C₆)-carbon ring,
R^{4A} is halogen, (C₁-C₆)-alkyl, (C₁-C₆)-haloalkyl, (C₂-C₆)-alkenyl, (C₂-C₆)-alkynyl, (C₃-C₆)-cycloalkyl, (C₁-C₆)-alkoxy, (C₁-C₆)-haloalkoxy, (C₁-C₆)-alkylthio, (C₁-C₆)-alkylsulfinyl, (C₁-C₆)-alkylsulfonyl, nitro or cyano,
R^{6A} is H, -SO₂CF₃, -C(=O)OZ^{3A}, (C₁-C₆)-alkyl, (C₂-C₆)-alkenyl, (C₂-C₆)-alkynyl, (C₁-C₆)-alkoxy-(C₁-C₆)-alkyl, or (C₇-C₁₂)-aralkyl which may be substituted by Z^{2A},
Z^{2A} is (C₁-C₆)-alkoxy,
Z^{3A} is (C₇-C₁₂)-aralkyl which may be substituted by Z^{2A}, or (C₁-C₆)-alkyl,
n is an integer of from 1 to 5, and
m is an integer of from 0 to 2.

7. A compound represented by the formula (IB) or its salt: 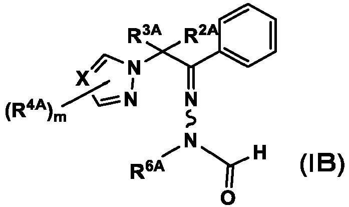
wherein X is N, CH or CR^{4A},
R^{2A} and R^{3A} are (C₁-C₆)-alkyl, or R^{2A} and R^{3A} together may form a (C₃-C₆)-carbon ring,
R^{4A} is halogen, (C₁-C₆)-alkyl, (C₁-C₆)-haloalkyl, (C₂-C₆)-alkenyl, (C₂-C₆)-alkynyl, (C₃-C₆)-cycloalkyl, (C₁-C₆)-alkoxy, (C₁-C₆)-haloalkoxy, (C₁-C₆)-alkylthio, (C₁-C₆)-alkylsulfinyl, (C₁-C₆)-alkylsulfonyl, nitro or cyano,
R^{6A} is H, -SO₂CF₃, -C(=O)OZ^{3A}, (C₁-C₆)-alkyl, (C₂-C₆)-alkenyl, (C₂-C₆)-alkynyl or (C₁-C₆)-alkoxy-(C₁-C₆)-alkyl, or (C₇-C₁₂)-aralkyl which may be substituted by Z^{2A},
Z^{2A} is (C₁-C₆)-alkoxy,
Z^{3A} is (C₇-C₁₂)-aralkyl which may be substituted by Z^{2A}, or (C₁-C₆)-alkyl, and
m is an integer of from 0 to 2.

8. The compound or its salt according to Claim 6, wherein in the compound represented by the formula (IA) or its salt, R^{1A} is halogen, (C₁-C₆)-alkyl, (C₁-C₆)-haloalkyl, (C₂-C₆)-alkenyl, (C₂-C₆)-alkynyl, (C₃-C₆)-cycloalkyl, (C₁-C₆)-alkoxy, or (C₁-C₆)-haloalkoxy.

9. The compound or its salt according to Claim 6, wherein in the compound represented by the formula (IA) or its salt, R^{2A} and R^{3A} are (C₁-C₃)-alkyl.

10. The compound or its salt according to Claim 6, wherein in the compound represented by the formula (IA) or its salt, R^{4A} is halogen, (C₁-C₆)-haloalkyl, (C₁-C₆)-alkylsulfinyl, (C₁-C₆)-alkylsulfonyl, nitro or cyano.

11. The compound or its salt according to Claim 7, wherein in the compound represented by the formula (IB) or its salt, R^{2A} are R^{3A} are (C₁-C₃)-alkyl.

12. The compound or its salt according to Claim 7, wherein in the compound represented by the formula (IB) or its salt, R^{4A} is halogen, (C₁-C₆)-haloalkyl, (C₁-C₆)-alkylsulfinyl, (C₁-C₆)-alkylsulfonyl, nitro or cyano.

13. A method for controlling nematodes, which comprises applying a nematicidal composition containing an effective amount of the compound or its salt as defined in Claim 6 or 7, to soil, to nematodes or to a plant body.

14. An agricultural and horticultural nematicide comprising as an active ingredient the compound or its salt as defined in Claim 6 or 7.
